# EUROPEAN PATENT APPLICATION

(11) **EP 4 840 461 A2**
(43) Date of publication of application: **18.11.2026**
(21) Application number: 26208235.7
(22) Date of filing: 25.10.2017
(51) Int. Cl.: A61K 39/12

(54) **PREFUSION CORONAVIRUS SPIKE PROTEINS AND THEIR USE**

(30) Priority: 25.10.2016 US 201662412703 P
(62) Document number(s) of the earlier application(s) in accordance with Art. 76 EPC: 17800655.7 / 3 532 095
(71) Applicant: Trustees of Dartmouth College, Hanover, NH 03755-1404 (US); The Scripps Research Institute, La Jolla, CA 92037 (US); The United States of America, as represented by The Secretary, Department of Health and Human Services, Bethesda, MD 20852-7660 (US)
(72) Inventor: GRAHAM, Barney, Bethesda, MD 20892 (US); MCLELLAN, Jason, Hanover, NH 03755 (US); WARD, Andrew, La Jolla, CA 92037 (US); COTTRELL, Christopher, La Jolla, CA 92037 (US); JOYCE, Michael, Gordon, Bethesda, MD 20892 (US); KANEKIYO, Masaru, Bethesda, MD 20892 (US); WANG, Nianshuang, Hanover, NH 03755 (US); PALLESEN, Jesper, La Jolla, CA 92037 (US); YASSINE, Hadi, Doha (QA); TURNER, Hannah, La Jolla, CA 92037 (US); KIRCHDOERFER, Robert, La Jolla, CA 92037 (US); CORBETT, Kizzmekia, Bethesda, MD 20892 (US)
(74) Representative: Vossius & Partner Patentanwälte Rechtsanwälte mbB

(57) **Abstract**

Coronavirus S ectodomain trimers stabilized in a prefusion conformation, nucleic acid molecules and vectors encoding these proteins, and methods of their use and production are disclosed. In several embodiments, the coronavirus S ectodomain trimers and/or nucleic acid molecules can be used to generate an immune response to coronavirus in a subject. In additional embodiments, the therapeutically effective amount of the coronavirus S ectodomain trimers and/or nucleic acid molecules can be administered to a subject in a method of treating or preventing coronavirus infection.

## Description

### CROSS REFERENCE TO RELATED APPLICATIONS

This application claims the benefit of U.S. Provisional Application No. 62/412,703, filed October 25, 2016, which is herein incorporated by reference in its entirety.

### FIELD OF THE DISCLOSURE

This disclosure relates to recombinant coronavirus spike (S) proteins, such as Middle East respiratory syndrome coronavirus (MERS-CoV) and severe acute respiratory syndrome coronavirus (SARS-CoV) S proteins, that are stabilized in a prefusion conformation by one or more amino acid substitutions, and their use as immunogens.

### BACKGROUND

Coronaviruses are enveloped, positive-sense single-stranded RNA viruses. They have the largest genomes (26-32 kb) among known RNA viruses, and are phylogenetically divided into four genera (α, β, γ, δ), with betacoronaviruses further subdivided into four lineages (A, B, C, D). Coronaviruses infect a wide range of avian and mammalian species, including humans. Of the six known human coronaviruses, four of them (HCoV-OC43, HCoV-229E, HCoV-HKU1 and HCoV-NL63) circulate annually in humans and generally cause mild respiratory diseases, although severity can be greater in infants, elderly, and the immunocompromised. In contrast, the Middle East respiratory syndrome coronavirus (MERS-CoV) and the severe acute respiratory syndrome coronavirus (SARS-CoV), belonging to betacoronavirus lineages C and B, respectively, are highly pathogenic. Both viruses emerged into the human population from animal reservoirs within the last 15 years and caused outbreaks with high case-fatality rates.

MERS-CoV was isolated in 2012 from a patient in Saudi Arabia and is still circulating across the Arabian Peninsula. Primary transmission, most likely from camels, is now considered to be the most common route of transmission, and camels are thought to be a secondary or intermediate reservoir for MERS-CoV, with bats serving as the primary reservoir. Human-to-human transmission, especially as a result of close contact between patients and hospital workers within health care settings, is another important route of transmission, and was responsible for an outbreak of MERS-CoV in South Korea. The high pathogenicity and airborne transmissibility of SARS-CoV and MERS-CoV have raised concern about the potential for another coronavirus pandemic. The high case-fatality rate, vaguely defined epidemiology, and absence of prophylactic or therapeutic measures against coronaviruses have created an urgent need for an effective vaccine and related therapeutic agents.

### SUMMARY

Disclosed herein are recombinant coronavirus S ectodomain trimers comprising protomers comprising one or more proline substitution(s) that stabilize the S protein trimer in the prefusion conformation. One class of mutation, comprising one or more (such as two) proline substitutions at or near the boundary between a Heptad Repeat 1 (HR1) and a central helix of the protomers of the coronavirus S ectodomain trimer was found to be surprisingly effective for stabilization of coronavirus S protein trimers in the prefusion conformation. Embodiments of such prefusion-stabilized coronavirus S ectodomain trimers are demonstrated to produce a superior immune response in an animal model compared to corresponding coronavirus S ectodomain trimers that are not stabilized in the prefusion conformation.

In some embodiments, an immunogen is provided that comprises a recombinant alphacoronavirus or betacoronavirus S ectodomain trimer comprising protomers comprising one or two proline substitutions at or near a junction between a heptad repeat 1 (HR1) and a central helix that stabilize the S ectodomain trimer in a prefusion conformation. The one or two proline substitutions can comprise two consecutive proline substitutions (a "double proline substitution"). In some embodiments, the recombinant alphacoronavirus or betacoronavirus S ectodomain trimer comprises S ectodomains from a NL63-CoV, 229E-CoV, OC43-CoV, SARS-CoV, MERS-CoV, HKU1-CoV, WIV1-CoV, mouse hepatitis virus (MHV), or HKU9-CoV, that comprise the one or two proline substitutions.

In some embodiments, the recombinant alphacoronavirus or betacoronavirus S ectodomain trimer comprises: a recombinant HKU1-CoV S ectodomain trimer, and the double proline substitution is between residues 1050 to 1070 of the protomers in the trimer (for example, N1067P and L1068P substitutions); a recombinant SARS-CoV S ectodomain trimer, and the double proline substitution is between residues 951 to 971 of the protomers in the trimer (for example, K968P and V969P substitutions); a recombinant MERS-CoV S ectodomain trimer, and the double proline substitution is between residues 1050 to 1069 of the protomers in the trimer (for example, V1060P and L1061P substitutions); a recombinant OC43-CoV S ectodomain trimer, and the double proline substitution is between residues 1062 to 1082 of the protomers in the trimer (for example, A1079P and L1080P substitutions); a recombinant HKU9-CoV S ectodomain trimer, and the double proline substitution is between residues 966 to 986 of the protomers in the trimer (for example, G1018P and L1019P substitutions); a recombinant NL63-CoV S ectodomain trimer, and the double proline substitution is between residues 1035 to 1055 of the protomers in the trimer (for example, S1052P and I1053P substitutions); a recombinant 229E-CoV S ectodomain trimer, and the double proline substitution is between residues 852 to 872 of the protomers in the trimer (for example, I869P and I870P substitutions); a recombinant WIV1-CoV S ectodomain trimer, and the double proline substitution is between residues 952 to 972 of the protomers in the trimer (for example, K969P and V970P substitutions); or a recombinant MHV S ectodomain trimer, and the double proline substitution is between residues 852 to 872 of the protomers in the trimer (for example, I869P and I870P substitutions).

In some embodiments, the protomers of the recombinant alphacoronavirus or betacoronavirus S ectodomain trimer further comprise one or more additional amino acid substitutions or deletions, such as amino acid substitutions that stabilize the recombinant alphacoronavirus or betacoronavirus S ectodomain trimer in the prefusion conformation, or amino acid substitutions to inhibit or prevent protease cleavage at a S1/S2 protease cleavage site and/or a S2' protease cleave site of the S ectodomain.

In some embodiments, the protomers of the recombinant alphacoronavirus or betacoronavirus S ectodomain trimer can be linked to a trimerization domain (such as T4 Fibritin trimerization domain). In additional embodiments, the protomers of the recombinant alphacoronavirus or betacoronavirus S ectodomain trimer can be linked to a transmembrane domain.

In additional embodiments, the recombinant coronavirus S ectodomain trimer can be included on a protein nanoparticle, such as a ferritin protein nanoparticle. Nucleic acid molecules encoding a protomer of the disclosed recombinant coronavirus S ectodomain trimers are also provided, as are vectors including the nucleic acid molecules, and methods of producing the disclosed coronavirus S ectodomain trimers.

Immunogenic compositions including the recombinant coronavirus S ectodomain trimer that are suitable for administration to a subject are also provided, and may also be contained in a unit dosage form. The compositions can further include an adjuvant. The recombinant coronavirus S ectodomain trimers may also be conjugated to a carrier to facilitate presentation to the immune system.

Methods of inducing an immune response in a subject are disclosed, as are methods of treating, inhibiting or preventing a coronavirus infection in a subject, by administering to the subject an effective amount of a disclosed recombinant coronavirus S ectodomain trimer, nucleic acid molecule, or vector.

The foregoing and other features and advantages of this disclosure will become more apparent from the following detailed description of several embodiments which proceeds with reference to the accompanying figures.

### BRIEF DESCRIPTION OF THE FIGURES

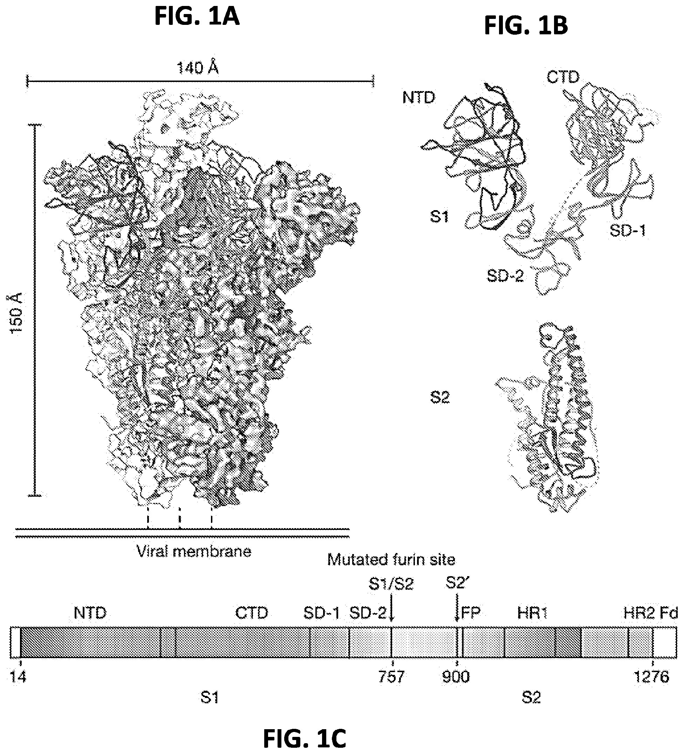

**FIGs. 1A-1C** illustrate the structure of the HKU1-CoV prefusion spike ectodomain. (1A) A single protomer of the trimeric S protein is shown in cartoon representation colored as a rainbow from the N to C terminus (blue to red) with the reconstructed EM density of remaining protomers shown in white and grey. (1B) The S1 subunit is composed of the N-terminal domain (NTD) and C-terminal domain (CTD) as well as two sub-domains (SD-1 and SD-2). The S2 subunit contains the coronavirus fusion machinery and is primarily α-helical. (1C) Domain architecture of the HKU1-CoV S protein colored as in (1A).
**FIG.s. 2A-2D** illustrate the architecture of the HKU1-CoV S1 subunit. (2A) EM density corresponding to each S1 protomer is shown. The putative glycan-binding and protein-receptor-binding sites are indicated with dashed shapes on the NTD and CTD, respectively. (2B) The HKU1-CoV S1 CTD forms quaternary interactions with an adjacent CTD using a surface similar to that used by SARS-CoV CTD to bind its receptor, ACE2. (2C) SD-1 is composed of amino acid residues before and after the S1 CTD. (2D) SD-2 is composed of S1 sequence C-terminal to the CTD, a short peptide following the NTD, and the N-terminal strand of S2, which follows the S1/S2 furin-cleavage site.

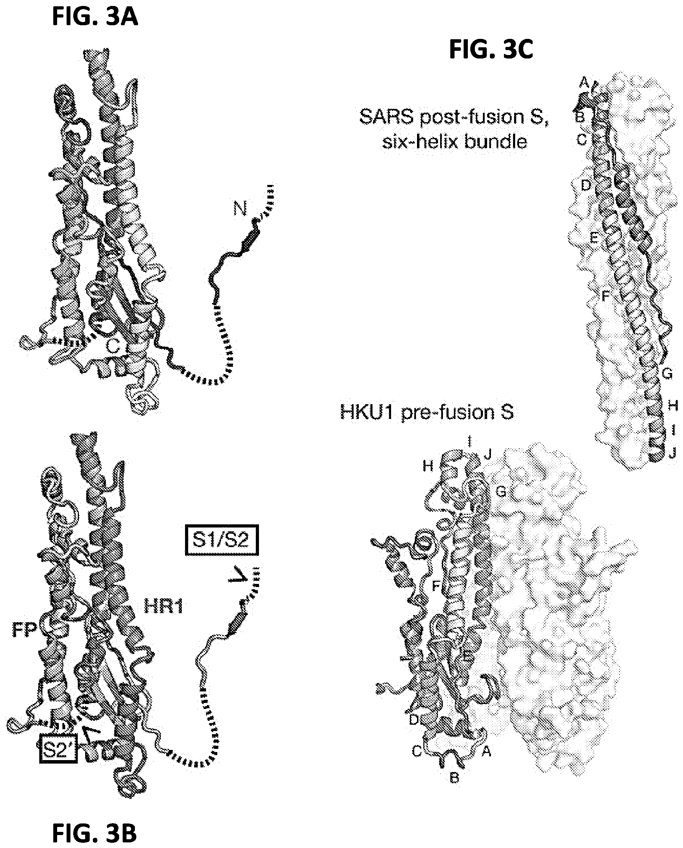

**FIGs. 3A-3C** illustrate the HKU1-CoV S2 subunit fusion machinery. (3A) The HKU1-CoV S2 subunit is colored like a rainbow from the N-terminal β-strand (blue), which participates in S1 sub-domain 2, to the C terminus (red) before HR2. (3B) The HKU1-CoV S2 structure contains the fusion peptide (FP) and a HR1. Protease-recognition sites are indicated within disordered regions of the protein (dashed lines). (3C) A comparison of coronavirus S2 HR1 in the pre- and post-fusion conformations. Five HR1 α-helices are labelled and colored like a rainbow from blue to red, N to C terminus, respectively. The structures are oriented to position similar portions of the central helix (red).

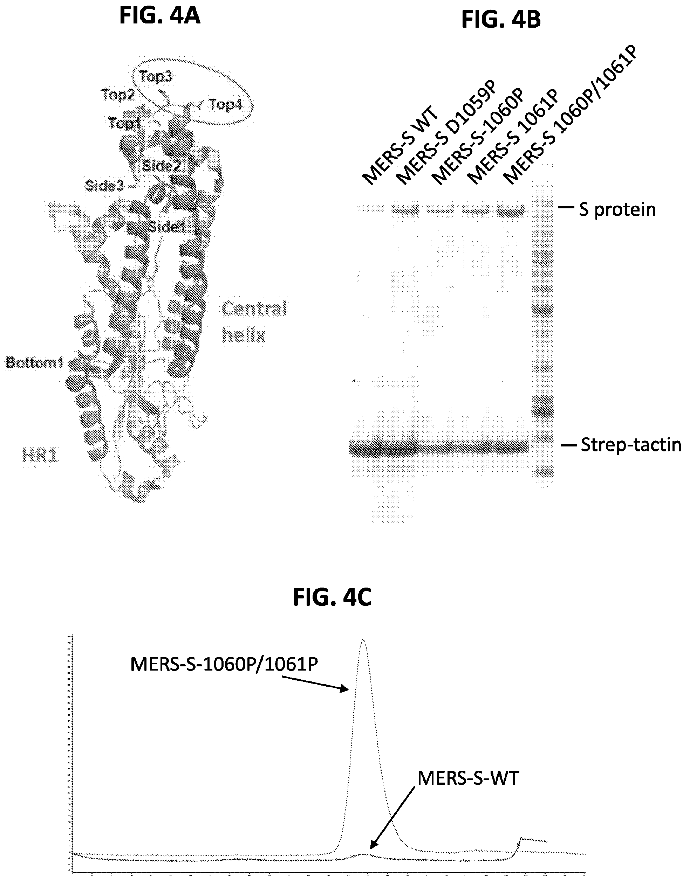

**FIGs. 4A-4C** illustrate stabilization of MERS-CoV S protein in a prefusion conformation by V1060P ("Top 3") and L1061P ("Top 4") amino acid substitutions. (4A) Location of various stabilization design conceptions. V1060P and L1061P (red circle) are located at the top of S2 HR1 and the S2 central helix. MERS-CoV S ectodomains with V1060P and L1061P mutations were expressed individually and in combination and purified. Protein expression levels and purity were determined by (4B) gel electrophoresis and (4C) size-exclusion chromatography.

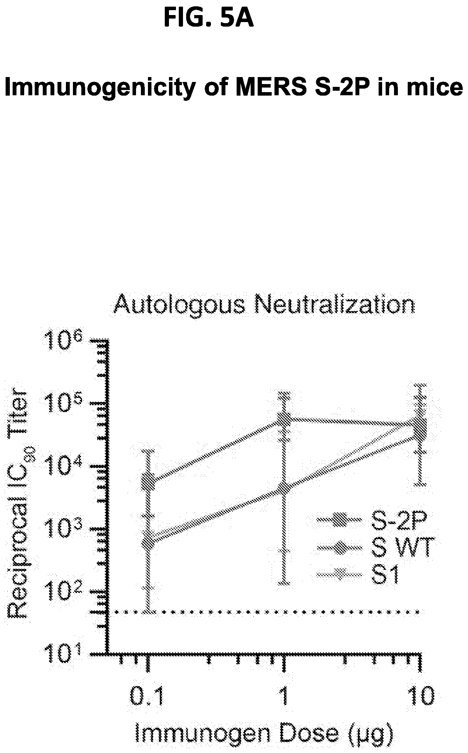

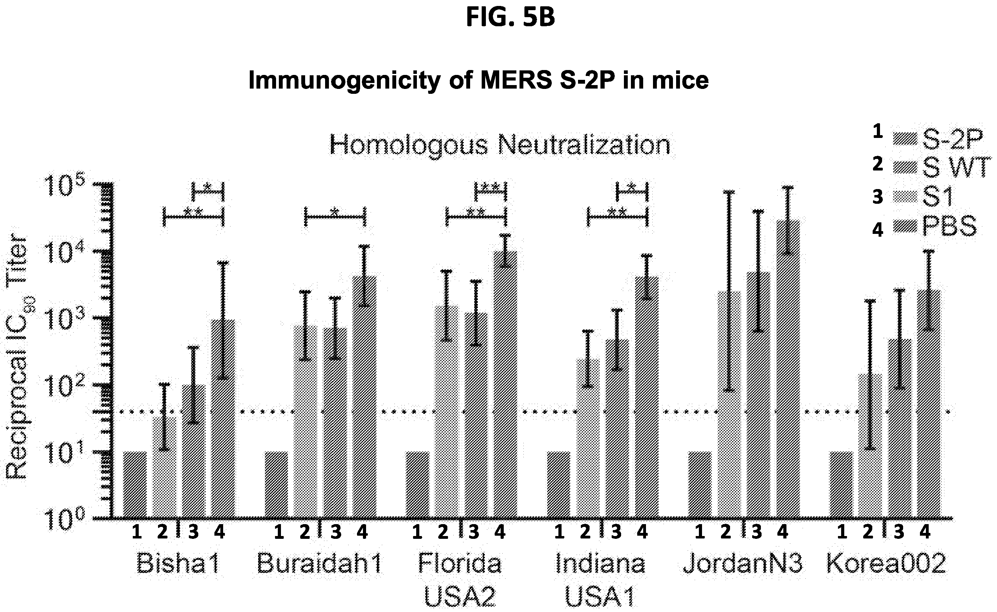

**FIGs. 5A-5B** are a set of graphs showing results from neutralization assays using sera from mice immunized with the MERS-CoV S prefusion stabilized (2P) ectodomain trimer. Mice (N= 5/group) were immunized with 0.1 µg of MERS-CoV wild-type S ectodomain trimer or MERS-CoV prefusion-stabilized S ectodomain trimer intramuscularly with Sigma Adjuvant System at weeks 0 and 3. Control mice were given PBS. Two weeks following the last immunization, serum was collected and tested for neutralizing antibodies against various MERS pseudovirus strains: England1, Florida USA2, Bisha1, Korea002, JordanN3, Buraidah1, and Indiana USA1. (FIG. 5A) Reciprocal serum IC₉₀ neutralizing activity against autologous MERS England1 pseudotyped lentivirus reporter plotted against vaccine dose. (FIG. 5B) Reciprocal serum IC₉₀ neutralizing activity against multiple homologous MERS-CoV pseudoviruses of sera from mice immunized with 0.1 µg of purified MERS-CoV S ectodomain trimer. For both panels, the geometric mean IC₉₀ titer (GMT) of cach group is represented by (FIG. 5A) symbols or (FIG. 5B) bars. Error bars represent geometric SDs. P values denoted as **P* < 0.05 and ***P* < 0.01. The limit of detection for the assay is represented by dotted lines; for sera below the limit of detection a reciprocal IC₉₀ titer of 10 was assigned.

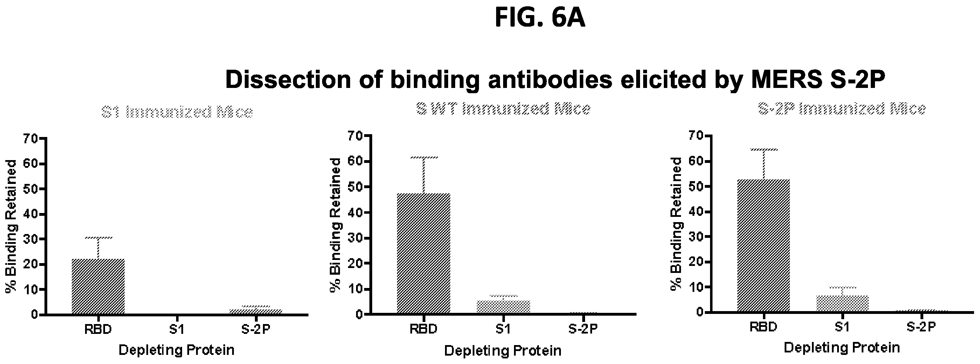

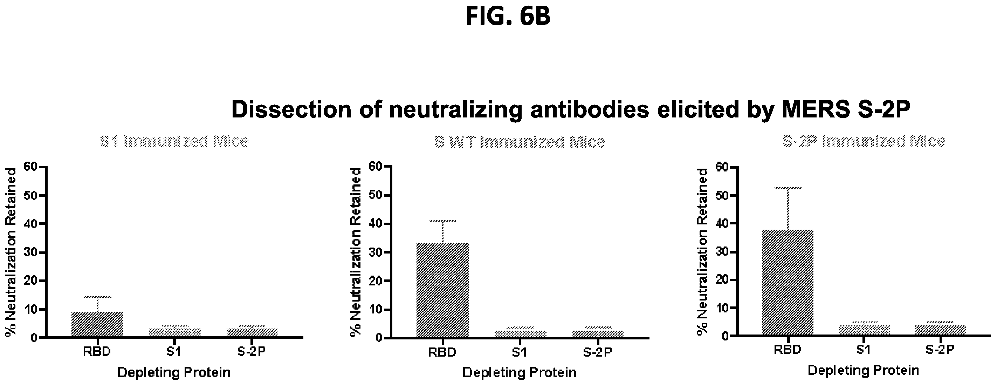

**FIGs. 6A** **and** **6B** shows results from the dissection of binding and neutralizing antibodies elicited by MERS S-2P. Serum from mice immunized with (A) MERS S1, (B) MERS S WT ectodomain trimer, and MERS S-2P ectodomain trimer were depleted of MERS RBD, MERS S1, and MERS S-2P ectodomain trimer specific antibodies by magnetic bead depletion. The resulting depleted serum was then tested for (FIG. 6A) MERS S-2P ectodomain trimer specific antibodies by ELISA or (FIG. 6B) neutralizing antibodies against MERS England1 pseudovirus. For the binding assays. endpoint ELISA titers were determined, and % binding retained was calculated as a measure of endpoint titers for each serum depleted with MERS protein compared to binding after depletion with a nonspecific protein. For the neutralization assays, IC50 titers were determined, and % neutralization retained was calculated as a measure of neutralization each serum depleted with MERS protein compared to binding after depletion with a nonspecific protein. Bars represent the mean of each group; error bars represent SD.

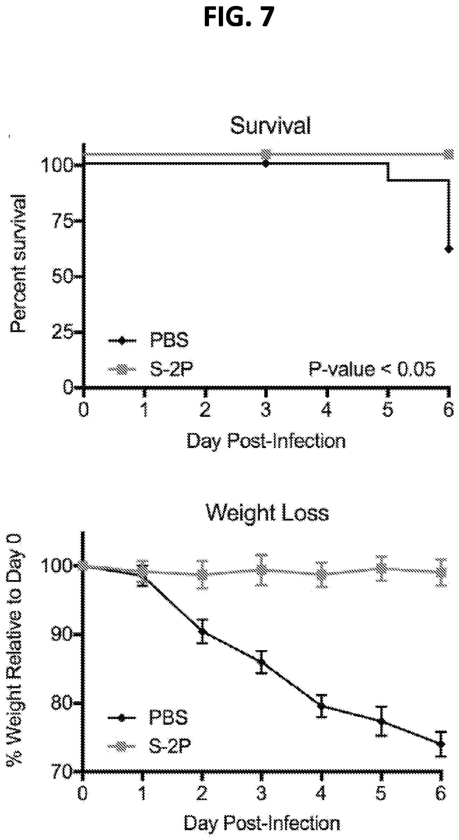

**FIG. 7** is a set of graphs showing that MERS-CoV S-2P immunization protects against lethal MERS challenge in mice. C57BL/6J mice were genetically engineered using CRISPR-Cas9 genomic editing to encode human DPP4 mutations (288L and 330R; "288/330^{+/+}") as previously described (see, Cockrell et al., "A mouse model for MERS coronavirus-induced acute respiratory distress syndrome." Nature Microbiology. 2:16226, 2016, which is incorporated by reference herein). 288/330^{+/+} mice were vaccinated with 0.1 µg MERS-CoV S-2P or PBS, with Sigma Adjuvant System at weeks 0 and 3. Four weeks following final vaccination, mice were challenged with a lethal dose of mouse-adapted MERS virus and monitored for survival and weight loss.

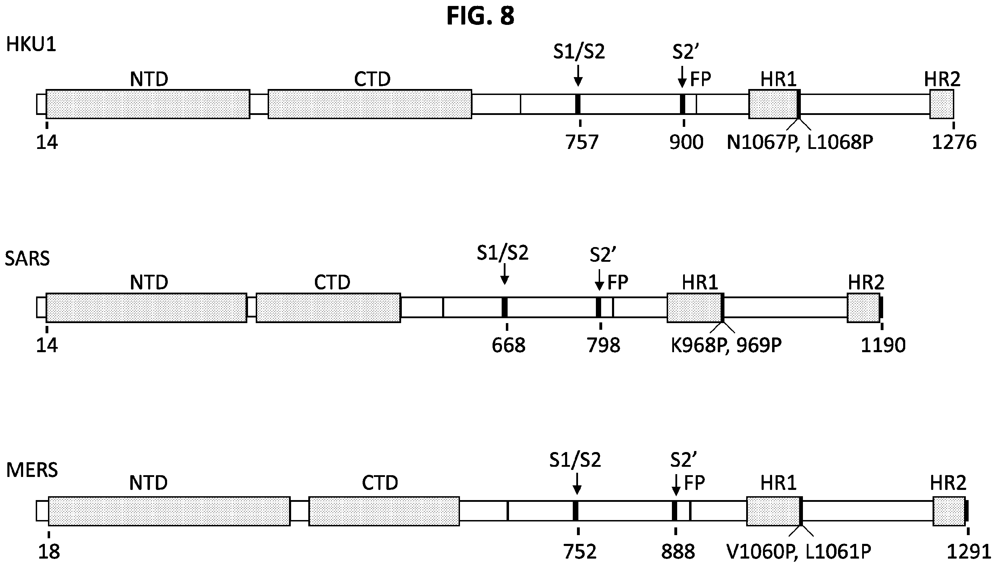

**FIG. 8** illustrates the structural domains of the HKU1-CoV, SARS-CoV, and MERS-CoV S proteins, as well as positioning of double proline substitutions to stabilize these proteins in the prefusion conformation.

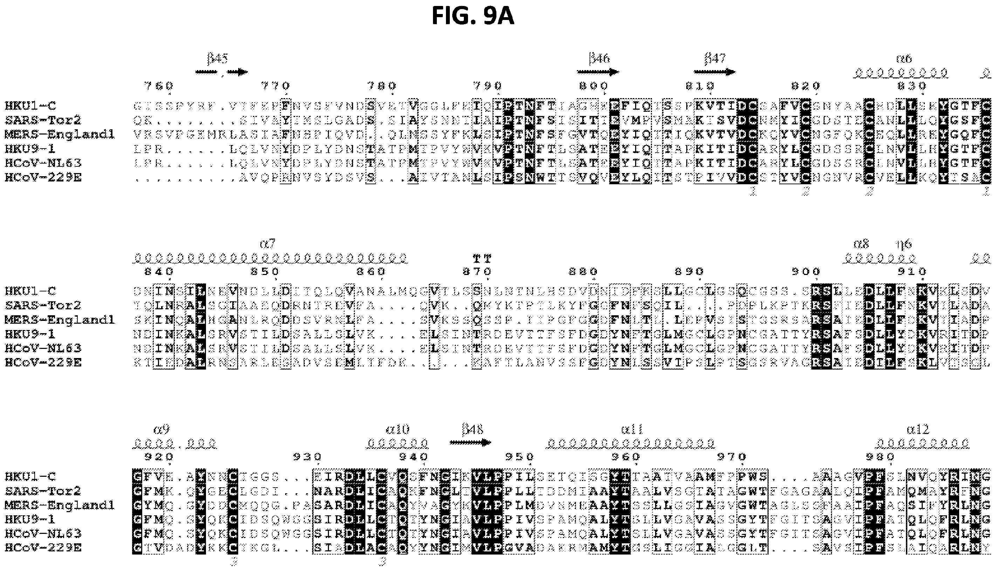

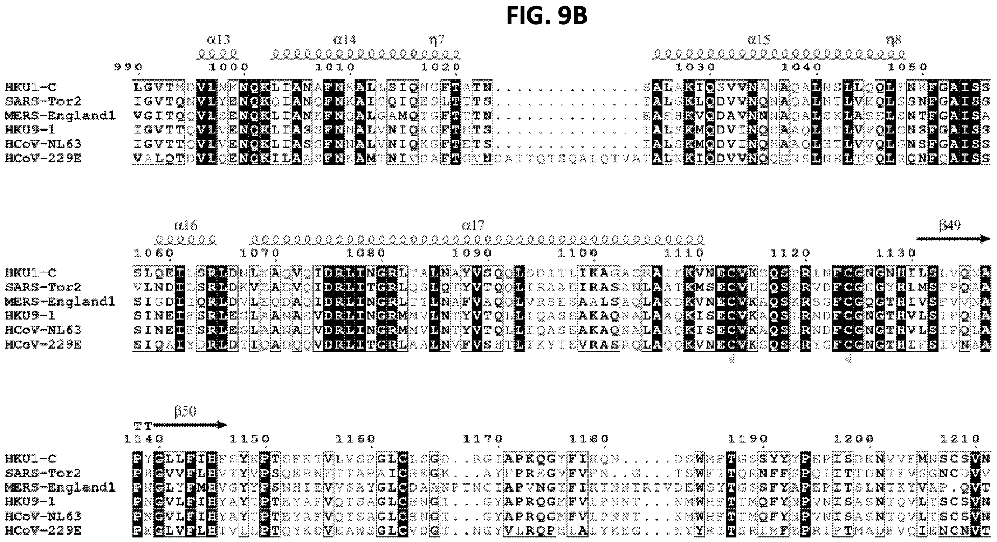

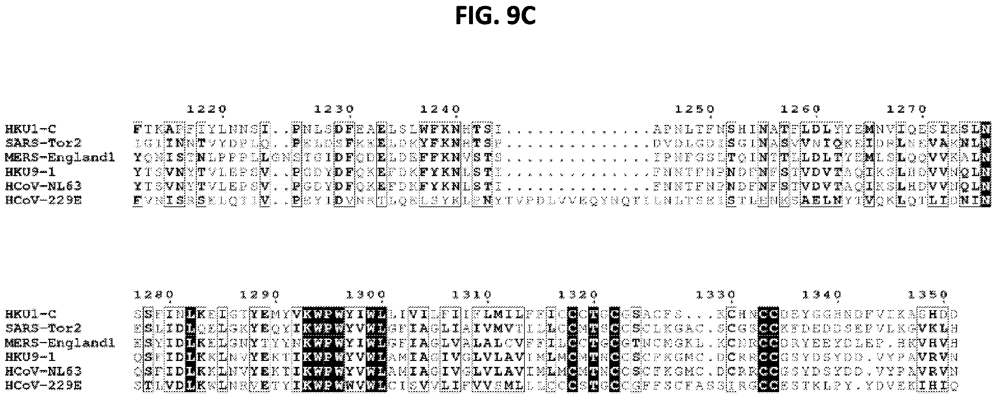

**FIGs 9A-9C** show a sequence alignment of the S2 subunit of the HKU1-CoV (SEQ ID NO: 8), SARS-CoV (SEQ ID NO: 6), MERS-CoV (SEQ ID NO: 1), HKU9-CoV (SEQ ID NO: 12), NL63-CoV (SEQ ID NO: 18), and 229E-CoV (SEQ ID NO: 20) S proteins, showing relevant sequence homology.

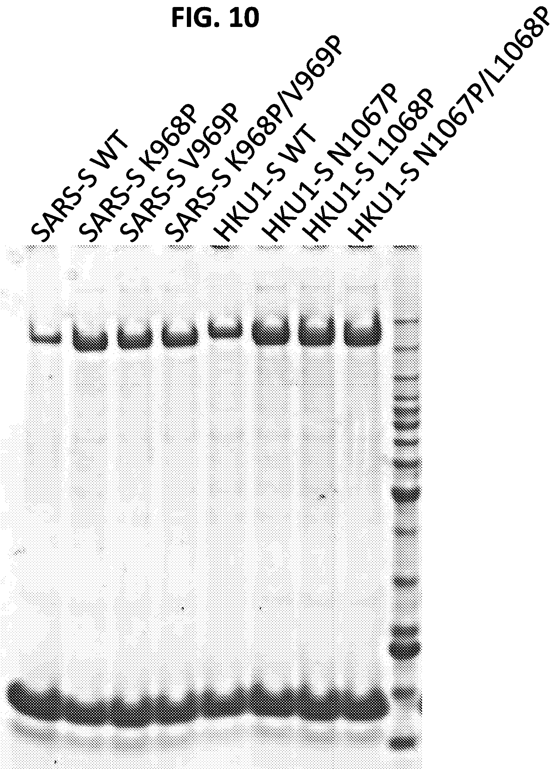

**FIG. 10** shows a Coomassie-stained polyacrylamide gel illustrating that introduction of proline substitutions in the SARS-CoV (K968P and V969P substitutions, SARS-S-2P) and HKU1-CoV (N1067P and L1068P substitutions, HKU1-S-2P) S ectodomains at the locations corresponding to the MERS-CoV S V1060P and L1061P substitutions boosts the expression of the SARS-CoV and HKU1-CoV S ectodomains.

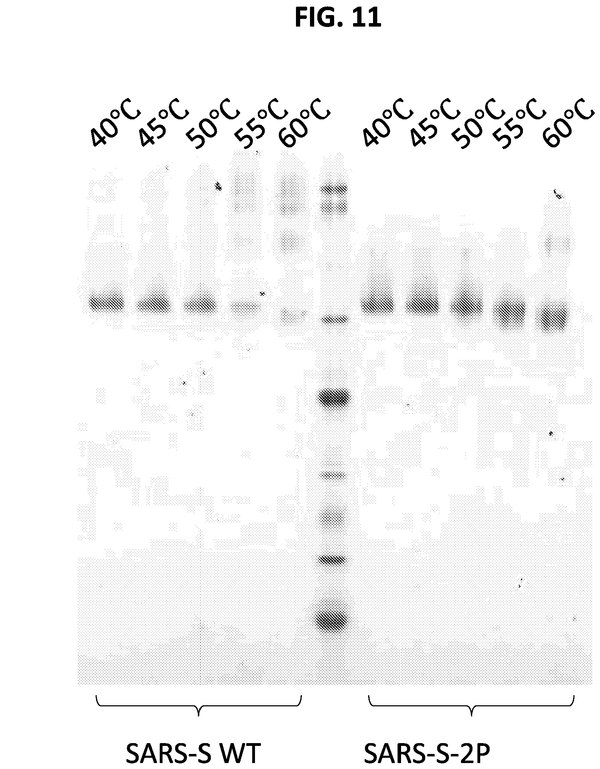

**FIG. 11** shows a Coomassie-stained polyacrylamide gel illustrating that the SARS-CoV S ectodomain with K968P and V969P substitutions (SARS-S-2P) has higher thermal stability than corresponding SARS-CoV S ectodomain having native sequence (SARS-S-WT).

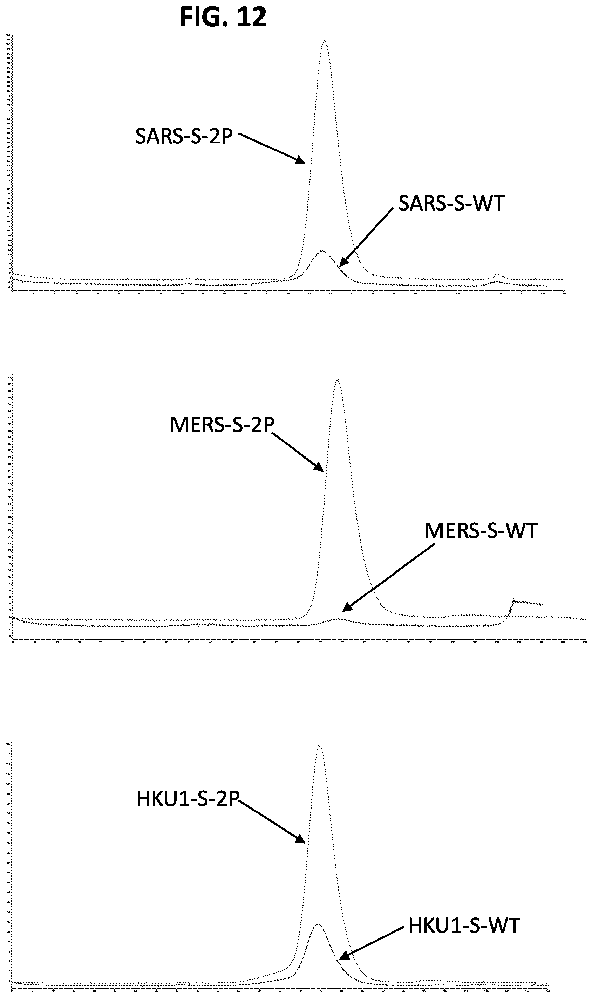

**FIG. 12** shows a set of graphs illustrating gel chromatography results of purified SARS-CoV, MERS-CoV, and HKU1-CoV S ectodomains having native (S-WT) sequence or double proline substitutions noted above (S-2P).

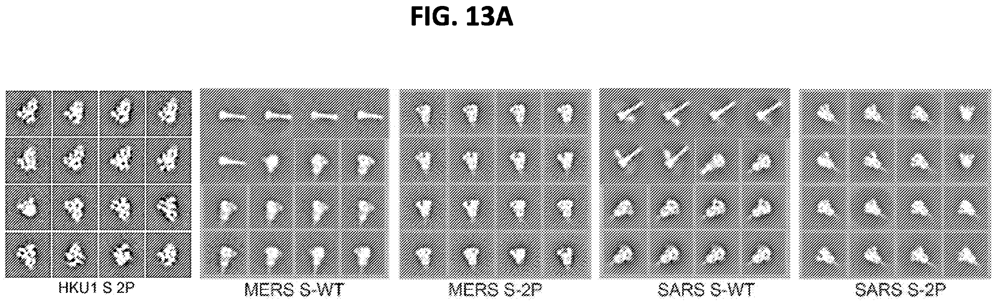

**FIGs. 13A-13C** show images of negative-stain electron microscopy of purified ectodomain trimers of MERS-CoV S 2P (V1060P and L1061P, SEQ ID NO: 28), SARS-CoV S 2P (K968P and V969P, SEQ ID NO: 30), HKU1-CoV S 2P (N1067P and L1068P, SEQ ID NO: 31), OC43-CoV S 2P (A1079P and L1080P, SEQ ID NO: 33), WIV1-CoV S 2P (K969P and V970P, SEQ ID NO: 34), PEDV-CoV S 2P (I1076P and L1077P, SEQ ID NO: 40), 229E S-2P (I869P and I870P, SEQ ID NO: 37), and SDCV 2-2P. Each of these ectodomain trimers was purified as a single peak and formed trimers in the typical prefusion conformation.

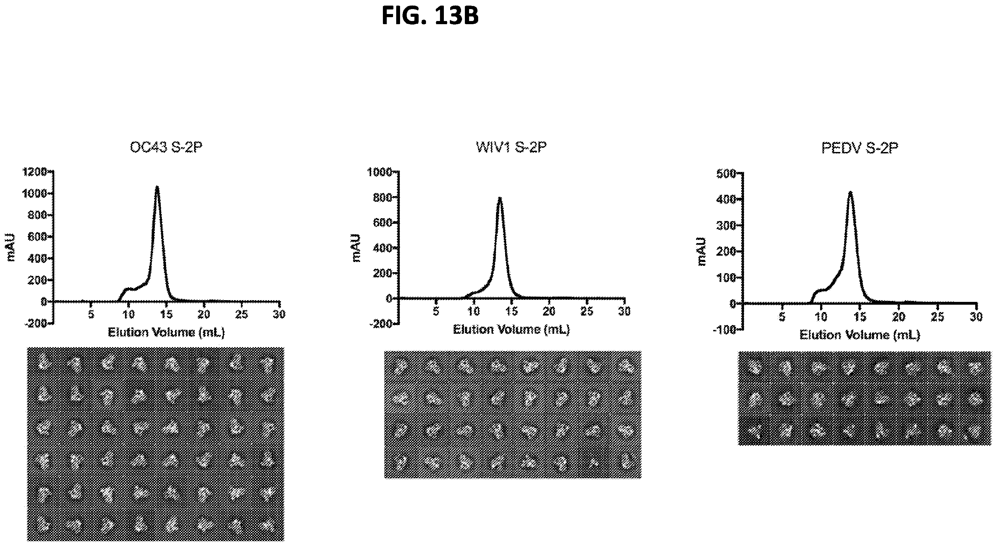

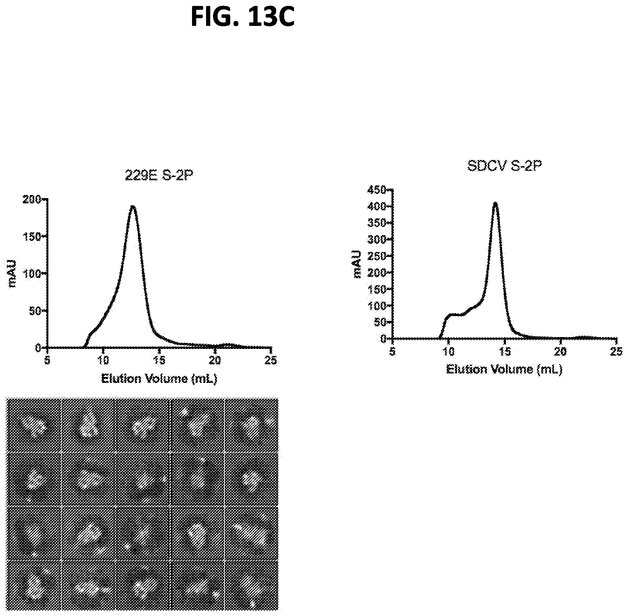

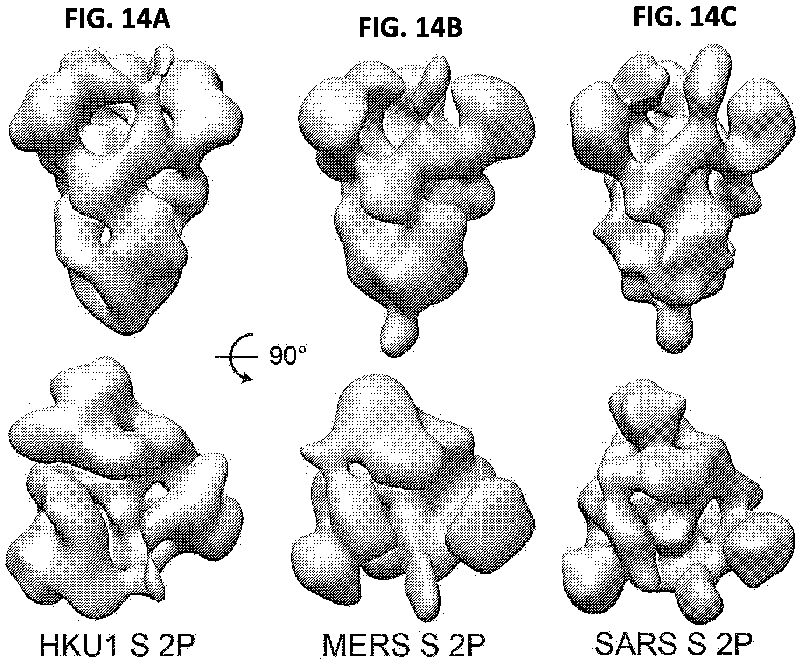

**FIGs. 14A-14G** show low-resolution negative-stain reconstructions of S trimer constructs from (14A) HKU1-CoV S 2P ectodomain trimer, (14B) MERS-CoV S 2P ectodomain trimer, (14C) SARS-CoV S 2P ectodomain trimer, (14D) OC43 S-2P ectodomain trimer, (14E) W1V1-CoV S 2P ectodomain trimer, (14F) PEDV-CoV S 2P ectodomain trimer, and (14G) 229E-CoV S 2P ectodomain trimer that were obtained from the negative-stain electron microscopy data shown in FIG. 13. The particles all formed homogeneous trimeric spike protein structures.

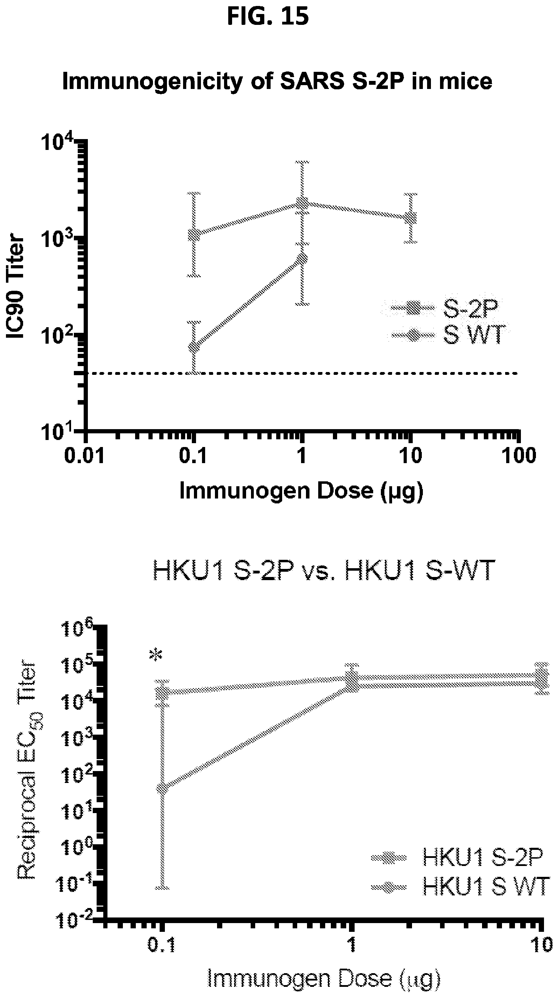

**FIG. 15** is a graph showing results of immunogenicity assays of HKU1-CoV S 2P ectodomain trimer and SARS S-2P ectodomain trimer in mice. Reciprocal serum IC₉₀ neutralizing activity against autologous pseudotyped lentivirus reporter (SARS Urbani for the SARS immunization) plotted against vaccine dose. The geometric mean IC₉₀ titer (GMT) of each group is represented by symbols. Error bars represent geometric SDs. The limit of detection for the assay is represented by dotted lines; for sera below the limit of detection a reciprocal IC₉₀ titer of 10 was assigned.

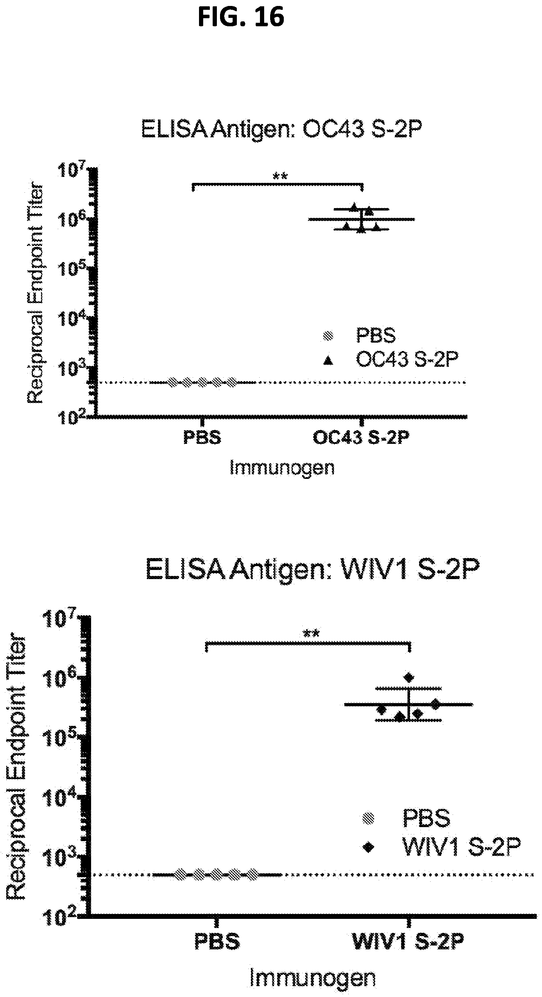

**FIG. 16** shows results from immunogenicity assays in mice using the OC43-CoV S-2P and WIV1-CoV S-2P ectodomain trimer immunogens. BALB/c mice were vaccinated with 1 µg of OC43 S-2P ectodomain trimer or WIV1-CoV S-2P ectodomain trimer, with Sigma Adjuvant System at weeks 0 and 3. Two weeks following final vaccination, mice were bled for antibody analysis. Binding antibody titers to OC43 S-2P ectodomain trimer or WIV1-CoV S-2P ectodomain trimer were measured by ELISA. The geometric mean titer (GMT) and geometric SDs of each group are represented. The dotted line represents the assay limit of detection. ** denotes p-value < 0.01, determined by Mann-Whitney t-test.

### SEQUENCE LISTING

The nucleic and amino acid sequences listed in the accompanying sequence listing are shown using standard letter abbreviations for nucleotide bases, and three letter code for amino acids, as defined in 37 C.F.R. 1.822. Only one strand of each nucleic acid sequence is shown, but the complementary strand is understood as included by any reference to the displayed strand. The Sequence Listing is submitted as an ASCII text file in the form of the file named "Sequence.txt" (~404 kb), which was created on October 24, 2017, and which is incorporated by reference herein.

### DETAILED DESCRIPTION

Past efforts to develop coronavirus vaccines have used whole-inactivated virus, live-attenuated virus, recombinant protein subunit, or genetic approaches (Graham et al., Nature reviews. Microbiology 11, 836, 2013). This disclosure provides CoV Spike glycoprotein (S) ectodomain trimers that are stabilized in the prefusion conformation and which are shown to elicit a neutralizing immune response in animal models.

### I. Summary of Terms

Unless otherwise noted, technical terms are used according to conventional usage. Definitions of common terms in molecular biology may be found in Benjamin Lewin, Genes X, published by Jones & Bartlett Publishers, 2009; and Meyers et al. (eds.), The Encyclopedia of Cell Biology and Molecular Medicine, published by Wiley-VCH in 16 volumes, 2008; and other similar references.

As used herein, the singular forms "a," "an," and "the," refer to both the singular as well as plural, unless the context clearly indicates otherwise. For example, the term "an antigen" includes single or plural antigens and can be considered equivalent to the phrase "at least one antigen." As used herein, the term "comprises" means "includes." It is further to be understood that any and all base sizes or amino acid sizes, and all molecular weight or molecular mass values, given for nucleic acids or polypeptides are approximate, and are provided for descriptive purposes, unless otherwise indicated. Although many methods and materials similar or equivalent to those described herein can be used, particular suitable methods and materials are described herein. In case of conflict, the present specification, including explanations of terms, will control. In addition, the materials, methods, and examples are illustrative only and not intended to be limiting. To facilitate review of the various embodiments, the following explanations of terms are provided:
**Adjuvant:** A vehicle used to enhance antigenicity. In some embodiments, an adjuvant can include a suspension of minerals (alum, aluminum hydroxide, or phosphate) on which antigen is adsorbed; or water-in-oil emulsion, for example, in which antigen solution is emulsified in mineral oil (Freund incomplete adjuvant), sometimes with the inclusion of killed mycobacteria (Freund's complete adjuvant) to further enhance antigenicity (inhibits degradation of antigen and/or causes influx of macrophages). In some embodiments, the adjuvant used in a disclosed immunogenic composition is a combination of lecithin and carbomer homopolymer (such as the ADJUPLEX^{™} adjuvant available from Advanced BioAdjuvants, LLC, see also Wegmann, Clin Vaccine Immunol, 22(9): 1004-1012, 2015). Additional adjuvants for use in the disclosed immunogenic compositions include the QS21 purified plant extract, Matrix M, AS01, MF59, and ALFQ adjuvants. Immunostimulatory oligonucleotides (such as those including a CpG motif) can also be used as adjuvants. Adjuvants include biological molecules (a "biological adjuvant"), such as costimulatory molecules. Exemplary adjuvants include IL-2, RANTES, GM-CSF, TNF-α, IFN-y, G-CSF, LFA-3, CD72, B7-1, B7-2, OX-40L, 4-1BBL and toll-like receptor (TLR) agonists, such as TLR-9 agonists. Additional description of adjuvants can be found, for example, in Singh (ed.) Vaccine Adjuvants and Delivery Systems. Wiley-Interscience, 2007). Adjuvants can be used in combination with the disclosed immunogens.

**Administration:** The introduction of an agent, such as a disclosed immunogen, into a subject by a chosen route. Administration can be local or systemic. For example, if the chosen route is intranasal, the agent (such as an immunogen comprising a recombinant coronavirus S ectodomain trimer stabilized in a prefusion conformation) is administered by introducing the composition into the nasal passages of the subject. Exemplary routes of administration include, but are not limited to, oral, injection (such as subcutaneous, intramuscular, intradermal, intraperitoneal, and intravenous), sublingual, rectal, transdermal (for example, topical), intranasal, vaginal, and inhalation routes.

**Amino acid substitution:** The replacement of one amino acid in a polypeptide with a different amino acid.

**Antibody:** An immunoglobulin, antigen-binding fragment, or derivative thereof, that specifically binds and recognizes an analyte (antigen) such as a coronavirus S protein, an antigenic fragment thereof, or a dimer or multimer of the antigen. The term "antibody" is used herein in the broadest sense and encompasses various antibody structures, including but not limited to monoclonal antibodies, polyclonal antibodies, multispecific antibodies (*e.g*., bispecific antibodies), and antibody fragments, so long as they exhibit the desired antigen-binding activity. Non-limiting examples of antibodies include, for example, intact immunoglobulins and variants and fragments thereof that retain binding affinity for the antigen. Examples of antibody fragments include but are not limited to Fv, Fab, Fab', Fab'-SH, F(ab')₂; diabodies; linear antibodies; single-chain antibody molecules (*e.g*. scFv); and multispecific antibodies formed from antibody fragments. Antibody fragments include antigen binding fragments either produced by the modification of whole antibodies or those synthesized *de novo* using recombinant DNA methodologies (see, *e.g.,* Kontermann and Dubel (Ed), Antibody Engineering, Vols. 1-2, 2nd Ed., Springer Press, 2010).

**Carrier:** An immunogenic molecule to which an antigen can be linked. When linked to a carrier, the antigen may become more immunogenic. Carriers are chosen to increase the immunogenicity of the antigen and/or to elicit antibodies against the carrier which are diagnostically, analytically, and/or therapeutically beneficial. Useful carriers include polymeric carriers, which can be natural (for example, proteins from bacteria or viruses), semi-synthetic or synthetic materials containing one or more functional groups to which a reactant moiety can be attached.

**Cavity-filling amino acid substitution:** An amino acid substitution that fills a cavity within the protein core of a protein, such as a coronavirus S protein ectodomain. Cavities are essentially voids within a folded protein where amino acids or amino acid side chains are not present. In several embodiments, a cavity-filling amino acid substitution is introduced to fill a cavity present in the prefusion conformation of a coronavirus S ectodomain core that collapses *(e.g.,* has reduced volume) after transition to the postfusion conformation.

**Conservative variants:** "Conservative" amino acid substitutions are those substitutions that do not substantially affect or decrease a function of a protein, such as the ability of the protein to induce an immune response when administered to a subject. The term conservative variation also includes the use of a substituted amino acid in place of an unsubstituted parent amino acid. Furthermore, deletions or additions which alter, add or delete a single amino acid or a small percentage of amino acids (for instance less than 5%, in some embodiments less than 1%) in an encoded sequence are conservative variations where the alterations result in the substitution of an amino acid with a chemically similar amino acid.

The following six groups are examples of amino acids that are considered to be conservative substitutions for one another:
1) Alanine (A), Serine (S), Threonine (T);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V); and
6) Phenylalanine (F), Tyrosine (Y), Tryptophan (W).

Non-conservative substitutions are those that reduce an activity or function of the recombinant coronavirus S ectodomain trimer, such as the ability to induce an immune response when administered to a subject. For instance, if an amino acid residue is essential for a function of the protein, even an otherwise conservative substitution may disrupt that activity. Thus, a conservative substitution does not alter the basic function of a protein of interest.

**Control:** A reference standard. In some embodiments, the control is a negative control sample obtained from a healthy patient. In other embodiments, the control is a positive control sample obtained from a patient diagnosed with a coronavirus infection, such as MERS-CoV or SARS-CoV. In still other embodiments, the control is a historical control or standard reference value or range of values (such as a previously tested control sample, such as a group of patients infected with a coronavirus with known prognosis or outcome, or group of samples that represent baseline or normal values).

A difference between a test sample and a control can be an increase or conversely a decrease. The difference can be a qualitative difference or a quantitative difference, for example a statistically significant difference. In some examples, a difference is an increase or decrease, relative to a control, of at least about 5%, such as at least about 10%, at least about 20%, at least about 30%, at least about 40%, at least about 50%, at least about 60%, at least about 70%, at least about 80%, at least about 90%, at least about 100%, at least about 150%, at least about 200%, at least about 250%, at least about 300%, at least about 350%, at least about 400%, at least about 500%, or greater than 500%.

**Coronavirus:** A family of positive-sense, single-stranded RNA viruses that are known to cause severe respiratory illness. Viruses currently known to infect human from the coronavirus family are from the alphacoronavirus and betacoronavirus genera. Additionally, it is believed that the gammacoronavirus and deltacoronavirus genera may infect humans in the future.

Non-limiting examples of betacoronaviruses include Middle East respiratory syndrome coronavirus (MERS-CoV), Severe Acute Respiratory Syndrome coronavirus (SARS-CoV), Human coronavirus HKU1 (HKU1-CoV), Human coronavirus OC43 (OC43-CoV), Murine Hepatitis Virus (MHV-CoV), Bat SARS-like coronavirus WIV1 (WIV1-CoV), and Human coronavirus HKU9 (HKU9-CoV). Non-limiting examples of alphacoronaviruses include human coronavirus 229E (229E-CoV), human coronavirus NL63 (NL63-CoV), porcine epidemic diarrhea virus (PEDV), and Transmissible gastroenteritis coronavirus (TGEV). A non-limiting example of a deltacoronaviruses is the Swine Delta Coronavirus (SDCV). Exemplary sequences of the ectodomains of S proteins from these viruses are provided herein.

The viral genome is capped, polyadenylated, and covered with nucleocapsid proteins. The coronavirus virion includes a viral envelope containing type I fusion glycoproteins referred to as the spike (S) protein. Most coronaviruses have a common genome organization with the replicase gene included in the 5'-portion of the genome, and structural genes included in the 3'-portion of the genome.

**Coronavirus Spike (S) protein:** A class I fusion glycoprotein initially synthesized as a precursor protein. Individual precursor S polypeptides form a homotrimer and undergo glycosylation within the Golgi apparatus as well as processing to remove the signal peptide, and cleavage by a cellular protease to generate separate S1 and S2 polypeptide chains, which remain associated as S1/S2 protomers within the homotrimer and is therefore a trimer of heterodimers. The S1 subunit is distal to the virus membrane and contains the receptor-binding domain (RBD) that mediates virus attachment to its host receptor. The S2 subunit contains fusion protein machinery, such as the fusion peptide, two heptad-repeat sequences (HR1 and HR2) and a central helix typical of fusion glycoproteins, a transmembrane domain, and the cytosolic tail domain.

**Coronavirus Spike (S) protein prefusion conformation:** A structural conformation adopted by the ectodomain of the coronavirus S protein following processing into a mature coronavirus S protein in the secretory system, and prior to triggering of the fusogenic event that leads to transition of coronavirus S to the postfusion conformation. The three-dimensional structure of an exemplary coronavirus S protein (HKU1-CoV) in a prefusion conformation is disclosed herein (see Example 1) and provided in Kirchdoerfer et al., "Pre-fusion structure of a human coronavirus spike protein," Nature, 531: 118-121, 2016 (incorporated by reference herein).

A coronavirus S ectodomain trimer "stabilized in a prefusion conformation" comprises one or more amino acid substitutions, deletions, or insertions compared to a native coronavirus S sequence that provide for increased retention of the prefusion conformation compared to coronavirus S ectodomain trimers formed from a corresponding native coronavirus S sequence. The "stabilization" of the prefusion conformation by the one or more amino acid substitutions, deletions, or insertions can be, for example, energetic stabilization (for example, reducing the energy of the prefusion conformation relative to the post-fusion open conformation) and/or kinetic stabilization (for example, reducing the rate of transition from the prefusion conformation to the postfusion conformation). Additionally, stabilization of the coronavirus S ectodomain trimer in the prefusion conformation can include an increase in resistance to denaturation compared to a corresponding native coronavirus S sequence. Methods of determining if a coronavirus S ectodomain trimer is in the prefusion conformation are provided herein, and include (but are not limited to) negative-stain electron microscopy and antibody binding assays using a prefusion-conformation-specific antibody.

**Degenerate variant:** In the context of the present disclosure, a "degenerate variant" refers to a polynucleotide encoding a polypeptide that includes a sequence that is degenerate as a result of the genetic code. There are 20 natural amino acids, most of which are specified by more than one codon. Therefore, all degenerate nucleotide sequences encoding a peptide are included as long as the amino acid sequence of the peptide encoded by the nucleotide sequence is unchanged.

**Effective amount:** An amount of agent, such as an immunogen, that is sufficient to elicit a desired response, such as an immune response in a subject. It is understood that to obtain a protective immune response against an antigen of interest can require multiple administrations of a disclosed immunogen, and/or administration of a disclosed immunogen as the "prime" in a prime boost protocol wherein the boost immunogen can be different from the prime immunogen. Accordingly, an effective amount of a disclosed immunogen can be the amount of the immunogen sufficient to elicit a priming immune response in a subject that can be subsequently boosted with the same or a different immunogen to elicit a protective immune response.

In one example, a desired response is to inhibit or reduce or prevent CoV (such as MERS-CoV) infection. The CoV infection does not need to be completely eliminated or reduced or prevented for the method to be effective. For example, administration of an effective amount of the immunogen can induce an immune response that decreases the CoV infection (for example, as measured by infection of cells, or by number or percentage of subjects infected by the CoV) by a desired amount, for example by at least 50%, at least 60%, at least 70%, at least 80%, at least 90%, at least 95%, at least 98%, or even at least 100% (elimination or prevention of detectable CoV infection), as compared to a suitable control.

**Epitope:** An antigenic determinant. These are particular chemical groups or peptide sequences on a molecule that are antigenic, such that they elicit a specific immune response, for example, an epitope is the region of an antigen to which B and/or T cells respond. An antibody can bind to a particular antigenic epitope, such as an epitope on coronavirus S ectodomain, such as a MERS-CoV S ectodomain. Epitopes can be formed both from contiguous amino acids or noncontiguous amino acids juxtaposed by tertiary folding of a protein.

**Expression:** Transcription or translation of a nucleic acid sequence. For example, a gene is expressed when its DNA is transcribed into an RNA or RNA fragment, which in some examples is processed to become mRNA. A gene may also be expressed when its mRNA is translated into an amino acid sequence, such as a protein or a protein fragment. In a particular example, a heterologous gene is expressed when it is transcribed into an RNA. In another example, a heterologous gene is expressed when its RNA is translated into an amino acid sequence. The term "expression" is used herein to denote either transcription or translation. Regulation of expression can include controls on transcription, translation, RNA transport and processing, degradation of intermediary molecules such as mRNA, or through activation, inactivation, compartmentalization or degradation of specific protein molecules after they are produced.

**Expression Control Sequences:** Nucleic acid sequences that regulate the expression of a heterologous nucleic acid sequence to which it is operatively linked. Expression control sequences are operatively linked to a nucleic acid sequence when the expression control sequences control and regulate the transcription and, as appropriate, translation of the nucleic acid sequence. Thus expression control sequences can include appropriate promoters, enhancers, transcription terminators, a start codon (ATG) in front of a protein-encoding gene, splicing signal for introns, maintenance of the correct reading frame of that gene to permit proper translation of mRNA, and stop codons. The term "control sequences" is intended to include, at a minimum, components whose presence can influence expression, and can also include additional components whose presence is advantageous, for example, leader sequences and fusion partner sequences. Expression control sequences can include a promoter.

A promoter is a minimal sequence sufficient to direct transcription. Also included are those promoter elements which are sufficient to render promoter-dependent gene expression controllable for cell-type specific, tissue-specific, or inducible by external signals or agents; such elements may be located in the 5' or 3' regions of the gene. Both constitutive and inducible promoters are included (see for example, Bitter et al., Methods in Enzymology 153:516-544, 1987). For example, when cloning in bacterial systems, inducible promoters such as pL of bacteriophage lambda, plac, ptrp, ptac (ptrp-lac hybrid promoter) and the like may be used. In one embodiment, when cloning in mammalian cell systems, promoters derived from the genome of mammalian cells (such as metallothionein promoter) or from mammalian viruses (such as the retrovirus long terminal repeat; the adenovirus late promoter; the vaccinia virus 7.5K promoter) can be used. Promoters produced by recombinant DNA or synthetic techniques may also be used to provide for transcription of the nucleic acid sequences.

**Expression vector:** A vector comprising a recombinant polynucleotide comprising expression control sequences operatively linked to a nucleotide sequence to be expressed. An expression vector comprises sufficient cis- acting elements for expression; other elements for expression can be supplied by the host cell or in an in vitro expression system. Expression vectors include all those known in the art, such as cosmids, plasmids (*e.g.,* naked or contained in liposomes) and viruses (*e.g.,* lentiviruses, retroviruses, adenoviruses, and adeno-associated viruses) that incorporate the recombinant polynucleotide.

**Glycosylation site:** An amino acid sequence on the surface of a polypeptide, such as a protein, which accommodates the attachment of a glycan. An *N*-linked glycosylation site is triplet sequence of NX(S/T) in which N is asparagine, X is any residues except proline, and (S/T) is a serine or threonine residue. A glycan is a polysaccharide or oligosaccharide. Glycan may also be used to refer to the carbohydrate portion of a glycoconjugate, such as a glycoprotein, glycolipid, or a proteoglycan.

**Heterologous:** Originating from a different genetic source. A nucleic acid molecule that is heterologous to a cell originated from a genetic source other than the cell in which it is expressed. In one specific, non-limiting example, a heterologous nucleic acid molecule encoding a recombinant coronavirus S ectodomain is expressed in a cell, such as a mammalian cell. Methods for introducing a heterologous nucleic acid molecule in a cell or organism are well known in the art, for example transformation with a nucleic acid, including electroporation, lipofection, particle gun acceleration, and homologous recombination.

**Ferritin:** A protein that stores iron and releases it in a controlled fashion. The protein is produced by almost all living organisms. Ferritin polypeptides assemble into a globular protein complex of 24 protein subunits, and each of the 24 subunits includes a single ferritin polypeptide. In some examples, ferritin is used to form a nanoparticle presenting antigens on its surface, for example, a coronavirus S ectodomain trimer.

**Host cells:** Cells in which a vector can be propagated and its DNA expressed. The cell may be prokaryotic or eukaryotic. The term also includes any progeny of the subject host cell. It is understood that all progeny may not be identical to the parental cell since there may be mutations that occur during replication. However, such progeny are included when the term "host cell" is used.

**Immune response:** A response of a cell of the immune system, such as a B cell, T cell, or monocyte, to a stimulus. In one embodiment, the response is specific for a particular antigen (an "antigen-specific response"). In one embodiment, an immune response is a T cell response, such as a CD4+ response or a CD8+ response. In another embodiment, the response is a B cell response, and results in the production of specific antibodies.

**Immunogen:** A compound, composition, or substance (for example, a recombinant coronavirus S ectodomain trimer) that can elicit an immune response in an animal, including compositions that are injected or absorbed into an animal. Administration of an immunogen to a subject can lead to protective immunity against a pathogen of interest.

**Immunogenic composition:** A composition comprising a disclosed recombinant coronavirus S ectodomain trimer that induces a measurable CTL response against the coronavirus, or induces a measurable B cell response (such as production of antibodies) against the coronavirus, when administered to a subject. It further refers to isolated nucleic acid molecules and vectors encoding a protomer of a disclosed recombinant coronavirus S ectodomain trimer that can be used to express the protomer (and thus be used to elicit an immune response against recombinant coronavirus S ectodomain trimer). For *in vivo* use, the immunogenic composition will typically include the recombinant coronavirus S ectodomain trimer or a nucleic acid molecule encoding a protomer of the recombinant coronavirus S ectodomain trimer in a pharmaceutically acceptable carrier and may also include other agents, such as an adjuvant.

**Inhibiting or treating a disease:** Inhibiting the full development of a disease or condition, for example, in a subject who is at risk for a disease such as a CoV infection. "Treatment" refers to a therapeutic intervention that ameliorates a sign or symptom of a disease or pathological condition after it has begun to develop. The term "ameliorating," with reference to a disease or pathological condition, refers to any observable beneficial effect of the treatment. Inhibiting a disease can include preventing or reducing the risk of the disease, such as preventing or reducing the risk of viral infection. The beneficial effect can be evidenced, for example, by a delayed onset of clinical symptoms of the disease in a susceptible subject, a reduction in severity of some or all clinical symptoms of the disease, a slower progression of the disease, a reduction in the viral load, an improvement in the overall health or well-being of the subject, or by other parameters that are specific to the particular disease. A "prophylactic" treatment is a treatment administered to a subject who does not exhibit signs of a disease or exhibits only early signs for the purpose of decreasing the risk of developing pathology.

**Isolated:** An "isolated" biological component has been substantially separated or purified away from other biological components, such as other biological components in which the component naturally occurs, such as other chromosomal and extrachromosomal DNA, RNA, and proteins. Proteins, peptides, nucleic acids, and viruses that have been "isolated" include those purified by standard purification methods. Isolated does not require absolute purity, and can include protein, peptide, nucleic acid, or virus molecules that are at least 50% isolated, such as at least 75%, 80%, 90%, 95%, 98%, 99%, or even 99.9% isolated.

**Linker and Linked:** A bi-functional molecule that can be used to link two molecules into one contiguous molecule. Non-limiting examples of peptide linkers include glycine-serine peptide linkers. Unless context indicates otherwise, reference to "linking" a first polypeptide and a second polypeptide, or to two polypeptides "linked" together, or to a first polypeptide having a "linkage" to a second polypeptide, refers to covalent linkage by peptide bond (for example via a peptide linker) such that the first and second polypeptides form a contiguous polypeptide chain. If a peptide linker is involved, the covalent linkage of the first and second polypeptides can be to the N- and C-termini of the peptide linker. Typically, such linkage is accomplished using molecular biology techniques to genetically manipulate DNA encoding the first polypeptide linked to the second polypeptide by the peptide linker.

**Native protein, sequence, or disulfide bond:** A polypeptide, sequence or disulfide bond that has not been modified, for example, by selective mutation. For example, selective mutation to focus the antigenicity of the antigen to a target epitope, or to introduce a disulfide bond into a protein that does not occur in the native protein. Native protein or native sequence are also referred to as wild-type protein or wild-type sequence. A non-native disulfide bond is a disulfide bond that is not present in a native protein, for example, a disulfide bond that forms in a protein due to introduction of one or more cysteine residues into the protein by genetic engineering.

**Nucleic acid molecule:** A polymeric form of nucleotides, which may include both sense and antisense strands of RNA, cDNA, genomic DNA, and synthetic forms and mixed polymers of the above. A nucleotide refers to a ribonucleotide, deoxynucleotide or a modified form of either type of nucleotide. The term "nucleic acid molecule" as used herein is synonymous with "nucleic acid" and "polynucleotide." A nucleic acid molecule is usually at least 10 bases in length, unless otherwise specified. The term includes single- and double-stranded forms of DNA. A polynucleotide may include either or both naturally occurring and modified nucleotides linked together by naturally occurring and/or non-naturally occurring nucleotide linkages. "cDNA" refers to a DNA that is complementary or identical to an mRNA, in either single stranded or double stranded form. "Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Generally, operably linked nucleic acid sequences are contiguous and, where necessary to join two protein-coding regions, in the same reading frame.

**Pharmaceutically acceptable carriers:** The pharmaceutically acceptable carriers of use are conventional. Remington's Pharmaceutical Sciences, by E. W. Martin, Mack Publishing Co., Easton, PA, 19th Edition, 1995, describes compositions and formulations suitable for pharmaceutical delivery of the disclosed immunogens.

In general, the nature of the carrier will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions (such as immunogenic compositions) to be administered can contain minor amounts of non-toxic auxiliary substances, such as wetting or emulsifying agents, preservatives, and pH buffering agents and the like, for example sodium acetate or sorbitan monolaurate. In particular embodiments, suitable for administration to a subject the carrier may be sterile, and/or suspended or otherwise contained in a unit dosage form containing one or more measured doses of the composition suitable to induce the desired immune response. It may also be accompanied by medications for its use for treatment purposes. The unit dosage form may be, for example, in a sealed vial that contains sterile contents or a syringe for injection into a subject, or lyophilized for subsequent solubilization and administration or in a solid or controlled release dosage.

**Polypeptide:** Any chain of amino acids, regardless of length or post-translational modification (e.g., glycosylation or phosphorylation). "Polypeptide" applies to amino acid polymers including naturally occurring amino acid polymers and non-naturally occurring amino acid polymer as well as in which one or more amino acid residue is a non-natural amino acid, for example, an artificial chemical mimetic of a corresponding naturally occurring amino acid. A "residue" refers to an amino acid or amino acid mimetic incorporated in a polypeptide by an amide bond or amide bond mimetic. A polypeptide has an amino terminal (N-terminal) end and a carboxy terminal (C-terminal) end. "Polypeptide" is used interchangeably with peptide or protein, and is used herein to refer to a polymer of amino acid residues.

**Prime-boost vaccination:** An immunotherapy including administration of a first immunogenic composition (the primary vaccine) followed by administration of a second immunogenic composition (the booster vaccine) to a subject to induce an immune response. The priming vaccine and/or the booster vaccine include a vector (such as a viral vector, RNA, or DNA vector) expressing the antigen to which the immune response is directed. The booster vaccine is administered to the subject after the priming vaccine; a suitable time interval between administration of the priming vaccine and the booster vaccine, and examples of such timeframes are disclosed herein. In some embodiments, the priming vaccine, the booster vaccine, or both primer vaccine and the booster vaccine additionally include an adjuvant. In one non-limiting example, the priming vaccine is a DNA-based vaccine (or other vaccine based on gene delivery), and the booster vaccine is a protein subunit or protein nanoparticle based vaccine.

**Protein nanoparticle:** A multi-subunit, self-assembling, protein-based polyhedron shaped structure. The subunits are each composed of proteins (for example a glycosylated polypeptide), and, optionally of single or multiple features of the following: nucleic acids, prosthetic groups, organic and inorganic compounds. In some embodiments, protomers of the disclosed trimeric spike proteins can be fused to the subunits of the protein nanoparticles to provide multiple copies of the trimeric spike on each protein nanoparticle. Non-limiting examples of protein nanoparticles include ferritin nanoparticles (see, *e.g.,* Zhang, Y. Int. J. Mol. Sci., 12:5406-5421, 2011, incorporated by reference herein), encapsulin nanoparticles (see, *e.g.,* Sutter et al., Nature Struct. and Mol. Biol., 15:939-947, 2008, incorporated by reference herein), Sulfur Oxygenase Reductase (SOR) nanoparticles (see, *e.g.,* Urich et al., Science, 311:996-1000, 2006, incorporated by reference herein), lumazine synthase nanoparticles (see, *e.g.,* Zhang et al., J. Mol. Biol., 306: 1099-1114, 2001), and pyruvate dehydrogenase nanoparticles (see, *e.g.,* Izard et al., PNAS 96: 1240-1245, 1999, incorporated by reference herein). Ferritin, encapsulin, SOR, lumazine synthase, and pyruvate dehydrogenase are monomeric proteins that self-assemble into a globular protein complexes that in some cases consists of 24, 60, 24, 60, and 60 protein subunits, respectively. Additional protein nanoparticle structures are described by Heinze et al., J Phys Chem B., 120(26):5945-52, 2016; Hsia et al., Nature, 535(7610):136-9, 2016; and King et al., Nature, 510(7503):103-8, 2014; each of which is incorporated by reference herein.

**Recombinant:** A recombinant nucleic acid molecule is one that has a sequence that is not naturally occurring, for example, includes one or more nucleic acid substitutions, deletions or insertions, and/or has a sequence that is made by an artificial combination of two otherwise separated segments of sequence. This artificial combination can be accomplished by chemical synthesis or, more commonly, by the artificial manipulation of isolated segments of nucleic acids, for example, by genetic engineering techniques. A recombinant virus is one that includes a genome that includes a recombinant nucleic acid molecule. A recombinant protein is one that has a sequence that is not naturally occurring or has a sequence that is made by an artificial combination of two otherwise separated segments of sequence. In several embodiments, a recombinant protein is encoded by a heterologous (for example, recombinant) nucleic acid that has been introduced into a host cell, such as a bacterial or eukaryotic cell, or into the genome of a recombinant virus.

**Sequence identity:** The similarity between amino acid sequences is expressed in terms of the similarity between the sequences, otherwise referred to as sequence identity. Sequence identity is frequently measured in terms of percentage identity; the higher the percentage, the more similar the two sequences are. Homologs, orthologs, or variants of a polypeptide will possess a relatively high degree of sequence identity when aligned using standard methods.

Methods of alignment of sequences for comparison are well known in the art. Various programs and alignment algorithms are described in: Smith & Waterman, Adv. Appl. Math. 2:482, 1981; Needleman & Wunsch, J. Mol. Biol. 48:443, 1970; Pearson & Lipman, Proc. Natl. Acad. Sci. USA 85:2444, 1988; Higgins & Sharp, Gene, 73:237-44, 1988; Higgins & Sharp, CABIOS 5:151-3, 1989; Corpet et al., Nuc. Acids Res. 16:10881-90, 1988; Huang et al. Computer Appls. in the Biosciences 8, 155-65, 1992; and Pearson et al., Meth. Mol. Bio. 24:307-31, 1994. Altschul et al., J. Mol. Biol. 215:403-10, 1990, presents a detailed consideration of sequence alignment methods and homology calculations.

Homologs and variants of a polypeptide (such as a coronavirus S ectodomain) are typically characterized by possession of at least about 75%, for example at least about 80%, 85%, 90%, 91%, 92%, 93%, 94%, 95%, 96%, 97%, 98% or 99% sequence identity counted over the full length alignment with the amino acid sequence of interest. Proteins with even greater similarity to the reference sequences will show increasing percentage identities when assessed by this method, such as at least 80%, at least 85%, at least 90%, at least 95%, at least 98%, or at least 99% sequence identity. When less than the entire sequence is being compared for sequence identity, homologs and variants will typically possess at least 80% sequence identity over short windows of 10-20 amino acids, and may possess sequence identities of at least 85% or at least 90% or 95% depending on their similarity to the reference sequence. Methods for determining sequence identity over such short windows are available at the NCBI website on the internet.

As used herein, reference to "at least 90% identity" or similar language refers to "at least 90%, at least 91%, at least 92%, at least 93%, at least 94%, at least 95%, at least 96%, at least 97%, at least 98%, at least 99%, or even 100% identity" to a specified reference sequence.

**Signal Peptide:** A short amino acid sequence (*e.g.,* approximately 10-35 amino acids in length) that directs newly synthesized secretory or membrane proteins to and through membranes (for example, the endoplasmic reticulum membrane). Signal peptides are typically located at the N-terminus of a polypeptide and are removed by signal peptidases. Signal peptide sequences typically contain three common structural features: an N-terminal polar basic region (n-region), a hydrophobic core, and a hydrophilic c-region).

**Single chain coronavirus S ectodomain:** A recombinant coronavirus S ectodomain including the coronavirus S₁ and S₂ proteins in a single contiguous polypeptide chain. Single chain coronavirus S ectodomain can trimerize to form a coronavirus S ectodomain trimer. A single coronavirus S ectodomain includes mutations to prevent protease cleavage at the S₁/S₂ cleavage site and the S₂' cleavage site in the S ectodomain. Therefore, when produced in cells, the S polypeptide is not cleaved into separate S₁ and S₂ polypeptide chains.

**Soluble protein:** A protein capable of dissolving in aqueous liquid at room temperature and remaining dissolved. The solubility of a protein may change depending on the concentration of the protein in the water-based liquid, the buffering condition of the liquid, the concentration of other solutes in the liquid, for example salt and protein concentrations, and the heat of the liquid. In several embodiments, a soluble protein is one that dissolves to a concentration of at least 0.5 mg/ml in phosphate buffered saline (pH 7.4) at room temperature and remains dissolved for at least 48 hours.

**Subject:** Living multi-cellular vertebrate organisms, a category that includes human and non-human mammals, such as non-human primates, pigs, camels, bats, sheep, cows, dogs, cats, rodents, and the like. In an example, a subject is a human. In a particular example, the subject is a camel or a bat. The subject can be a domestic animal (such as a dog or a cat) or a farm animal (such as a cow or a pig). In an additional example, a subject is selected that is in need of inhibiting of a coronavirus infection, such as a SARS-CoV or MERS-CoV infection. For example, the subject is either uninfected and at risk of the coronavirus infection or is infected and in need of treatment.

**T4 Fibritin trimerization domain:** Also referred to as a "foldon" domain, the T4 Fibritin trimerization domain comprises an amino acid sequence that naturally forms a trimeric structure. In some examples, a T4 Fibritin trimerization domain can be linked to the C-terminus of a disclosed recombinant coronavirus S protein ectodomain. In one example, a T4 Fibritin trimerization domain comprises the amino acid sequence set forth as (GYIPEAPRDGQAYVRKDGEWVLLSTF (SEQ ID NO: 22). In some embodiments, a protease cleavage site (such as a thrombin cleavage site) can be included between the C-terminus of the recombinant coronavirus ectodomain and the T4 Fibritin trimerization domain to facilitate removal of the trimerization domain as needed, for example, following expression and purification of the recombinant coronavirus S ectodomain.

**Transmembrane domain:** An amino acid sequence that inserts into a lipid bilayer, such as the lipid bilayer of a cell or virus or virus-like particle. A transmembrane domain can be used to anchor an antigen to a membrane. In some examples a transmembrane domain is a coronavirus S transmembrane domain, such as a MERS-CoV or SARS-CoV S transmembrane domain.

**Vaccine:** A pharmaceutical composition that induces a prophylactic or therapeutic immune response in a subject. In some cases, the immune response is a protective immune response. Typically, a vaccine induces an antigen-specific immune response to an antigen of a pathogen, for example a viral pathogen, or to a cellular constituent correlated with a pathological condition. A vaccine may include a polynucleotide (such as a nucleic acid encoding a disclosed antigen), a peptide or polypeptide (such as a disclosed antigen), a virus, a cell or one or more cellular constituents. In a non-limiting example, a vaccine induces an immune response that reduces the severity of the symptoms associated with a coronavirus infection (such as a SARS-CoV or MERS-CoV infection) and/or decreases the viral load compared to a control. In another non-limiting example, a vaccine induces an immune response that reduces and/or prevents a coronavirus infection (such as a SARS-CoV or MERS-CoV infection) compared to a control.

**Vector:** An entity containing a DNA or RNA molecule bearing a promoter(s) that is operationally linked to the coding sequence of an antigen(s) of interest and can express the coding sequence. Non-limiting examples include a naked or packaged (lipid and/or protein) DNA, a naked or packaged RNA, a subcomponent of a virus or bacterium or other microorganism that may be replication-incompetent, or a virus or bacterium or other microorganism that may be replication-competent. A vector is sometimes referred to as a construct. Recombinant DNA vectors are vectors having recombinant DNA. A vector can include nucleic acid sequences that permit it to replicate in a host cell, such as an origin of replication. A vector can also include one or more selectable marker genes and other genetic elements known in the art. Viral vectors are recombinant nucleic acid vectors having at least some nucleic acid sequences derived from one or more viruses.

**Virus-like particle (VLP):** A non-replicating, viral shell, derived from any of several viruses. VLPs are generally composed of one or more viral proteins, such as, but not limited to, those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art. The presence of VLPs following recombinant expression of viral proteins can be detected using conventional techniques known in the art, such as by electron microscopy, biophysical characterization, and the like. Further, VLPs can be isolated by known techniques, *e.g*., density gradient centrifugation and identified by characteristic density banding. See, for example, Baker et al. (1991) Biophys. J. 60:1445-1456; and Hagensee et al. (1994) J. Virol. 68:4503-4505; Vincente, J Invertebr Pathol., 2011; Schneider-Ohrum and Ross, Curr. Top. Microbiol. Immunol., 354: 53073, 2012).

### II. Immunogens

Disclosed herein are recombinant coronavirus (such as alphacoronavirus or betacoronavirus) S ectodomain trimers comprising protomers comprising one or more proline substitution(s). The proline substitutions inhibit a conformational change in the S protein from the prefusion conformation to the postfusion conformation, and therefore stabilize the S ectodomain trimer in the prefusion conformation. In some embodiments, the recombinant coronavirus (such as alphacoronavirus or betacoronavirus) S ectodomain trimer comprises protomers comprising one or more (such as two) proline substitutions at or near the boundary between a HR1 domain and a central helix domain of the protomers. In some embodiments, the one or more (such as two, for example two consecutive) proline substitutions that stabilize the protomers of the S ectodomain in the prefusion conformation are located between a position 15 amino acids N-terminal of a C-terminal residue of the HR1 and a position 5 amino acids C-terminal of a N-terminal residue of the central helix. Exemplary embodiments are shown to produce a superior immune response in an animal model compared to corresponding coronavirus S ectodomain trimers that are not stabilized in the prefusion conformation.

In some embodiments, the recombinant S ectodomain trimer comprises recombinant S ectodomain protomers from an alphacoronavirus, such as NL63-CoV or 229E-CoV, that have been mutated to include the one or more proline substitutions for stabilization in the prefusion conformation. In some embodiments, the recombinant S ectodomain trimers comprise S ectodomain protomers from a betacoronavirus, such as OC43-CoV, SARS-CoV, MERS-CoV, HKU1-CoV, WIV1-CoV, mouse hepatitis virus (MHV), or HKU9-CoV, that have been mutated to include the one or more proline substitutions for stabilization in the prefusion conformation. Additional description is provided below.

### A. MERS-CoV

In some embodiments, the immunogen comprises a recombinant MERS-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the MERS-CoV S ectodomain trimer in the prefusion conformation are located between residues 1050 to 1069 (such as between residues 1053 to 1063, or between residues 1058 to 1063) of the S ectodomain protomers in the trimer. In some embodiments, the MERS-CoV S ectodomain trimer is stabilized in the prefusion conformation by one or two of: L1058P, D1059P, V1060P, and L1061P substitutions in the S ectodomain protomers in the trimer. In some embodiments, the MERS-CoV S ectodomain trimer is stabilized in the prefusion conformation by V1060P and L1061P substitutions ("2P") in the S ectodomain protomers in the trimer. The amino acid numbering for MERS-CoV S proteins is with reference to the MERS-CoV S sequence provided as SEQ ID NO: 1.

In some embodiments, the recombinant MERS-CoV S ectodomain trimer stabilized in the prefusion conformation comprises protomers of single-chain S ectodomains comprising mutations to the S1/S2 and/or S2' protease cleavage sites to prevent protease cleavage at these sites. Non-limiting examples of such mutations include 748-RSVR-751 (residues 748-751 of SEQ ID NO: 1) to 748-ASVG-751 (residues 748-751 of SEQ ID NO: 3) substitutions to inhibit cleavage at the S1/S2 cleavage site, and 884-RSAR-887 (residues 884-887 of SEQ ID NO: 1) to 884-GSAG-887 (residues 884-887 of SEQ ID NO: 3) substitutions to inhibit cleavage at the S2' site.

In some embodiments, the recombinant MERS-CoV S ectodomain trimer comprising protomers stabilized in the prefusion conformation by the one or more proline substitutions (such as V1060P and L1061P substitutions) comprises additional modifications for stabilization in the prefusion conformation. In some embodiments, the recombinant MERS-CoV S ectodomain trimer comprising protomers stabilized in the prefusion conformation by the one or more proline substitutions (such as V1060P and L1061P substitutions) further comprises cavity filling substitutions to stabilize the S ectodomain the prefusion conformation, such as one of: N1072F and A1083I; N1072F and L1086F; N1072F and V10871; N1072F and E1090I; T1076F and A1083I; T1076F and L1086F; T1076F and V1087I; T1076F and E1090I; T1076I and A1083I; T1076I and L1086F; T1076I and V1087I; T1076I and E1090I; A1018V; or A1018I.

In some embodiments, the recombinant MERS-CoV S ectodomain trimer stabilized in the prefusion conformation by the one or more proline substitutions (such as V1060P and L1061P substitutions) further comprises a repacking substitution to stabilize the S ectodomain the prefusion conformation, such as one of: E793M and K1102F; E793M, K1102F, and H1138F; D1068M and R1069W; A1083L; A1083L and V1087I; A1083L, V1087, and E1090L; A834L and Q1084M; Q1066M; S454F; R921W; S612F and G1052F; or P476V, T477A, and R1057W.

In some embodiments, the recombinant MERS-CoV S ectodomain trimer stabilized in the prefusion conformation by the one or more proline substitutions (such as V1060P and L1061P substitutions) further comprises one of A1083S, E1090I, Q1097I, D1101F, or A653W to stabilize the S ectodomain the prefusion conformation.

In some embodiments, the recombinant MERS-CoV S ectodomain trimer stabilized in the prefusion conformation by the one or more proline substitutions (such as V1060P and L1061P substitutions) further comprises a non-native disulfide bond formed between cysteines introduced by one of: T63C and V631C; T63C and Q638C; Q733C and D940C; S676C and D910C; V1087C (which forms a disulfide bond with a cysteine present in the native sequence); A432C and L1058C; or A432C and D1059C to stabilize the S ectodomain the prefusion conformation.

In some embodiments, the recombinant MERS-CoV S ectodomain trimer stabilized in the prefusion conformation by the one or more proline substitutions (such as V1060P and L1061P substitutions) further comprises an additional proline substitution to stabilize the S ectodomain the prefusion conformation, such as one of: K801P; V802P; T803P; V804P; S919P; A920P; A968P; A969P; I970P; F972P; A973P; T1014P; N1042P; T1043P; F1044P; G1045P; A1046P; I1047P; or A1049P.

Any of the substitutions described above can be combined in the MERS-CoV S ectodomain trimer, as long as the trimer is stabilized in the prefusion conformation and can be used to generate a neutralizing immune response to MERS-CoV in a subject.

With reference to the MERS-CoV S protein sequence provided as SEQ ID NO: 1, the ectodomain of the MERS-CoV S protein includes about residues 18-1291. Residues 1-17 are the signal peptide, which is removed during cellular processing. The S1/S2 cleavage site is located at about position 751/752. The S2' cleavage site is located at about position 887/888. The HR1 is located at about residues 989-1057. The central helix is located at about residues 1062-1103. The HR2 is located at about 1246-1277. The C-terminal end of the S2 ectodomain is located at about residue 1291. In some embodiments, the protomers of the prefusion-stabilized MERS-CoV S ectodomain trimer can have a C-terminal residue (which can be linked to a trimerization domain, or a transmembrane domain, for example) of the C-terminal residue of the HR2 (e.g., position 1277), or the ectodomain (e.g., position 1291) or from one of positions 1277-1291. The position numbering of the S protein may vary between MERS-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary MERS-CoV S protein sequences are provided below. Any of the MERS-CoV S protein mutations (such as V1060P and L1061P, and/or modifications to generate a single chain) can be incorporated in the MERS-CoV S protein sequences.

An exemplary sequence of MERS-CoV S protein including the ectodomain and TM and CT domains) England1 strain is provided as SEQ ID NO: 1:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK
MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPRSVRSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSRSARSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDVLEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL
NTKYVAPQVTYQNISTNLPPPLLGNSTGIDFQDELDEFFKNVSTSIPNFGSLTQINTTLLDLTYEMLSLQQVVKALNESY
IDLKELGNYTYYNKWPWYIWLGFIAGLVALALCVFFILCCTGCGTNCMGKLKCNRCCDRYEEYDLEPHKVHVH

An exemplary sequence of MERS-CoV S ectodomain England1 strain including V1060P and L1061P substitutions is provided as SEQ ID NO: 2:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK

MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPRSVRSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSRSARSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDPPEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL
NTKYVAPQVTYQNISTNLPPPLLGNSTGIDFQDELDEFFKNVSTSIPNFGSLTQINTTLLDLTYEMLSLQQVVKALNESY
IDLKELGNYTY

An exemplary sequence of MERS-CoV S ectodomain England1 strain including V1060P and L1061P substitutions and 748-RSVR-751 (residues 748-751 of SEQ ID NO: 1) to 748-ASVG-751 (residues 748-751 of SEQ ID NO: 3) substitutions to remove the S1/S2 cleavage site is provided as SEQ ID NO: 3:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK
MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPASVGSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSRSARSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDPPEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL
NTKYVAPQVTYQNISTNLPPPLLGNSTGIDFQDELDEFFKNVSTSIPNFGSLTQINTTLLDLTYEMLSLQQVV
KALNESYIDLKELGNYTY

An exemplary sequence of MERS-CoV S ectodomain England1 strain including V1060P and L1061P substitutions and 748-RSVR-751 (residues 748-751 of SEQ ID NO: 1) to 748-ASVG-751 (residues 748-751 of SEQ ID NO: 3) and 884-RSAR-887 (residues 884-887 of SEQ ID NO: 1) to 884-GSAG-887 (residues 884-887 of SEQ ID NO: 3) substitutions to remove the S1/S2 cleavage site and the S2' cleavage site is provided as SEQ ID NO: 4:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK
MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPASVGSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSGSAGSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDPPEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL

NTKYVAPQVTYQNISTNLPPPLLGNSTGIDFQDELDEFFKNVSTSIPNFGSLTQINTTLLDLTYEMLSLQQVV
KALNESYIDLKELGNYTY

A C-terminal trimerization domain can be added to the protomers of the MERS-CoV S ectodomains trimer to promote trimerization of the ectodomain.

An exemplary sequence of MERS-CoV S ectodomain England1 strain including V1060P and L1061P substitutions and 748-RSVR-751 (residues 748-751 of SEQ ID NO: 1) to 748-ASVG-751 (residues 748-751 of SEQ ID NO: 3) substitutions to remove the S1/S2 cleavage site, and a T4 fibritin trimerization domain is provided as SEQ ID NO: 28:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK
MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPASVGSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSRSARSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDPPEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL
NTKYVAPQVTYQNISTNLPPPLLGNSTGIDFQDELDEFFKNVSTSIPNFGSLTQINTTLLDLTYEMLSLQQVV
KALNESYIDLKELGNYTYGGYIPEAPRDGQAYVRKDGEWVLLSTF

An exemplary sequence of MERS-CoV S ectodomain England1 strain including V1060P and L1061P substitutions and 748-RSVR-751 to 748-ASVG-751 and 884-RSAR-887 (residues 884-887 of SEQ ID NO: 1) to 884-GSAG-887 (residues 884-887 of SEQ ID NO: 3) substitutions to remove the S1/S2 cleavage site and the S2' cleavage site, and a T4 fibritin trimerization domain is provided as SEQ ID NO: 29:

MIHSVFLLMFLLTPTESYVDVGPDSVKSACIEVDIQQTFFDKTWPRPIDVSKADGIIYPQGRTYSNITITYQGLFPYQGD
HGDMYVYSAGHATGTTPQKLFVANYSQDVKQFANGFVVRIGAAANSTGTVIISPSTSATIRKIYPAFMLGSSVGNFSDGK
MGRFFNHTLVLLPDGCGTLLRAFYCILEPRSGNHCPAGNSYTSFATYHTPATDCSDGNYNRNASLNSFKEYFNLRNCTFM
YTYNITEDEILEWFGITQTAQGVHLFSSRYVDLYGGNMFQFATLPVYDTIKYYSIIPHSIRSIQSDRKAWAAFYVYKLQP
LTFLLDFSVDGYIRRAIDCGFNDLSQLHCSYESFDVESGVYSVSSFEAKPSGSVVEQAEGVECDFSPLLSGTPPQVYNFK
RLVFTNCNYNLTKLLSLFSVNDFTCSQISPAAIASNCYSSLILDYFSYPLSMKSDLSVSSAGPISQFNYKQSFSNPTCLI
LATVPHNLTTITKPLKYSYINKCSRFLSDDRTEVPQLVNANQYSPCVSIVPSTVWEDGDYYRKQLSPLEGGGWLVASGST
VAMTEQLQMGFGITVQYGTDTNSVCPKLEFANDTKIASQLGNCVEYSLYGVSGRGVFQNCTAVGVRQQRFVYDAYQNLVG
YYSDDGNYYCLRACVSVPVSVIYDKETKTHATLFGSVACEHISSTMSQYSRSTRSMLKRRDSTYGPLQTPVGCVLGLVNS
SLFVEDCKLPLGQSLCALPDTPSTLTPASVGSVPGEMRLASIAFNHPIQVDQLNSSYFKLSIPTNFSFGVTQEYIQTTIQ
KVTVDCKQYVCNGFQKCEQLLREYGQFCSKINQALHGANLRQDDSVRNLFASVKSSQSSPIIPGFGGDFNLTLLEPVSIS
TGSGSAGSAIEDLLFDKVTIADPGYMQGYDDCMQQGPASARDLICAQYVAGYKVLPPLMDVNMEAAYTSSLLGSIAGVGW
TAGLSSFAAIPFAQSIFYRLNGVGITQQVLSENQKLIANKFNQALGAMQTGFTTTNEAFHKVQDAVNNNAQALSKLASEL
SNTFGAISASIGDIIQRLDPPEQDAQIDRLINGRLTTLNAFVAQQLVRSESAALSAQLAKDKVNECVKAQSKRSGFCGQG
THIVSFVVNAPNGLYFMHVGYYPSNHIEVVSAYGLCDAANPTNCIAPVNGYFIKTNNTRIVDEWSYTGSSFYAPEPITSL
NTKYVAPQVTYQNISTNLPPPLLGNSTGIDFQDELDEFFKNVSTSIPNFGSLTQINTTLLDLTYEMLSLQQVV
KALNESYIDLKELGNYTYGGYIPEAPRDGQAYVRKDGEWVLLSTF

In some embodiments, the recombinant MERS-CoV S ectodomain trimer comprises protomers comprising the ectodomain sequence of any one of SEQ ID NOs: 2-4 and 29. In some embodiments, the recombinant MERS-CoV S ectodomain trimer comprises protomers comprising residues 18-1291 of any one of SEQ ID NOs: 2-4 or residues 18-1318 of SEQ ID NO: 29. In some embodiments, the recombinant MERS-CoV S ectodomain trimer comprises protomers comprising an ectodomain sequence at least 90% identical to the ectodomain sequence of any one of SEQ ID NOs: 2-4, wherein the MERS-CoV S ectodomain trimer is stabilized in the prefusion conformation and comprises the "2P" substitution and/or modifications to remove the S1/S2 cleavage site and the S2' cleavage site of the protomers. In some embodiments, the recombinant MERS-CoV S ectodomain trimer comprises protomers comprising an amino acid sequence at least 90% identical to residues 18-1291 of any one of SEQ ID NOs: 2-4 or residues 18-1318 of SEQ ID NO: 29, wherein the MERS-CoV S ectodomain trimer is stabilized in the prefusion conformation and comprises the "2P" substitution and/or modifications to remove the S1/S2 cleavage site and the S2' cleavage site of the protomers.

### B. SARS-CoV

In some embodiments, the immunogen comprises a recombinant SARS-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the SARS-CoV S ectodomain trimer in the prefusion conformation are located between residues 951 to 971 (such as between residues 961 to 971 or between residues 966 to 971) of the S ectodomain protomers in the trimer. In some embodiments, the SARS-CoV S ectodomain trimer is stabilized in the prefusion conformation by K968P and V969P substitutions ("2P") in the S ectodomain protomers in the trimer. The amino acid numbering for SARS-CoV S proteins is with reference to the SARS-CoV S sequence provided as SEQ ID NO: 6.

In some embodiments, the recombinant SARS-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant SARS-CoV S ectodomain trimer stabilized in the prefusion conformation by the one or more proline substitutions (such as K968P and V969P substitutions) comprises additional modifications for stabilization in the prefusion conformation.

With reference to the SARS-CoV S protein sequence provided as SEQ ID NO: 6, the ectodomain of the SARS-CoV S protein includes about residues 14-1190. Residues 1-13 are the signal peptide, which is removed during cellular processing. The S1/S2 cleavage site is located at position 667/668 or 678/679. The S2' cleavage site is located at about position 797/798. The HR1 is located at about residues 897-965. The central helix is located at about residues 970-1011. The HR2 is located at about 1145-1176. The C-terminal end of the S2 ectodomain is located at about residue 1190. In some embodiments, the protomers of the prefusion-stabilized SARS-CoV S ectodomain trimer can have a C-terminal residue (which can be linked to a trimerization domain, or a transmembrane domain, for example) of the C-terminal residue of the HR2 (e.g., position 1176), or the ectodomain (e.g., position 1190), or from one of positions 1176-1190. The position numbering of the S protein may vary between SARS-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary SARS-CoV S protein sequences are provided below. The prefusion stabilizing substitutions disclosed herein (and other modifications, such as substitutions to generate a single chain) can be incorporated into SARS-CoV S protein sequences.

An exemplary sequence of SARS-CoV S protein (including the ectodomain and TM and CT domains) is provided as SEQ ID NO: 6 (GenBank GI: 30795145, incorporated by reference herein):

MFIFLLFLTLTSGSDLDRCTTFDDVQAPNYTQHTSSMRGVYYPDEIFRSDTLYLTQDLFLPFYSNVTGFHTINHTFGNPV
IPFKDGIYFAATEKSNVVRGWVFGSTMNNKSQSVIIINNSTNVVIRACNFELCDNPFFAVSKPMGTQTHTMIFDNAFNCT
FEYISDAFSLDVSEKSGNFKHLREFVFKNKDGFLYVYKGYQPIDVVRDLPSGFNTLKPIFKLPLGINITNFRAILTAFSP
AQDIWGTSAAAYFVGYLKPTTFMLKYDENGTITDAVDCSQNPLAELKCSVKSFEIDKGIYQTSNFRVVPSGDVVRFPNIT
NLCPFGEVFNATKFPSVYAWERKKISNCVADYSVLYNSTFFSTFKCYGVSATKLNDLCFSNVYADSFVVKGDDVRQIAPG
QTGVIADYNYKLPDDFMGCVLAWNTRNIDATSTGNYNYKYRYLRHGKLRPFERDISNVPFSPDGKPCTPPALNCYWPLND
YGFYTTTGIGYQPYRVVVLSFELLNAPATVCGPKLSTDLIKNQCVNFNFNGLTGTGVLTPSSKRFQPFQQFGRDVSDFTD
SVRDPKTSEILDISPCAFGGVSVITPGTNASSEVAVLYQDVNCTDVSTAIHADQLTPAWRIYSTGNNVFQTQAGCLIGAE
HVDTSYECDIPIGAGICASYHTVSLLRSTSQKSIVAYTMSLGADSSIAYSNNTIAIPTNFSISITTEVMPVSMAKTSVDC
NMYICGDSTECANLLLQYGSFCTQLNRALSGIAAEQDRNTREVFAQVKQMYKTPTLKYFGGFNFSQILPDPLKPTKRSFI
EDLLFNKVTLADAGFMKQYGECLGDINARDLICAQKFNGLTVLPPLLTDDMIAAYTAALVSGTATAGWTFGAGAALQIPF
AMQMAYRFNGIGVTQNVLYENQKQIANQFNKAISQIQESLTTTSTALGKLQDVVNQNAQALNTLVKQLSSNFGAISSVLN
DILSRLDKVEAEVQIDRLITGRLQSLQTYVTQQLIRAAEIRASANLAATKMSECVLGQSKRVDFCGKGYHLMSFPQAAPH
GVVFLHVTYVPSQERNFTTAPAICHEGKAYFPREGVFVFNGTSWFITQRNFFSPQIITTDNTFVSGNCDVVIGIINNTVY
DPLQPELDSFKEELDKYFKNHTSPDVDLGDISGINASVVNIQKEIDRLNEVAKNLNESLIDLQELGKYEQYIKWPWYVWL
GFIAGLIAIVMVTILLCCMTSCCSCLKGACSCGSCCKFDEDDSEPVLKGVKLHYT

An exemplary sequence of SARS-CoV S ectodomain (TOR2 strain) including a double proline substitution for stabilization in the prefusion conformation is provided as SEQ ID NO: 7:

MFIFLLFLTLTSGSDLDRCTTFDDVQAPNYTQHTSSMRGVYYPDEIFRSDTLYLTQDLFLPFYSNVTGFHTINHTFGNPV
IPFKDGIYFAATEKSNVVRGWVFGSTMNNKSQSVIIINNSTNVVIRACNFELCDNPFFAVSKPMGTQTHTMIFDNAFNCT
FEYISDAFSLDVSEKSGNFKHLREFVFKNKDGFLYVYKGYQPIDVVRDLPSGFNTLKPIFKLPLGINITNFRAILTAFSP
AQDIWGTSAAAYFVGYLKPTTFMLKYDENGTITDAVDCSQNPLAELKCSVKSFEIDKGIYQTSNFRVVPSGDVVRFPNIT
NLCPFGEVFNATKFPSVYAWERKKISNCVADYSVLYNSTFFSTFKCYGVSATKLNDLCFSNVYADSFVVKGDDVRQIAPG
QTGVIADYNYKLPDDFMGCVLAWNTRNIDATSTGNYNYKYRYLRHGKLRPFERDISNVPFSPDGKPCTPPALNCYWPLND
YGFYTTTGIGYQPYRVVVLSFELLNAPATVCGPKLSTDLIKNQCVNFNFNGLTGTGVLTPSSKRFQPFQQFGRDVSDFTD
SVRDPKTSEILDISPCAFGGVSVITPGTNASSEVAVLYQDVNCTDVSTAIHADQLTPAWRIYSTGNNVFQTQAGCLIGAE
HVDTSYECDIPIGAGICASYHTVSLLRSTSQKSIVAYTMSLGADSSIAYSNNTIAIPTNFSISITTEVMPVSMAKTSVDC
NMYICGDSTECANLLLQYGSFCTQLNRALSGIAAEQDRNTREVFAQVKQMYKTPTLKYFGGFNFSQILPDPLKPTKRSFI
EDLLFNKVTLADAGFMKQYGECLGDINARDLICAQKFNGLTVLPPLLTDDMIAAYTAALVSGTATAGWTFGAGAALQIPF
AMQMAYRFNGIGVTQNVLYENQKQIANQFNKAISQIQESLTTTSTALGKLQDVVNQNAQALNTLVKQLSSNFGAISSVLN
DILSRLDPPEAEVQIDRLITGRLQSLQTYVTQQLIRAAEIRASANLAATKMSECVLGQSKRVDFCGKGYHLMSFPQAAPH
GVVFLHVTYVPSQERNFTTAPAICHEGKAYFPREGVFVFNGTSWFITQRNFFSPQIITTDNTFVSGNCDVVIGIINNTVY
DPLQPELDSFKEELDKYFKNHTSPDVDLGDISGINASVVNIQKEIDRLNEVAKNLNESLIDLQELGKYEQ

A C-terminal trimerization domain can be added to the protomers of the SARS-CoV S ectodomains trimer to promote trimerization of the ectodomain.

An exemplary sequence of SARS-CoV S ectodomain (TOR2 strain) including a double proline substitution for stabilization in the prefusion conformation, and a T4 fibritin trimerization domain is provided as SEQ ID NO: 30:

MFIFLLFLTLTSGSDLDRCTTFDDVQAPNYTQHTSSMRGVYYPDEIFRSDTLYLTQDLFLPFYSNVTGFHTINHTFGNPV
IPFKDGIYFAATEKSNVVRGWVFGSTMNNKSQSVIIINNSTNVVIRACNFELCDNPFFAVSKPMGTQTHTMIFDNAFNCT
FEYISDAFSLDVSEKSGNFKHLREFVFKNKDGFLYVYKGYQPIDVVRDLPSGFNTLKPIFKLPLGINITNFRAILTAFSP
AQDIWGTSAAAYFVGYLKPTTFMLKYDENGTITDAVDCSQNPLAELKCSVKSFEIDKGIYQTSNFRVVPSGDVVRFPNIT
NLCPFGEVFNATKFPSVYAWERKKISNCVADYSVLYNSTFFSTFKCYGVSATKLNDLCFSNVYADSFVVKGDDVRQIAPG
QTGVIADYNYKLPDDFMGCVLAWNTRNIDATSTGNYNYKYRYLRHGKLRPFERDISNVPFSPDGKPCTPPALNCYWPLND
YGFYTTTGIGYQPYRVVVLSFELLNAPATVCGPKLSTDLIKNQCVNFNFNGLTGTGVLTPSSKRFQPFQQFGRDVSDFTD
SVRDPKTSEILDISPCAFGGVSVITPGTNASSEVAVLYQDVNCTDVSTAIHADQLTPAWRIYSTGNNVFQTQAGCLIGAE
HVDTSYECDIPIGAGICASYHTVSLLRSTSQKSIVAYTMSLGADSSIAYSNNTIAIPTNFSISITTEVMPVSMAKTSVDC
NMYICGDSTECANLLLQYGSFCTQLNRALSGIAAEQDRNTREVFAQVKQMYKTPTLKYFGGFNFSQILPDPLKPTKRSFI
EDLLFNKVTLADAGFMKQYGECLGDINARDLICAQKFNGLTVLPPLLTDDMIAAYTAALVSGTATAGWTFGAGAALQIPF
AMQMAYRFNGIGVTQNVLYENQKQIANQFNKAISQIQESLTTTSTALGKLQDVVNQNAQALNTLVKQLSSNFGAISSVLN
DILSRLDPPEAEVQIDRLITGRLQSLQTYVTQQLIRAAEIRASANLAATKMSECVLGQSKRVDFCGKGYHLMSFPQAAPH
GVVFLHVTYVPSQERNFTTAPAICHEGKAYFPREGVFVFNGTSWFITQRNFFSPQIITTDNTFVSGNCDVVIGIINNTVY
DPLQPELDSFKEELDKYFKNHTSPDVDLGDISGINASVVNIQKEIDRLNEVAKNLNESLIDLQELGKYEQGGYIPEAPR
DGQAYVRKDGEWVLLSTF

In some embodiments, the recombinant SARS-CoV S ectodomain trimer comprises protomers comprising the ectodomain sequence of SEQ ID NO: 7. In some embodiments, the recombinant SARS-CoV S ectodomain trimer comprises protomers comprising residues 14-1190 of SEQ ID NO: 7 or residues 14-1217 of SEQ ID NO: 30. In some embodiments, the recombinant SARS-CoV S ectodomain trimer comprises protomers comprising an ectodomain sequence at least 90% identical to the ectodomain sequence of SEQ ID NO: 7, wherein the SARS-CoV S ectodomain trimer is stabilized in the prefusion conformation and comprises the "2P" substitution and/or modifications to remove the S1/S2 cleavage site and the S2' cleavage site of the protomers. In some embodiments, the recombinant SARS-CoV S ectodomain trimer comprises protomers comprising an amino acid sequence at least 90% identical to residues 14-1190 of SEQ ID NO: 7, wherein the SARS-CoV S ectodomain trimer is stabilized in the prefusion conformation and comprises the "2P" substitution and/or modifications to remove the S1/S2 cleavage site and the S2' cleavage site of the protomers..

### C. HKU1-CoV

In some embodiments, the immunogen comprises a recombinant HKU1-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the HKU1-CoV S ectodomain trimer in the prefusion conformation are located between residues 1050 to 1070 (such as between residues 1060 to 1070 or between residues 1065 to 1070) of the S ectodomain protomers in the trimer. In some embodiments, the HKU1-CoV S ectodomain trimer is stabilized in the prefusion conformation by N1067P and L1068P substitutions ("2P") in the S ectodomain protomers in the trimer. The amino acid numbering for HKU1-CoV S proteins is with reference to the HKU1-CoV S sequence provided as SEQ ID NO: 7.

In some embodiments, the recombinant HKU1-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant HKU1-CoV S ectodomain trimer stabilized in the prefusion conformation by the one or more proline substitutions (such as N1067P and L1068P substitutions) comprise additional modifications for stabilization in the prefusion conformation.

With reference to the HKU1-CoV S protein sequence provided as SEQ ID NO: 8, the ectodomain of the HKU1-CoV S protein includes about residues 14-1290. Residues 1-13 are the signal peptide, which is removed during cellular processing. The S1/S2 cleavage site is located at about position 756/757. The S2' cleavage site is located at about position 900/901. The HR1 is located at about residues 996-1064. The central helix is located at about residues 1069-1110. The HR2 is located at about 1245-1276. The C-terminal end of the S2 ectodomain is located at about residue 1290. In some embodiments, the protomers of the prefusion-stabilized HKU1-CoV S ectodomain trimer can have a C-terminal residue (which can be linked to a trimerization domain, or a transmembrane domain, for example) of the C-terminal residue of the HR2 (e.g., position 1276), or the ectodomain (e.g., position 1290), or from one of positions 1276-1290. The position numbering of the S protein may vary between HKU1-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary HKU1-CoV S protein sequences are provided below. The prefusion stabilizing substitutions disclosed herein (and other modifications, such as substitutions to generate a single chain) can be incorporated into HKU1-CoV S protein sequences.

An exemplary sequence of HKU1-CoV S protein (including the ectodomain and TM and CT domains) is provided as SEQ ID NO: 8 (GenBank GI: 123867264, incorporated by reference herein):

MFLIIFILPTTLAV[?]GDFNCTNSFINDYNKTIPRISEDVVDVSLGLGTYYVLN[?]VYLNTTLLFTGYFPKSGANFRDLALK
GSIYLSTLWYKPPFLSDFNNGIFSKVKNTKLYVNNTLYSEFSTIV[?]GSVFVNTSYTIVVQPHNGILEITACQYTMCEYPH
TVCKSKGSIRNESWHIDSSEPLCLFKKNFTYNVSADWLYFHFYQERGVFYAYYADVGMPTTFLFSLYLGTILSHYYVMPL
TCNAISSNTDNETLEYWVTPLSRRQYLLNFDEHGVITNAVDCSSSFLSEIQCKTQSFAPNTGVY[?]LSGFTVKPVATVYRR
IPNLPDCDIDNWLNNVSVPSPLNWERRIFSNCNFNLSTLLRLVHVDSFSCNNL[?]KSKIFGSCFNSITVDKFAIPNRRRDD
LQLGSSGFLQSSNYKIDISSSSCQLYYSLPLVNVTINNFNPSSWNRRYGFGSFNLSSYDVVYSD[?]CFSVNSDFCPCADPS
VVNSCAKSKPPSAICPAGTKYRHCDLDTTLYVKNWCRCSCLPDPISTYSPNTC[?]QKKVVVGIGE[?]CPGLGINEEKCGTQL
NHSSCFCSPDAFLGWSFDSCISN[?]RCNIFSNFIFNGINSGTTCSNDLLYSNTEISTGVCVNYDLYGITGQGIFKEVSAAY
YNNWQNLLYDSNGN[?]IGFKDFLT[?]KTYTILPCYSGRVSAAFYQNSSSPALLYRNLKCSYVLNNISFISQPFYFDSYLGCV
LNAVNLTSYSVSSCDLRMGSGFCIDYALPSSRRKRRGISSPYRFVTFEPFNVS[?]VNDSVETVGGLFEIQIPTNFTIAGHE

EFIQTSSPKVTIDCSAFVCSNYAACHDLLSEYGTFCDNINSILNEVNDLLDITQLQVANALMQGVTLSSNLNTNLHSDVD
NIDFKSLLGCLGSQCGSSSRSLLEDLLFNKVKLSDVGFVEAYNNCTGGSEIRDLLCVQSFNGIKVLPPILSETQISGYTT
AATVAAMFPPWSAAAGVPFSLNVQYRINGLGVTMDVLNKNQKLIANAFNKALLSIQNGFTATNSALAKIQSVVNANAQAL
NSLLQQLFNKFGAISSSLQEILSRLDNLEAQVQIDRLINGRLTALNAYVSQQLSDITLIKAGASRAIEKVNECVKSQSPR
INFCGNGNHILSLVQNAPYGLLFIHFSYKPTSFKTVLVSPGLCLSGDRGIAPKQGYFIKQNDSWMFTGSSYYYPEPISDK
NVVFMNSCSVNFTKAPFIYLNNSIPNLSDFEAELSLWFKNHTSIAPNLTFNSHINATFLDLYYEMNVIQESIKSLNSSFI
NLKEIGTYEMYVKWPWYIWLLIVILFIIFLMILFFICCCTGCGSACFSKCHNCCDEYGGHNDFVIKASHDD

An exemplary sequence of HKU1-CoV S ectodomain including a double proline substitution for stabilization in the prefusion conformation is provided as (SEQ ID NO: 9, which also includes mutations to eliminate the S1/S2 cleavage site:

MFLIIFILPTTLAVIGDFNCTNSFINDYNKTIPRISEDVVDVSLGLGTYYVLNRVYLNTTLLFTGYFPKSGANFRDLALK
GSIYLSTLWYKPPFLSDFNNGIFSKVKNTKLYVNNTLYSEFSTIVIGSVFVNTSYTIVVQPHNGILEITACQYTMCEYPH
TVCKSKGSIRNESWHIDSSEPLCLFKKNFTYNVSADWLYFHFYQERGVFYAYYADVGMPTTFLFSLYLGTILSHYYVMPL
TCNAISSNTDNETLEYWVTPLSRRQYLLNFDEHGVITNAVDCSSSFLSEIQCKTQSFAPNTGVYDLSGFTVKPVATVYRR
IPNLPDCDIDNWLNNVSVPSPLNWERRIFSNCNFNLSTLLRLVHVDSFSCNNLDKSKIFGSCFNSITVDKFAIPNRRRDD
LQLGSSGFLQSSNYKIDISSSSCQLYYSLPLVNVTINNFNPSSWNRRYGFGSFNLSSYDVVYSDHCFSVNSDFCPCADPS
VVNSCAKSKPPSAICPAGTKYRHCDLDTTLYVKNWCRCSCLPDPISTYSPNTCPQKKVVVGIGEHCPGLGINEEKCGTQL
NHSSCFCSPDAFLGWSFDSCISNNRCNIFSNFIFNGINSGTTCSNDLLYSNTEISTGVCVNYDLYGITGQGIFKEVSAAY
YNNWQNLLYDSNGNIIGFKDFLTNKTYTILPCYSGRVSAAFYQNSSSPALLYRNLKCSYVLNNISFISQPFYFDSYLGCV
LNAVNLTSYSVSSCDLRMGSGFCIDYALPSSGGSGSGISSPYRFVTFEPFNVSFVNDSVETVGGLFEIQIPTNFTIAGHE
EFIQTSSPKVTIDCSAFVCSNYAACHDLLSEYGTFCDNINSILNEVNDLLDITQLQVANALMQGVTLSSNLNTNLHSDVD
NIDFKSLLGCLGSQCGSSSRSLLEDLLFNKVKLSDVGFVEAYNNCTGGSEIRDLLCVQSFNGIKVLPPILSETQISGYTT
AATVAAMFPPWSAAAGVPFSLNVQYRINGLGVTMDVLNKNQKLIANAFNKALLSIQNGFTATNSALAKIQSVVNANAQAL
NSLLQQLFNKFGAISSSLQEILSRLDPPEAQVQIDRLINGRLTALNAYVSQQLSDITLIKAGASRAIEKVNECVKSQSPR
INFCGNGNHILSLVQNAPYGLLFIHFSYKPTSFKTVLVSPGLCLSGDRGIAPKQGYFIKQNDSWMFTGSSYYYPEPISDK
NVVFMNSCSVNFTKAPFIYLNNSIPNLSDFEAELSLWFKNHTSIAPNLTFNSHINATFLDLYYEMNVIQESIKSLN

A C-terminal trimerization domain can be added to the protomers of the HKU1-CoV S ectodomains trimer to promote trimerization of the ectodomain.

An exemplary sequence of HKU1-CoV S ectodomain including a double proline substitution for stabilization in the prefusion conformation, mutations to eliminate the S1/S2 cleavage site, and a T4 fibritin trimerization domain is provided as SEQ ID NO: 31:

MFLIIFILPTTLAVIGDFNCTNSFINDYNKTIPRISEDVVDVSLGLGTYYVLNRVYLNTTLLFTGYFPKSGANFRDLALK
GSIYLSTLWYKPPFLSDFNNGIFSKVKNTKLYVNNTLYSEFSTIVIGSVFVNTSYTIVVQPHNGILEITACQYTMCEYPH
TVCKSKGSIRNESWHIDSSEPLCLFKKNFTYNVSADWLYFHFYQERGVFYAYYADVGMPTTFLFSLYLGTILSHYYVMPL
TCNAISSNTDNETLEYWVTPLSRRQYLLNFDEHGVITNAVDCSSSFLSEIQCKTQSFAPNTGVYDLSGFTVKPVATVYRR
IPNLPDCDIDNWLNNVSVPSPLNWERRIFSNCNFNLSTLLRLVHVDSFSCNNLDKSKIFGSCFNSITVDKFAIPNRRRDD
LQLGSSGFLQSSNYKIDISSSSCQLYYSLPLVNVTINNFNPSSWNRRYGFGSFNLSSYDVVYSDHCFSVNSDFCPCADPS
VVNSCAKSKPPSAICPAGTKYRHCDLDTTLYVKNWCRCSCLPDPISTYSPNTCPQKKVVVGIGEHCPGLGINEEKCGTQL
NHSSCFCSPDAFLGWSFDSCISNNRCNIFSNFIFNGINSGTTCSNDLLYSNTEISTGVCVNYDLYGITGQGIFKEVSAAY
YNNWQNLLYDSNGNIIGFKDFLTNKTYTILPCYSGRVSAAFYQNSSSPALLYRNLKCSYVLNNISFISQPFYFDSYLGCV
LNAVNLTSYSVSSCDLRMGSGFCIDYALPSSGGSGSGISSPYRFVTFEPFNVSFVNDSVETVGGLFEIQIPTNFTIAGHE
EFIQTSSPKVTIDCSAFVCSNYAACHDLLSEYGTFCDNINSILNEVNDLLDITQLQVANALMQGVTLSSNLNTNLHSDVD
NIDFKSLLGCLGSQCGSSSRSLLEDLLFNKVKLSDVGFVEAYNNCTGGSEIRDLLCVQSFNGIKVLPPILSETQISGYTT
AATVAAMFPPWSAAAGVPFSLNVQYRINGLGVTMDVLNKNQKLIANAFNKALLSIQNGFTATNSALAKIQSVVNANAQAL
NSLLQQLFNKFGAISSSLQEILSRLDPPEAQVQIDRLINGRLTALNAYVSQQLSDITLIKAGASRAIEKVNECVKSQSPR
INFCGNGNHILSLVQNAPYGLLFIHFSYKPTSFKTVLVSPGLCLSGDRGIAPKQGYFIKQNDSWMFTGSSYYYPEPISDK
NVVFMNSCSVNFTKAPFIYLNNSIPNLSDFEAELSLWFKNHTSIAPNLTFNSHINATFLDLYYEMNVIQESIKSLNGGY
IPEAPRDGQAYVRKDGEWVLLSTF

In some embodiments, the recombinant HKU1-CoV S ectodomain trimer comprises protomers comprising the ectodomain sequence of SEQ ID NO: 9. In some embodiments, the recombinant HKU1 - CoV S ectodomain trimer comprises protomers comprising residues 14-1276 of SEQ ID NO: 9 or residues 14-1303 of SEQ ID NO: 31. In some embodiments, the recombinant HKU1-CoV S ectodomain trimer comprises protomers comprising an ectodomain sequence at least 90% identical to the ectodomain sequence of SEQ ID NO: 9, wherein the HKU1-CoV S ectodomain trimer is stabilized in the prefusion conformation and comprises the "2P" substitution and/or modifications to remove the S1/S2 cleavage site and the S2' cleavage site of the protomers. In some embodiments, the recombinant HKU1-CoV S ectodomain trimer comprises protomers comprising an amino acid sequence at least 90% identical to residues 14-1276 of SEQ ID NO: 9 or residues 14-1303 of SEQ ID NO: 31, wherein the HKU1-CoV S ectodomain trimer is stabilized in the prefusion conformation and comprises the "2P" substitution and/or modifications to remove the S1/S2 cleavage site and the S2' cleavage site of the protomers..

### D. HKU9-CoV

In some embodiments, the immunogen comprises a recombinant HKU9-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the HKU9-CoV S ectodomain trimer in the prefusion conformation are located between residues 966 to 986 (such as between residues 976 to 986 or between residues 981 to 986) of the S ectodomain protomers in the trimer. In some embodiments, the HKU9-CoV S ectodomain trimer is stabilized in the prefusion conformation by G1018P and L1019P substitutions ("2P") in the S ectodomain protomers in the trimer. The amino acid numbering for HKU9-CoV S proteins is with reference to the HKU9-CoV S sequence provided as SEQ ID NO: 12.

In some embodiments, the recombinant HKU9-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant HKU9-CoV S ectodomain trimer stabilized in the prefusion conformation by the one or more proline substitutions (such as G1018P and L1019P substitutions) comprises additional modifications for stabilization in the prefusion conformation.

With reference to the HKU9-CoV S protein sequence provided as SEQ ID NO: 12, the ectodomain of the HKU9-CoV S protein includes about residues 15-1207. Residues 1-14 are the signal peptide, which is removed during cellular processing. The S1/S2 cleavage site is located at about position 676/677. The S2' cleavage site is located at about position 809/810. The HR1 is located at about residues 912-980. The central helix is located at about residues 986-1026. The HR2 is located at about 1162-1193. The C-terminal end of the S2 ectodomain is located at about residue 1207. In some embodiments, the protomers of the prefusion-stabilized HKU9-CoV S ectodomain trimer can have a C-terminal residue (which can be linked to a trimerization domain, or a transmembrane domain, for example) of the C-terminal residue of the HR2 (e.g., position 1193), or the ectodomain (e.g., position 1207), or from one of positions 1193-1207. The position numbering of the S protein may vary between HKU9-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary HKU9-CoV S protein sequences are provided below. The prefusion stabilizing substitutions disclosed herein (and other modifications, such as substitutions to generate a single chain) can be incorporated into HKU9-CoV S protein sequences.

An exemplary sequence of HKU9-CoV S protein (including the ectodomain and TM and CT domains) is provided as SEQ ID NO: 12 (GenBank GI:148841195, incorporated by reference herein):

MLLILVLGVSLAAASRPECFNPRFTLTPLNHTLNYTSIKAKVSNVLLPDPYIAYSGQTLRQNLFMADMSNTILYPVTPPA
NGANGGFIYNTSIIPVSAGLFVNTWMYRQPASSRAYCQEPFGVAFGDTFENDRIAILIMAPDNLGSWSAVAPRNQTNIYL
LVCSNATLCINPGFNRWGPAGSFIAPDALVDHSNSCFVNNTFSVN[?]STSRISLAFLFKDGDLLIYHSGWLPTSNFEHGFS
RGSHPMTYFMSLPVGGNLPRAQFFQSIVRSNAIDKGDGMCTNFDVNLHVAHLINRDLLVSYFNNGSVANAADCADSAAEE
LYCVTGSFDPPTGVYPLSRYRAQVAGFVRVTQRGSYCTPPYSVLQDPPQPVVW[?]RYMLYDCVFDFTVVVDSLPTHQLQCY
GVSPRRLASMCYGSVTLDVMRINETHLNNLFNRVPDTFSLYNYALPDNFYGCL[?]AFYLNSTAPYAVANRFPIKPGGRQSN
SAFIDTVINAAHYSPFSYVYGLAVITLKPAAGSKLVCPVANDTVV[?]TDRCVQYNLYGYTGTGVLSKNTSLVIPDGKVFTA
SSTGTIIGVSINSTTYSIMPCVTVPVSVGY[?]PNFERALLFNGLSCSQRSRAVT[?]PVSVLWSASATAQDAFDTPSGCVVNV
ELRNTTIVNTCAMP[?]GNSLCFINGSIATANADSLPRLQLVNYDPLYDNSTATPMTPVYWVKVPTNFTLSATEEYIQTTAP
KITIDCARYLCGDSSRCLNVLLHYGTFCNDINKALSRVSTILDSALLSLVKELSINTRDEVTTFSFDGDYNFTGLMGCLG
PNCGATTYRSAFSDLLYDKVRITDPGFMQSYQKCIDSQWGGSIRDLLCTQTYNGIAVLPPIVSPAMQALYTSLLVGAVAS
SGYTFGITSAGVIPFATQLQFRLNGIGVTTQVLVENQKLIASSFNNALVNIQKGFTETSIALSKMQDVINQHAAQLHTLV
VQLGNSFGAISSSINEIFSRLEGLAANAEVDRLINGRMMVLNTYVTQLLIQAS[?]AKAQNALAAQKISECVKAQSLRNDFC
GNGTHVLSIPQLAPNGVLFIHYAYTPTEYAFVQTSAGLCHNGTGYAPRQGMFVLPNNTNMWHFTTMQFYNPVNISASNTQ
VLTSCSVNYTSVNYTVLEPSVPGDYDFQKEFDKFYKNLSTIFNNTFNPNDFNFSTVDVTAQIKSLHDVVNQLNQSFIDLK
KLNVYEKTIKWPWYVWLAMIAGIVGLVLAVIMLMCMTNCCSCFKGMCDCRRCCGSYDSYDDVYPAVRVNKKRTV

An exemplary sequence of HKU9-CoV S protein including a double proline substitution for stabilization in the prefusion conformation is provided as SEQ ID NO: 13:

MLLILVLGVSLAAASRPECFNPRFTLTPLNHTLNYTSIKAKVSNVLLPDPYIAYSGQTLRQNLFMADMSNTILYPVTPPA
NGANGGFIYNTSIIPVSAGLFVNTWMYRQPASSRAYCQEPFGVAFGDTFENDRIAILIMAPDNLGSWSAVAPRNQTNIYL
LVCSNATLCINPGFNRWGPAGSFIAPDALVDHSNSCFVNNTFSVNISTSRISLAFLFKDGDLLIYHSGWLPTSNFEHGFS
RGSHPMTYFMSLPVGGNLPRAQFFQSIVRSNAIDKGDGMCTNFDVNLHVAHLINRDLLVSYFNNGSVANAADCADSAAEE
LYCVTGSFDPPTGVYPLSRYRAQVAGFVRVTQRGSYCTPPYSVLQDPPQPVVWRRYMLYDCVFDFTVVVDSLPTHQLQCY
GVSPRRLASMCYGSVTLDVMRINETHLNNLFNRVPDTFSLYNYALPDNFYGCLHAFYLNSTAPYAVANRFPIKPGGRQSN
SAFIDTVINAAHYSPFSYVYGLAVITLKPAAGSKLVCPVANDTVVITDRCVQYNLYGYTGTGVLSKNTSLVIPDGKVFTA
SSTGTIIGVSINSTTYSIMPCVTVPVSVGYHPNFERALLFNGLSCSQRSRAVTEPVSVLWSASATAQDAFDTPSGCVVNV
ELRNTTIVNTCAMPIGNSLCFINGSIATANADSLPRLQLVNYDPLYDNSTATPMTPVYWVKVPTNFTLSATEEYIQTTAP
KITIDCARYLCGDSSRCLNVLLHYGTFCNDINKALSRVSTILDSALLSLVKELSINTRDEVTTFSFDGDYNFTGLMGCLG
PNCGATTYRSAFSDLLYDKVRITDPGFMQSYQKCIDSQWGGSIRDLLCTQTYNGIAVLPPIVSPAMQALYTSLLVGAVAS
SGYTFGITSAGVIPFATQLQFRLNGIGVTTQVLVENQKLIASSFNNALVNIQKGFTETSIALSKMQDVINQHAAQLHTLV
VQLGNSFGAISSSINEIFSRLEPPAANAEVDRLINGRMMVLNTYVTQLLIQASEAKAQNALAAQKISECVKAQSLRNDFC
GNGTHVLSIPQLAPNGVLFIHYAYTPTEYAFVQTSAGLCHNGTGYAPRQGMFVLPNNTNMWHFTTMQFYNPVNISASNTQ
VLTSCSVNYTSVNYTVLEPSVPGDYDFQKEFDKFYKNLSTIFNNTFNPNDFNFSTVDVTAQIKSLHDVVNQLNQSFIDLK
KLNVYEK

A C-terminal trimerization domain can be added to the protomers of the HKU9-CoV S ectodomains trimer to promote trimerization of the ectodomain.

An exemplary sequence of HKU9-CoV S protein including a double proline substitution for stabilization in the prefusion conformation, and a T4 fibritin trimerization domain is provided as SEQ ID NO: 32:

MLLILVLGVSLAAASRPECFNPRFTLTPLNHTLNYTSIKAKVSNVLLPDPYIAYSGQTLRQNLFMADMSNTILYPVTTPPA
NGANGGFIYNTSIIPVSAGLFVNTWMYRQPASSRAYCQEPFGVAFGDTFENDRIAILIMAPDNLGSWSAVAPRNQTNIYL
LVCSNATLCINPGFNRWGPAGSFIAPDALVDHSNSCFVNNTFSVNISTSRISLAFLFKDGDLLIYHSGWLPTSNFEHGFS
RGSHPMTYFMSLPVGGNLPRAQFFQSIVRSNAIDKGDGMCTNFDVNLHVAHLINRDLLVSYFNNGSVANAADCADSAAEE
LYCVTGSFDPPTGVYPLSRYRAQVAGFVRVTQRGSYCTPPYSVLQDPPQPVVWRRYMLYDCVFDFTVVVDSLPTHQLQCY
GVSPRRLASMCYGSVTLDVMRINETHLNNLFNRVPDTFSLYNYALPDNFYGCLHHAFYLNSTAPYAVANRFPIKPGGRQSN
SAFIDTVINAAHYSPFSYVYGLAVITLKPAAGSKLVCPVANDTVVITDRCVQYNLYGYTGTGVLSKNTSLVIPDGKVFTA
SSTGTIIGVSINSTTYSIMPCVTVPVSVGYHPNFERALLFNGLSCSQRSRAVTEPVSVLWSASATAQDAFDTPSGCVVNV
ELRNTTIVNTCAMPIGNSLCFINGSIATANADSLPRLQLVNYDPLYDNSTATPMTPVYWVKVPTNFTLSATEEYIQTTAP
KITIDCARYLCGDSSRCLNVLLHYGTFCNDINKALSRVSTILDSALLSLVKELSINTRDEVTTFSFDGDYNFTGLMGCLG
PNCGATTYRSAFSDLLYDKVRITDPGFMQSYQKCIDSQWGGSIRDLLCTQTYNGIAVLPPIVSPAMQALYTSLLVGAVAS
SGYTFGITSAGVIPFATQLQFRLNGIGVTTQVLVENQKLIASSFNNALVNIQKGFTETSIALSKMQDVINQHAAQLHTLV
VQLGNSFGAISSSINEIFSRLEPPAANAEVDRLINGRMMVLNTYVTQLLIQASEAKAQNALAAQKISECVKAQSLRNDFC
GNGTHVLSIPQLAPNGVLFIHYAYTPTEYAFVQTSAGLCHNGTGYAPRQGMFVLPNNTNMWHFTTMQFYNPVNISASNTQ
VLTSCSVNYTSVNYTVLEPSVPGDYDFQKEFDKFYKNLSTIFNNTFNPNDFNFSTVDVTAQIKSLHDVVNQLNQSFIDLK
KLNVYEKGGYIPEAPRDGQAYVRKDGEWVLLSTF

In some embodiments, the recombinant HKU9-CoV S ectodomain trimer comprises protomers comprising the ectodomain sequence of SEQ ID NO: 13. In some embodiments, the recombinant HKU9-CoV S ectodomain trimer comprises protomers comprising residues 15-1207 of SEQ ID NO: 13 or residues 15-1234 of SEQ ID NO: 32. In some embodiments, the recombinant HKU9-CoV S ectodomain trimer comprises protomers comprising an ectodomain sequence at least 90% identical to the ectodomain sequence of SEQ ID NO: 13, wherein the HKU9-CoV S ectodomain trimer is stabilized in the prefusion conformation and comprises the "2P" substitution and/or modifications to remove the S1/S2 cleavage site and the S2' cleavage site of the protomers. In some embodiments, the recombinant HKU9-CoV S ectodomain trimer comprises protomers comprising an amino acid sequence at least 90% identical to residues 15-1207 of SEQ ID NO: 13 or residues 15-1234 of SEQ ID NO: 32, wherein the HKU9-CoV S ectodomain trimer is stabilized in the prefusion conformation and comprises the "2P" substitution and/or modifications to remove the S1/S2 cleavage site and the S2' cleavage site of the protomers..

### E. OC43-CoV

In some embodiments, the immunogen comprises a recombinant OC43-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the OC43-CoV S ectodomain trimer in the prefusion conformation are located between residues 1062-1082 (such as between residues 1072-1082 or between residues 1077-1082) of the S cctodomain protomers in the trimer. In some embodiments, the OC43-CoV S ectodomain trimer is stabilized in the prefusion conformation by A1079P and L1080P substitutions ("2P") in the S ectodomain protomers in the trimer. The amino acid numbering for OC43-CoV S proteins is with reference to the OC43-CoV S sequence provided as SEQ ID NO: 10.

In some embodiments, the recombinant OC43-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant OC43-CoV S ectodomain trimer stabilized in the prefusion conformation by the one or more proline substitutions (such as A1079P and L1080P substitutions) comprise additional modifications for stabilization in the prefusion conformation.

With reference to the OC43-CoV S protein sequence provided as SEQ ID NO: 10, the ectodomain of the OC43-CoV S protein includes about residues 15-1301. Residues 1-14 are the signal peptide, which is removed during cellular processing. The S1/S2 cleavage site is located at about position 767/768. The S2' cleavage site is located at about position 912/913. The HR1 is located at about residues 1008-1076. The central helix is located at about residues 1081-1122. The HR2 is located at about 1257-1287. The C-terminal end of the S2 ectodomain is located at about residue 1301. In some embodiments, the protomers of the prefusion-stabilized OC43-CoV S ectodomain trimer can have a C-terminal residue (which can be linked to a trimerization domain, or a transmembrane domain, for example) of the C-terminal residue of the HR2 (e.g., position 1287), or the ectodomain (e.g., position 1301), or from one of positions 1287-1301. The position numbering of the S protein may vary between OC43-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary OC43-CoV S protein sequences are provided below. The prefusion stabilizing substitutions disclosed herein (and other modifications, such as substitutions to generate a single chain) can be incorporated into OC43-CoV S protein sequences.

An exemplary sequence of OC43-CoV S protein (including the ectodomain and TM and CT domains) is provided as GenBank GI: 744516696, incorporated by reference herein. Another exemplary sequence of OC43-CoV S protein is provided as GenBank GI:549302, incorporated by reference herein):

MFLILLISLPTAFAVIGDLKCPLDSRTGSLNNIDTGPPSISTATVDVTNGLGTYYVLDRVYLNTTLFLNGYYPTSGSTYR
NMALKGTDKLSTLWFKPPFLSDFINGIFAKVKNTKVFKDGVMYSEFPAITIGSTFVNTSYSVVVQPRTINSTQDGVNKLQ
GLLEVSVCQYNMCEYPHTICHPKLGNHFKELWHMDTGVVSCLYKRNFTYDVNATYLYFHFYQEGGTFYAYFTDTGVVTKF
LFNVYLGMALSHYYVMPLTCISRRDIGFTLEYWVTPLTSRQYLLAFNQDGIIFNAVDCMSDFMSEIKCKTQSIAPPTGVY
ELNGYTVQPIADVYRRKPDLPNCNIEAWLNDKSVPSPLNWERKTFSNCNFNMSSLMSFIQADSFTCNNIDAAKIYGMCFS
SITIDKFAIPNGRKVDLQLGNLGYLQSFNYRIDTTATSCQLYYNLPAANVSVSRFNPSTWNKRFGFIENSVFKPQPAGVL
TNHDVVYAQHCFKAPKNFCPCKLNSSLCVGSGPGKNNGIGTCPAGTNYLTCHNLCNPDPITFTGPYKCPQTKSLVGIGEH
CSGLAVKSDYCGGNPCTCQPQAFLGWSADSCLQGDKCNIFANLILHDVNSGLTCSTDLQKANTDIKLGVCVNYDLYGISG
QGIFVEVNATYYNSWQNLLYDSNGNLYGFRDYITNRTFMIRSCYSGRVSAAFHANSSEPALLFRNIKCNYVFNNSLIRQL
QPINYFDSYLGCVVNAYNSTAISVQTCDLTVGSGYCVDYSKNRRSRRAITTGYRFTNFEPFTVNSVNDSLEPVGGLYEIQ
IPSEFTIGNMEEFIQTSSPKVTIDCAAFVCGDYAACKSQLVEYGSFCDNINAILTEVNELLDTTQLQVANSLMNGVTLST
KLKDGVNFNVDDINFSSVLGCLGSECSKASSRSAIEDLLFDKVKLSDVGFVAAYNNCTGGAEIRDLICVQSYKGIKVLPP
LLSENQISGYTLAATSASLFPPWTAAAGVPFYLNVQYRINGLGVTMDVLSQNQKLIANAFNNALDAIQEGFDATNSALVK
IQAVVNANAEALNNLLQQLSNRFGAISSSLQEILSRLDALEAEAQIDRLINGRLTALNAYVSQQLSDSTLVKFSAAQAME

KVNECVKSQSSRINFCGNGNHIISLVQNAPYGLYFIHFSYVPTKYVTAKVSPGLCIAGDRGIAPKSGYFVNVNNTWMYTG
SGYYYPEPITENNVVVMSTCAVNYTKAPYVMLNTSTPNLPDFREELDQWFKNQTSVAPDLSLDYINVTFLDLQVEMNRLQ
EAIKVLNQSYINLKDIGTYEYYVKWPWYVWLLIGLAGVAMLVLLFFICCCTGCGTSCFKKCGGCCDDYTGYQELVIKTSH
DD (SEQ ID NO: 10)

An exemplary sequence of OC43-CoV S ectodomain including a double proline substitution for stabilization in the prefusion conformation is provided as SEQ ID NO: 11, which also includes mutations to eliminate the S1/S2 cleavage site:

MFLILLISLPTAFAVIGDLKCPLDSRTGSLNNIDTGPPSISTATVDVTNGLGTYYVLDRVYLNTTLFLNGYYPTSGSTYR
NMALKGTDKLSTLWFKPPFLSDFINGIFAKVKNTKVFKDGVMYSEFPAITIGSTFVNTSYSVVVQPRTINSTQDGVNKLQ
GLLEVSVCQYNMCEYPHTICHPKLGNHFKELWHMDTGVVSCLYKRNFTYDVNATYLYFHFYQEGGTFYAYFTDTGVVTKF
LFNVYLGMALSHYYVMPLTCISRRDIGFTLEYWVTPLTSRQYLLAFNQDGIIFNAVDCMSDFMSEIKCKTQSIAPPTGVY
ELNGYTVQPIADVYRRKPDLPNCNIEAWLNDKSVPSPLNWERKTFSNCNFNMSSLMSFIQADSFTCNNIDAAKIYGMCFS
SITIDKFAIPNGRKVDLQLGNLGYLQSFNYRIDTTATSCQLYYNLPAANVSVSRFNPSTWNKRFGFIENSVFKPQPAGVL
TNHDVVYAQHCFKAPKNFCPCKLNSSLCVGSGPGKNNGIGTCPAGTNYLTCHNLCNPDPITFTGPYKCPQTKSLVGIGEH
CSGLAVKSDYCGGNPCTCQPQAFLGWSADSCLQGDKCNIFANLILHDVNSGLTCSTDLQKANTDIKLGVCVNYDLYGISG
QGIFVEVNATYYNSWQNLLYDSNGNLYGFRDYITNRTFMIRSCYSGRVSAAFHANSSEPALLFRNIKCNYVFNNSLIRQL
QPINYFDSYLGCVVNAYNSTAISVQTCDLTVGSGYCVDYSKNGGSGSAITTGYRFTNFEPFTVNSVNDSLEPVGGLYEIQ
IPSEFTIGNMEEFIQTSSPKVTIDCAAFVCGDYAACKSQLVEYGSFCDNINAILTEVNELLDTTQLQVANSLMNGVTLST
KLKDGVNFNVDDINFSSVLGCLGSECSKASSRSAIEDLLFDKVKLSDVGFVAAYNNCTGGAEIRDLICVQSYKGIKVLPP
LLSENQISGYTLAATSASLFPPWTAAAGVPFYLNVQYRINGLGVTMDVLSQNQKLIANAFNNALDAIQEGFDATNSALVK
IQAVVNANAEALNNLLQQLSNRFGAISSSLQEILSRLDPPEAEAQIDRLINGRLTALNAYVSQQLSDSTLVKFSAAQAME
KVNECVKSQSSRINFCGNGNHIISLVQNAPYGLYFIHFSYVPTKYVTAKVSPGLCIAGDRGIAPKSGYFVNVNNTWMYTG
SGYYYPEPITENNVVVMSTCAVNYTKAPYVMLNTSTPNLPDFREELDQWFKNQTSVAPDLSLDYINVTFLDLQVEMNRLQ
EAIKVLN

A C-terminal trimerization domain can be added to the protomers of the OC43-CoV S ectodomains trimer to promote trimerization of the ectodomain.

An exemplary sequence of OC43-CoV S ectodomain including a double proline substitution for stabilization in the prefusion conformation, mutations to eliminate the S1/S2 cleavage site, and a T4 fibritin trimerization domain is provided as SEQ ID NO: 33:

MFLILLISLPTAFAVIGDLKCPLDSRTGSLNNIDTGPPSISTATVDVTNGLGTYYVLDRVYLNTTLFLNGYYPTSGSTYR
NMALKGTDKLSTLWFKPPFLSDFINGIFAKVKNTKVFKDGVMYSEFPAITIGSTFVNTSYSVVVQPRTINSTQDGVNKLQ
GLLEVSVCQYNMCEYPHTICHPKLGNHFKELWHMDTGVVSCLYKRNFTYDVNATYLYFHFYQEGGTFYAYFTDTGVVTKF
LFNVYLGMALSHYYVMPLTCISRRDIGFTLEYWVTPLTSRQYLLAFNQDGIIFNAVDCMSDFMSEIKCKTQSIAPPTGVY
ELNGYTVQPIADVYRRKPDLPNCNIEAWLNDKSVPSPLNWERKTFSNCNFNMSSLMSFIQADSFTCNNIDAAKIYGMCFS
SITIDKFAIPNGRKVDLQLGNLGYLQSFNYRIDTTATSCQLYYNLPAANVSVSRFNPSTWNKRFGFIENSVFKPQPAGVL
TNHDVVYAQHCFKAPKNFCPCKLNSSLCVGSGPGKNNGIGTCPAGTNYLTCHNLCNPDPITFTGPYKCPQTKSLVGIGEH
CSGLAVKSDYCGGNPCTCQPQAFLGWSADSCLQGDKCNIFANLILHDVNSGLTCSTDLQKANTDIKLGVCVNYDLYGISG
QGIFVEVNATYYNSWQNLLYDSNGNLYGFRDYITNRTFMIRSCYSGRVSAAFHANSSEPALLFRNIKCNYVFNNSLIRQL
QPINYFDSYLGCVVNAYNSTAISVQTCDLTVGSGYCVDYSKNGGSGSAITTGYRFTNFEPFTVNSVNDSLEPVGGLYEIQ
IPSEFTIGNMEEFIQTSSPKVTIDCAAFVCGDYAACKSQLVEYGSFCDNINAILTEVNELLDTTQLQVANSLMNGVTLST
KLKDGVNFNVDDINFSSVLGCLGSECSKASSRSAIEDLLFDKVKLSDVGFVAAYNNCTGGAEIRDLICVQSYKGIKVLPP
LLSENQISGYTLAATSASLFPPWTAAAGVPFYLNVQYRINGLGVTMDVLSQNQKLIANAFNNALDAIQEGFDATNSALVK
IQAVVNANAEALNNLLQQLSNRFGAISSSLQEILSRLDPPEAEAQIDRLINGRLTALNAYVSQQLSDSTLVKFSAAQAME
KVNECVKSQSSRINFCGNGNHIISLVQNAPYGLYFIHFSYVPTKYVTAKVSPGLCIAGDRGIAPKSGYFVNVNNTWMYTG
SGYYYPEPITENNVVVMSTCAVNYTKAPYVMLNTSTPNLPDFREELDQWFKNQTSVAPDLSLDYINVTFLDLQVEMNRLQ
EAIKVLNGGYIPEAPRDGQAYVRKDGEWVLLSTF

In some embodiments, the recombinant OC43-CoV S ectodomain trimer comprises protomers comprising the ectodomain sequence of SEQ ID NO: 11. In some embodiments, the recombinant OC43-CoV S ectodomain trimer comprises protomers comprising residues 15-1287 of SEQ ID NO: 11 or residues 15-1314 of SEQ ID NO: 33. In some embodiments, the recombinant OC43-CoV S ectodomain trimer comprises protomers comprising an ectodomain sequence at least 90% identical to the ectodomain sequence of SEQ ID NO: 11 or residues 15-1314 of SEQ ID NO: 33, wherein the OC43-CoV S ectodomain trimer is stabilized in the prefusion conformation and comprises the "2P" substitution and/or modifications to remove the S1/S2 cleavage site and the S2' cleavage site of the protomers. In some embodiments, the recombinant OC43-CoV S ectodomain trimer comprises protomers comprising an amino acid sequence at least 90% identical to residues 15-1287 of SEQ ID NO: 11, wherein the OC43-CoV S ectodomain trimer is stabilized in the prefusion conformation and comprises the "2P" substitution and/or modifications to remove the S1/S2 cleavage site and the S2' cleavage site of the protomers..

### F. WIVI-CoV

In some embodiments, the immunogen comprises a recombinant WIV1-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the WIV1-CoV S ectodomain trimer in the prefusion conformation are located between residues 952 to 972 (such as between residues 962 to 972 or between residues 967 to 972) of the S ectodomain protomers in the trimer. In some embodiments, the WIV1-CoV S ectodomain trimer is stabilized in the prefusion conformation by K969P and V970P substitutions ("2P") in the S ectodomain protomers in the trimer. The amino acid numbering for WIV1-CoV S proteins is with reference to the WIV1-CoV S sequence provided as SEQ ID NO: 14.

In some embodiments, the recombinant WIV1-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant WIV1-CoV S ectodomain trimer stabilized in the prefusion conformation by the one or more proline substitutions (such as K969P and V970P substitutions) comprises additional modifications for stabilization in the prefusion conformation.

With reference to the WIV1-CoV S protein sequence provided as SEQ ID NO: 14, the ectodomain of the WIV1-CoV S protein includes about residues 16-1191. Residues 1-15 are the signal peptide, which is removed during cellular processing. The S1/S2 cleavage site is located at about position 668/669. The S2' cleavage site is located at about position 798/799. The HR1 is located at about residues 898-996. The central helix is located at about residues 971-1012. The HR2 is located at about 1146-1177. The C-terminal end of the S2 ectodomain is located at about residue 1191. In some embodiments, the protomers of the prefusion-stabilized WIV1-CoV S ectodomain trimer can have a C-terminal residue (which can be linked to a trimerization domain, or a transmembrane domain, for example) of the C-terminal residue of the HR2 (e.g., position 1177), or the ectodomain (e.g., position 1191), or from one of positions 1177-1191. The position numbering of the S protein may vary between WIV1-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary WIV1-CoV S protein sequences are provided below. The prefusion stabilizing substitutions disclosed herein (and other modifications, such as substitutions to generate a single chain) can be incorporated into WIV1-CoV S protein sequences.

An exemplary sequence of WIV1-CoV S protein (including the ectodomain and TM and CT domains) is provided as SEQ ID NO: 14 (GenBank GI: 556015140, incorporated by reference herein):

MKLLVLVFATLVSSYTIEKCLDFDDRTPPANTQFLSSHRGVYYPDDIFRSNVLHLVQDHFLPFDSNVTRFITFGLNFDNP
IIPFKDGIYFAATEKSNVIRGWVFGSTMNNKSQSVIIMNNSTNLVIRACNFELCDNPFFVVLKSNNTQIPSYIFNNAFNC
TFEYVSKDFNLDLGEKPGNFKDLREFVFRNKDGFLHVYSGYQPISAASGLPTGFNALKPIFKLPLGINITNFRTLLTAFP
PRPDYWGTSAAAYFVGYLKPTTFMLKYDENGTITDAVDCSQNPLAELKCSVKSFEIDKGIYQTSNFRVAPSKEVVRFPNI
TNLCPFGEVFNATTFPSVYAWERKRISNCVADYSVLYNSTSFSTFKCYGVSATKLNDLCFSNVYADSFVVKGDDVRQIAP
GQTGVIADYNYKLPDDFTGCVLAWNTRNIDATQTGNYNYKYRSLRHGKLRPFERDISNVPFSPDGKPCTPPAFNCYWPLN
DYGFYITNGIGYQPYRVVVLSFELLNAPATVCGPKLSTDLIKNQCVNFNFNGLTGTGVLTPSSKRFQPFQQFGRDVSDFT
DSVRDPKTSEILDISPCSFGGVSVITPGTNTSSEVAVLYQDVNCTDVPVAIHADQLTPSWRVHSTGNNVFQTQAGCLIGA
EHVDTSYECDIPIGAGICASYHTVSSLRSTSQKSIVAYTMSLGADSSIAYSNNTIAIPTNFSISITTEVMPVSMAKTSVD
CNMYICGDSTECANLLLQYGSFCTQLNRALSGIAVEQDRNTREVFAQVKQMYKTPTLKDFGGFNFSQILPDPLKPTKRSF
IEDLLFNKVTLADAGFMKQYGECLGDINARDLICAQKFNGLTVLPPLLTDDMIAAYTAALVSGTATAGWTFGAGAALQIP
FAMQMAYRFNGIGVTQNVLYENQKQIANQFNKAISQIQESLTTTSTALGKLQDVVNQNAQALNTLVKQLSSNFGAISSVL
NDILSRLDKVEAEVQIDRLITGRLQSLQTYVTQQLIRAAEIRASANLAATKMSECVLGQSKRVDFCGKGYHLMSFPQAAP
HGVVFLHVTYVPSQERNFTTAPAICHEGKAYFPREGVFVFNGTSWFITQRNFFSPQIITTDNTFVSGSCDVVIGIINNTV
YDPLQPELDSFKEELDKYFKNHTSPDVDLGDISGINASVVNIQKEIDRLNEVAKNLNESLIDLQELGKYEQYIKWPWYVW
LGFIAGLIAIVMVTILLCCMTSCCSCLKGACSCGSCCKFDEDDSEPVLKGVKLHYT

An exemplary sequence of WIV1-CoV S protein including a double proline substitution for stabilization in the prefusion conformation is provided as SEQ ID NO: 15:

MKLLVLVFATLVSSYTIEKCLDFDDRTPPANTQFLSSHRGVYYPDDIFRSNVLHLVQDHFLPFDSNVTRFITFGLNFDNP
IIPFKDGIYFAATEKSNVIRGWVFGSTMNNKSQSVIIMNNSTNLVIRACNFELCDNPFFVVLKSNNTQIPSYIFNNAFNC
TFEYVSKDFNLDLGEKPGNFKDLREFVFRNKDGFLHVYSGYQPISAASGLPTGFNALKPIFKLPLGINITNFRTLLTAFP
PRPDYWGTSAAAYFVGYLKPTTFMLKYDENGTITDAVDCSQNPLAELKCSVKSFEIDKGIYQTSNFRVAPSKEVVRFPNI
TNLCPFGEVFNATTFPSVYAWERKRISNCVADYSVLYNSTSFSTFKCYGVSATKLNDLCFSNVYADSFVVKGDDVRQIAP
GQTGVIADYNYKLPDDFTGCVLAWNTRNIDATQTGNYNYKYRSLRHGKLRPFERDISNVPFSPDGKPCTPPAFNCYWPLN
DYGFYITNGIGYQPYRVVVLSFELLNAPATVCGPKLSTDLIKNQCVNFNFNGLTGTGVLTPSSKRFQPFQQFGRDVSDFT
DSVRDPKTSEILDISPCSFGGVSVITPGTNTSSEVAVLYQDVNCTDVPVAIHADQLTPSWRVHSTGNNVFQTQAGCLIGA
EHVDTSYECDIPIGAGICASYHTVSSLRSTSQKSIVAYTMSLGADSSIAYSNNTIAIPTNFSISITTEVMPVSMAKTSVD
CNMYICGDSTECANLLLQYGSFCTQLNRALSGIAVEQDRNTREVFAQVKQMYKTPTLKDFGGFNFSQILPDPLKPTKRSF
IEDLLFNKVTLADAGFMKQYGECLGDINARDLICAQKFNGLTVLPPLLTDDMIAAYTAALVSGTATAGWTFGAGAALQIP
FAMQMAYRFNGIGVTQNVLYENQKQIANQFNKAISQIQESLTTTSTALGKLQDVVNQNAQALNTLVKQLSSNFGAISSVL
NDILSRLDPPEAEVQIDRLITGRLQSLQTYVTQQLIRAAEIRASANLAATKMSECVLGQSKRVDFCGKGYHLMSFPQAAP
HGVVFLHVTYVPSQERNFTTAPAICHEGKAYFPREGVFVFNGTSWFITQRNFFSPQIITTDNTFVSGSCDVVIGIINNTV
YDPLQPELDSFKEELDKYFKNHTSPDVDLGDISGINASVVNIQKEIDRLNEVAKNLNESLIDLQELGKYEQ

A C-terminal trimerization domain can be added to the protomers of the WIV1-CoV S ectodomains trimer to promote trimerization of the ectodomain.

An exemplary sequence of WIV1-CoV S protein including a double proline substitution for stabilization in the prefusion conformation, and a T4 fibritin trimerization domain is provided as SEQ ID NO: 34:

MKLLVLVFATLVSSYTIEKCLDFDDRTPPANTQFLSSHRGVYYPDDIFRSNVLHLVQDHFLPFDSNVTRFITFGLNFDNP
IIPFKDGIYFAATEKSNVIRGWVFGSTMNNKSQSVIIMNNSTNLVIRACNFELCDNPFFVVLKSNNTQIPSYIFNNAFNC

TFEYVSKDFNLDLGEKPGNFKDLREFVFRNKDGFLHVYSGYQPISAASGLPTGFNALKPIFKLPLGINITNFRTLLTAFP
PRPDYWGTSAAAYFVGYLKPTTFMLKYDENGTITDAVDCSQNPLAELKCSVKSFEIDKGIYQTSNFRVAPSKEVVRFPNI
TNLCPFGEVFNATTFPSVYAWERKRISNCVADYSVLYNSTSFSTFKCYGVSATKLNDLCFSNVYADSFVVKGDDVRQIAP
GQTGVIADYNYKLPDDFTGCVLAWNTRNIDATQTGNYNYKYRSLRHGKLRPFERDISNVPFSPDGKPCTPPAFNCYWPLN
DYGFYITNGIGYQPYRVVVLSFELLNAPATVCGPKLSTDLIKNQCVNFNFNGLTGTGVLTPSSKRFQPFQQFGRDVSDFT
DSVRDPKTSEILDISPCSFGGVSVITPGTNTSSEVAVLYQDVNCTDVPVAIHADQLTPSWRVHSTGNNVFQTQAGCLIGA
EHVDTSYECDIPIGAGICASYHTVSSLRSTSQKSIVAYTMSLGADSSIAYSNNTIAIPTNFSISITTEVMPVSMAKTSVD
CNMYICGDSTECANLLLQYGSFCTQLNRALSGIAVEQDRNTREVFAQVKQMYKTPTLKDFGGFNFSQILPDPLKPTKRSF
IEDLLFNKVTLADAGFMKQYGECLGDINARDLICAQKFNGLTVLPPLLTDDMIAAYTAALVSGTATAGWTFGAGAALQIP
FAMQMAYRFNGIGVTQNVLYENQKQIANQFNKAISQIQESLTTTSTALGKLQDVVNQNAQALNTLVKQLSSNFGAISSVL
NDILSRLDPPEAEVQIDRLITGRLQSLQTYVTQQLIRAAEIRASANLAATKMSECVLGQSKRVDFCGKGYHLMSFPQAAP
HGVVFLHVTYVPSQERNFTTAPAICHEGKAYFPREGVFVFNGTSWFITQRNFFSPQIITTDNTFVSGSCDVVIGIINNTV
YDPLQPELDSFKEELDKYFKNHTSPDVDLGDISGINASVVNIQKEIDRLNEVAKNLNESLIDLQELGKYEQGGYIPEAP
RDGQAYVRKDGEWVLLSTF

In some embodiments, the recombinant WIV1-CoV S ectodomain trimer comprises protomers comprising the ectodomain sequence of SEQ ID NO: 15. In some embodiments, the recombinant WIV1-CoV S ectodomain trimer comprises protomers comprising residues 16-1191 of SEQ ID NO: 15 or residues 16-1218 of SEQ ID NO: 34. In some embodiments, the recombinant WIV1-CoV S ectodomain trimer comprises protomers comprising an ectodomain sequence at least 90% identical to the ectodomain sequence of SEQ ID NO: 15, wherein the WIV1-CoV S ectodomain trimer is stabilized in the prefusion conformation and comprises the "2P" substitution and/or modifications to remove the S1/S2 cleavage site and the S2' cleavage site of the protomers. In some embodiments, the recombinant WIV1-CoV S ectodomain trimer comprises protomers comprising an amino acid sequence at least 90% identical to residues 16-1191 of SEQ ID NO: 15 or residues 16-1218 of SEQ ID NO: 34, wherein the WIV1-CoV S ectodomain trimer is stabilized in the prefusion conformation and comprises the "2P" substitution and/or modifications to remove the S1/S2 cleavage site and the S2' cleavage site of the protomers..

### G. MHV-CoV

In some embodiments, the immunogen comprises a recombinant MHV-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the MHV-CoV S ectodomain trimer in the prefusion conformation are located between residues 852 to 872 (such as between residues 862 to 872 or between residues 867 to 872) of the S ectodomain protomers in the trimer. In some embodiments, the MHV-CoV S ectodomain trimer is stabilized in the prefusion conformation by I869P and I870P substitutions ("2P") in the S ectodomain protomers in the trimer. The amino acid numbering for MHV-CoV S proteins is with reference to the MHV-CoV S sequence provided as SEQ ID NO: 16.

In some embodiments, the recombinant MHV-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant MHV-CoV S ectodomain trimer stabilized in the prefusion conformation by the one or more proline substitutions (such as I869P and I870P substitutions) comprises additional modifications for stabilization in the prefusion conformation.

With reference to the MHV-CoV S protein sequence provided as SEQ ID NO: 16, the ectodomain of the MHV-CoV S protein includes about residues 15-1297. Residues 1-14 are the signal peptide, which is removed during cellular processing. The S1/S2 cleavage site is located at about position 757/758. The S2' cleavage site is located at about position 906/907. The HR1 is located at about residues 1002-1070. The central helix is located at about residues 1075-1116. The HR2 is located at about 1252-1283. The C-terminal end of the S2 ectodomain is located at about residue 1297. In some embodiments, the protomers of the prefusion-stabilized MHV-CoV S ectodomain trimer can have a C-terminal residue (which can be linked to a trimerization domain, or a transmembrane domain, for example) of the C-terminal residue of the HR2 (e.g., position 1283), or the ectodomain (e.g., position 1297), or from one of positions 1283-1297. The position numbering of the S protein may vary between MHV-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary MHV-CoV S protein sequences are provided below. The prefusion stabilizing substitutions disclosed herein (and other modifications, such as substitutions to generate a single chain) can be incorporated into MHV-CoV S protein sequences.

An exemplary sequence of MHV-CoV S protein (including the ectodomain and TM and CT domains) is provided as SEQ ID NO: 16 (GenBank GI:328496819, incorporated by reference herein). Another exemplary MHV-CoV sequence is provided as GenBank GI:81971726, incorporated by reference herein:

MLSVFILFLPSCLGYIGDFRCINLVNTDTSNASAPSVSTEVVDVSKGIGTYYVLDRVYLNATLLLTGYYPVDGSNYRNLA
LTGTNTLSLNWYKPPFLSEFNDGIFAKVKNLKASLPKDSTSYFPTIVIGSNFVTTSYTVVLEPYNGIIMASICQYTICLL
PYTDCKPNTGGNKLIGFWHIDLKSPVCILKRNFTFNVNADWLYFHFYQQGGTFYAYYADAGSATTFLFSSYIGDVLTQYF
VLPFVCTPTTTGVFSPQYWVTPLVKRQYLFNFNQKGTITSAVDCASSYTSEIKCKTQSMNPNTGVYDLSGYTVQPVGLVY
RRVRNLPDCRIEDWLAAKTVPSPLNWERKTFQNCNFNLSSLLRLVQAGSLSCSNIDAAKVYGMCFGSMSIDKFAIPNSRR
VDLQLGNSGFLQSFNYKIDTRATSCQLYYSLAQSNVTVNNHNPSSWNRRYGFNDVATFGRGKHDVAYAEACFTVGASYCP
CANPSIVSPCTTGKPKFANCPTGTTNRECNVLALGSNLFKCDCTCNPSPLTTYDLRCLQGRSMLGVGDHCEGLGVLEDKC
GGSNTCNCSADAFVGWAKDSCLSNGRCHIFSNLMLNGINSGTTCSTDLQLPNTEVVTGICVKYDLYGITGQGVFKEVKAD
YYNSWQNLLYDVNGNLNGFRDIVTNKTYLTRSCYSGRVSAAYHQDAPEPALLYRNLKCDYVFNNNIFREETPLNYFDSYL
GCVVNADNSTEQAVDACDLRMGSGLCVNYSTAHRARTSVSTGYKLTTFEPFTVSIVNDSVESVGGLYEMQIPTNFTIASH
QEFIQTRAPKVTIDCAAFVCGDYTTCRQQLVEYGSFCDNINAILGEVNNLIDTMQLQVASALIQGVTLSSRLADGISGQI
DDINFSPLLGCLGSQCSEGTMAAQGRSTVEDLLFDKVKLSDVGFVEAYNNCTGGQEVRDLLCVQSFNGIKVLPPVLSENQ
VSGYTAGATASSMFPPWSAAAGVPFSLSVQYRINGLGVTMNVLSENQKMIASAFNNAIGAIQEGFDATNSALAKIQSVVN
ANAEALNNLLNQLSNRFGAISASLQEILSRLDALEAQAQIDRLINGRLTALNAYVSKQLSDMTLIKVSAAQAIEKVNECV
KSQSPRINFCGNGNHILSLVQNAPYGLYFLHFSYVPTSFTTANVSPGLCISGDRGLAPKAGYFVQDDGEWKFTGSNYYYP
EPITDKNSVVMSSCAVNYTKAPEVFLNTSISNLPDFKEELDKWFKNQTSVAPDLSLDFEKLNVTFLDLSDEMNRIQEAIK
KLNESYINLKEIGTYEMYVKWPWYVWLLIGLAGVAVCVLLFFICCCTGCGSCCFKKCGNCCDEYGGHQDSIVIHNISSHE
D

An exemplary sequence of MHV-CoV S ectodomain including a double proline substitution for stabilization in the prefusion conformation is provided as SEQ ID NO: 17:

MLSVFILFLPSCLGYIGDFRCINLVNTDTSNASAPSVSTEVVDVSKGIGTYYVLDRVYLNATLLLTGYYPVDGSNYRNLA
LTGTNTLSLNWYKPPFLSEFNDGIFAKVKNLKASLPKDSTSYFPTIVIGSNFVTTSYTVVLEPYNGIIMASICQYTICLL
PYTDCKPNTGGNKLIGFWHIDLKSPVCILKRNFTFNVNADWLYFHFYQQGGTFYAYYADAGSATTFLFSSYIGDVLTQYF
VLPFVCTPTTTGVFSPQYWVTPLVKRQYLFNFNQKGTITSAVDCASSYTSEIKCKTQSMNPNTGVYDLSGYTVQPVGLVY
RRVRNLPDCRIEDWLAAKTVPSPLNWERKTFQNCNFNLSSLLRLVQAGSLSCSNIDAAKVYGMCFGSMSIDKFAIPNSRR
VDLQLGNSGFLQSFNYKIDTRATSCQLYYSLAQSNVTVNNHNPSSWNRRYGFNDVATFGRGKHDVAYAEACFTVGASYCP
CANPSIVSPCTTGKPKFANCPTGTTNRECNVLALGSNLFKCDCTCNPSPLTTYDLRCLQGRSMLGVGDHCEGLGVLEDKC
GGSNTCNCSADAFVGWAKDSCLSNGRCHIFSNLMLNGINSGTTCSTDLQLPNTEVVTGICVKYDLYGITGQGVFKEVKAD
YYNSWQNLLYDVNGNLNGFRDIVTNKTYLTRSCYSGRVSAAYHQDAPEPALLYRNLKCDYVFNNNIFREETPLNYFDSYL
GCVVNADNSTEQAVDACDLRMGSGLCVNYSTAHRARTSVSTGYKLTTFEPFTVSIVNDSVESVGGLYEMQIPTNFTIASH
QEFIQTRAPKVTIDCAAFVCGDYTTCRQQLVEYGSFCDNINAILGEVNNLIDTMQLQVASALIQGVTLSSRLADGISGQI
DDINFSPLLGCLGSQCSEGTMAAQGRSTVEDLLFDKVKLSDVGFVEAYNNCTGGQEVRDLLCVQSFNGIKVLPPVLSENQ
VSGYTAGATASSMFPPWSAAAGVPFSLSVQYRINGLGVTMNVLSENQKMIASAFNNAIGAIQEGFDATNSALAKIQSVVN
ANAEALNNLLNQLSNRFGAISASLQEILSRLDPPEAQAQIDRLINGRLTALNAYVSKQLSDMTLIKVSAAQAIEKVNECV
KSQSPRINFCGNGNHILSLVQNAPYGLYFLHFSYVPTSFTTANVSPGLCISGDRGLAPKAGYFVQDDGEWKFTGSNYYYP
EPITDKNSVVMSSCAVNYTKAPEVFLNTSISNLPDFKEELDKWFKNQTSVAPDLSLDFEKLNVTFLDLSDEMNRIQEAIK
KLNESYINLKEIGTYEM

A C-terminal trimerization domain can be added to the protomers of the MHV-CoV S ectodomains trimer to promote trimerization of the ectodomain.

An exemplary sequence of MHV-CoV S ectodomain including a double proline substitution for stabilization in the prefusion conformation, and a T4 fibritin trimerization domain is provided as SEQ ID NO: 35:

MLSVFILFLPSCLGYIGDFRCINLVNTDTSNASAPSVSTEVVDVSKGIGTYYVLDRVYLNATLLLTGYYPVDGSNYRNLA
LTGTNTLSLNWYKPPFLSEFNDGIFAKVKNLKASLPKDSTSYFPTIVIGSNFVTTSYTVVLEPYNGIIMASICQYTICLL
PYTDCKPNTGGNKLIGFWHIDLKSPVCILKRNFTFNVNADWLYFHFYQQGGTFYAYYADAGSATTFLFSSYIGDVLTQYF
VLPFVCTPTTTGVFSPQYWVTPLVKRQYLFNFNQKGTITSAVDCASSYTSEIKCKTQSMNPNTGVYDLSGYTVQPVGLVY
RRVRNLPDCRIEDWLAAKTVPSPLNWERKTFQNCNFNLSSLLRLVQAGSLSCSNIDAAKVYGMCFGSMSIDKFAIPNSRR
VDLQLGNSGFLQSFNYKIDTRATSCQLYYSLAQSNVTVNNHNPSSWNRRYGFNDVATFGRGKHDVAYAEACFTVGASYCP
CANPSIVSPCTTGKPKFANCPTGTTNRECNVLALGSNLFKCDCTCNPSPLTTYDLRCLQGRSMLGVGDHCEGLGVLEDKC
GGSNTCNCSADAFVGWAKDSCLSNGRCHIFSNLMLNGINSGTTCSTDLQLPNTEVVTGICVKYDLYGITGQGVFKEVKAD
YYNSWQNLLYDVNGNLNGFRDIVTNKTYLTRSCYSGRVSAAYHQDAPEPALLYRNLKCDYVFNNNIFREETPLNYFDSYL
GCVVNADNSTEQAVDACDLRMGSGLCVNYSTAHRARTSVSTGYKLTTFEPFTVSIVNDSVESVGGLYEMQIPTNFTIASH
QEFIQTRAPKVTIDCAAFVCGDYTTCRQQLVEYGSFCDNINAILGEVNNLIDTMQLQVASALIQGVTLSSRLADGISGQI
DDINFSPLLGCLGSQCSEGTMAAQGRSTVEDLLFDKVKLSDVGFVEAYNNCTGGQEVRDLLCVQSFNGIKVLPPVLSENQ
VSGYTAGATASSMFPPWSAAAGVPFSLSVQYRINGLGVTMNVLSENQKMIASAFNNAIGAIQEGFDATNSALAKIQSVVN
ANAEALNNLLNQLSNRFGAISASLQEILSRLDPPEAQAQIDRLINGRLTALNAYVSKQLSDMTLIKVSAAQAIEKVNECV
KSQSPRINFCGNGNHILSLVQNAPYGLYFLHFSYVPTSFTTANVSPGLCISGDRGLAPKAGYFVQDDGEWKFTGSNYYYP
EPITDKNSVVMSSCAVNYTKAPEVFLNTSISNLPDFKEELDKWFKNQTSVAPDLSLDFEKLNVTFLDLSDEMNRIQEAIK
KLNESYINLKEIGTYEMGGYIPEAPRDGQAYVRKDGEWVLLSTF

In some embodiments, the recombinant MHV-CoV S ectodomain trimer comprises protomers comprising the ectodomain sequence of SEQ ID NO: 17. In some embodiments, the recombinant MHV-CoV S ectodomain trimer comprises protomers comprising residues 15-1297 of SEQ ID NO: 17 or residues 15-1324 of SEQ ID NO: 35. In some embodiments, the recombinant MHV-CoV S ectodomain trimer comprises protomers comprising an ectodomain sequence at least 90% identical to the ectodomain sequence of SEQ ID NO: 17, wherein the MHV-CoV S ectodomain trimer is stabilized in the prefusion conformation and comprises the "2P" substitution and/or modifications to remove the S1/S2 cleavage site and the S2' cleavage site of the protomers. In some embodiments, the recombinant MHV-CoV S ectodomain trimer comprises protomers comprising an amino acid sequence at least 90% identical to residues 15-1297 of SEQ ID NO: 17 or residues 15-1324 of SEQ ID NO: 35, wherein the MHV-CoV S ectodomain trimer is stabilized in the prefusion conformation and comprises the "2P" substitution and/or modifications to remove the S1/S2 cleavage site and the S2' cleavage site of the protomers.

### H. NL63-CoV

In some embodiments, the immunogen comprises a recombinant NL63-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the NL63-CoV S ectodomain trimer in the prefusion conformation are located between residues 1035 to 1055 (such as between residues 1045 to 1055 or between residues 1050 to 1055) of the S ectodomain protomers in the trimer. In some embodiments, the NL63-CoV S ectodomain trimer is stabilized in the prefusion conformation by S1052P and I1053P substitutions ("2P") in the S ectodomain protomers in the trimer. The amino acid numbering for NL63-CoV S proteins is with reference to the NL63-CoV S sequence provided as SEQ ID NO: 18.

In some embodiments, the recombinant NL63-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant NL63-CoV S ectodomain trimer stabilized in the prefusion conformation by the one or more proline substitutions (such as S1052P and I1053P substitutions) comprises additional modifications for stabilization in the prefusion conformation.

With reference to the NL63-CoV S protein sequence provided as SEQ ID NO: 18, the ectodomain of the NL63-CoV S protein includes about residues 16-1291. Residues 1-15 are the signal peptide, which is removed during cellular processing. The S1/S2 cleavage site is located at about position 748/749. The S2' cleavage site is located at about position 870/871. The HR1 is located at about residues 967-1049. The central helix is located at about residues 1054-1095. The HR2 is located at about 1246-1272. The C-terminal end of the S2 ectodomain is located at about residue 1291. In some embodiments, the protomers of the prefusion-stabilized NL63-CoV S ectodomain trimer can have a C-terminal residue (which can be linked to a trimerization domain, or a transmembrane domain, for example) of the C-terminal residue of the HR2 (e.g., position 1277), or the ectodomain (e.g., position 1291), or from one of positions 1277-1291. The position numbering of the S protein may vary between NL63-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary NL63-CoV S protein sequences are provided below. The prefusion stabilizing substitutions disclosed herein (and other modifications, such as substitutions to generate a single chain) can be incorporated into NL63-CoV S protein sequences.

An exemplary sequence of NL63-CoV S protein (including the ectodomain and TM and CT domains) is provided as SEQ ID NO: 18 (GenBank GI: 71153773, incorporated by reference herein):

MKLFLILLVLPLASCFFTCNSNANLSMLQLGVPDNSSTIVTGLLPTHWFCANQSTSVYSANGFFYIDVGNHRSAFALHTG
YYDANQYYIYVTNEIGLNASVTLKICKFSRNTTFDFLSNASSSFDCIVNLLFTEQLGAPLGITISGETVRLHLYNVTRTF
YVPAAYKLTKLSVKCYFNYSCVFSVVNATVTVNVTTHNGRVVNYTVCDDCNGYTDNIFSVQQDGRIPNGFPFNNWFLLTN
GSTLVDGVSRLYQPLRLTCLWPVPGLKSSTGFVYFNATGSDVNCNGYQHNSVVDVMRYNLNFSANSLDNLKSGVIVFKTL
QYDVLFYCSNSSSGVLDTTIPFGPSSQPYYCFINSTINTTHVSTFVGILPPTVREIVVARTGQFYINGFKYFDLGFIEAV
NFNVTTASATDFWTVAFATFVDVLVNVSATNIQNLLYCDSPFEKLQCEHLQFGLQDGFYSANFLDDNVLPETYVALPIYY
QHTDINFTATASFGGSCYVCKPHQVNISLNGNTSVCVRTSHFSIRYIYNRVKSGSPGDSSWHIYLKSGTCPFSFSKLNNF
QKFKTICFSTVEVPGSCNFPLEATWHYTSYTIVGALYVTWSEGNSITGVPYPVSGIREFSNLVLNNCTKYNIYDYVGTGI
IRSSNQSLAGGITYVSNSGNLLGFKNVSTGNIFIVTPCNQPDQVAVYQQSIIGAMTAVNESRYGLQNLLQLPNFYYVSNG
GNNCTTAVMTYSNFGICADGSLIPVRPRNSSDNGISAIITANLSIPSNWTTSVQVEYLQITSTPIVVDCATYVCNGNPRC
KNLLKQYTSACKTIEDALRLSAHLETNDVSSMLTFDSNAFSLANVTSFGDYNLSSVLPQRNIRSSRIAGRSALEDLLFSK
VVTSGLGTVDVDYKSCTKGLSIADLACAQYYNGIMVLPGVADAERMAMYTGSLIGGMVLGGLTSAAAIPFSLALQARLNY
VALQTDVLQENQKILAASFNKAINNIVASFSSVNDAITQTAEAIHTVTIALNKIQDVVNQQGSALNHLTSQLRHNFQAIS
NSIQAIYDRLDSIQADQQVDRLITGRLAALNAFVSQVLNKYTEVRGSRRLAQQKINECVKSQSNRYGFCGNGTHIFSIVN
SAPDGLLFLHTVLLPTDYKNVKAWSGICVDGIYGYVLRQPNLVLYSDNGVFRVTSRIMFQPRLPVLSDFVQIYNCNVTFV
NISRVELHTVIPDYVDVNKTLQEFAQNLPKYVKPNFDLTPFNLTYLNLSSELKQLEAKTASLFQTTVELQGLIDQINSTY
VDLKLLNRFENYIKWPWWVWLIISVVFVVLLSLLVFCCLSTGCCGCCNCLTSSMRGCCDCGSTKLPYYEFEKVHVQ

An exemplary sequence of NL63-CoV S ectodomain including a double proline substitution for stabilization in the prefusion conformation is provided as SEQ ID NO: 19:

MKLFLILLVLPLASCFFTCNSNANLSMLQLGVPDNSSTIVTGLLPTHWFCANQSTSVYSANGFFYIDVGNHRSAFALHTG
YYDANQYYIYVTNEIGLNASVTLKICKFSRNTTFDFLSNASSSFDCIVNLLFTEQLGAPLGITISGETVRLHLYNVTRTF
YVPAAYKLTKLSVKCYFNYSCVFSVVNATVTNVTTHNGRVVNYTVCDDCNGYTDNIFSVQQDGRIPNGFPFNNWFLLTN
GSTLVDGVSRLYQPLRLTCLWPVPGLKSSTGFVYFNATGSDVNCNGYQHNSVVDVMRYNLNFSANSLDNLKSGVIVFKTL
QYDVLFYCSNSSSGVLDTTIPFGPSSQPYYCFINSTINTTHVSTFVGILPPTVREIVVARTGQFYINGFKYFDLGFIEAV
NFNVTTASATDFWTVAFATFVDVLVNVSATNIQNLLYCDSPFEKLQCEHLQFGLQDGFYSANFLDDNVLPETYVALPIYY
QHTDINFTATASFGGSCYVCKPHQVNISLNGNTSVCVRTSHFSIRYIYNRVKSGSPGDSSWHIYLKSGTCPFSFSKLNNF
QKFKTICFSTVEVPGSCNFPLEATWHYTSYTIVGALYVTWSEGNSITGVPYPVSGIREFSNLVLNNCTKYNIYDYVGTGI
IRSSNQSLAGGITYVSNSGNLLGFKNVSTGNIFIVTPCNQPDQVAVYQQSIIGAMTAVNESRYGLQNLLQLPNFYYVSNG
GNNCTTAVMTYSNFGICADGSLIPVRPRNSSDNGISAIITANLSIPSNWTTSVQVEYLQITSTPIVVDCATYVCNGNPRC
KNLLKQYTSACKTIEDALRLSAHLETNDVSSMLTFDSNAFSLANVTSFGDYNLSSVLPQRNIRSSRIAGRSALEDLLFSK
VVTSGLGTVDVDYKSCTKGLSIADLACAQYYNGIMVLPGVADAERMAMYTGSLIGGMVLGGLTSAAAIPFSLALQARLNY
VALQTDVLQENQKILAASFNKAINNIVASFSSVNDAITQTAEAIHTVTIALNKIQDVVNQQGSALNHLTSQLRHNFQAIS
NSIQAIYDRLDPPQADQQVDRLITGRLAALNAFVSQVLNKYTEVRGSRRLAQQKINECVKSQSNRYGFCGNGTHIFSIVN
SAPDGLLFLHTVLLPTDYKNVKAWSGICVDGIYGYVLRQPNLVLYSDNGVFRVTSRIMFQPRLPVLSDFVQIYNCNVTFV
NISRVELHTVIPDYVDVNKTLQEFAQNLPKYVKPNFDLTPFNLTYLNLSSELKQLEAKTASLFQTTVELQGLIDQINSTY
VDLKLLNRFEN

A C-terminal trimerization domain can be added to the protomers of the NL63-CoV S ectodomains trimer to promote trimerization of the ectodomain.

An exemplary sequence of NL63-CoV S ectodomain including a double proline substitution for stabilization in the prefusion conformation, and a T4 fibritin trimerization domain is provided as SEQ ID NO: 36:

MKLFLILLVLPLASCFFTCNSNANLSMLQLGVPDNSSTIVTGLLPTHWFCANQSTSVYSANGFFYIDVGNHRSAFALHTG
YYDANQYYIYVTNE[?]GLNASVTLKICKFSRNTTFDFLSNASSSFDCIVNLLFT[?]QLGAPLGITISGETVRLHLYNVTRTF

YVPAAYKLTKLSVKCYFNYSCVFSVVNATVTVNVTTHNGRVVNYTVCDDCNGYTDNIFSVQQDGRIPNGFPFNNWFLLTN
GSTLVDGVSRLYQPLRLTCLWPVPGLKSSTGFVYFNATGSDVNCNGYQHNSVVDVMRYNLNFSANSLDNLKSGVIVFKTL
QYDVLFYCSNSSSGVLDTTIPFGPSSQPYYCFINSTINTTHVSTFVGILPPTVREIVVARTGQFYINGFKYFDLGFIEAV
NFNVTTASATDFWTVAFATFVDVLVNVSATNIQNLLYCDSPFEKLQCEHLQFGLQDGFYSANFLDDNVLPETYVALPIYY
QHTDINFTATASFGGSCYVCKPHQVNISLNGNTSVCVRTSHFSIRYIYNRVKSGSPGDSSWHIYLKSGTCPFSFSKLNNF
QKFKTICFSTVEVPGSCNFPLEATWHYTSYTIVGALYVTWSEGNSITGVPYPVSGIREFSNLVLNNCTKYNIYDYVGTGI
IRSSNQSLAGGITYVSNSGNLLGFKNVSTGNIFIVTPCNQPDQVAVYQQSIIGAMTAVNESRYGLQNLLQLPNFYYVSNG
GNNCTTAVMTYSNFGICADGSLIPVRPRNSSDNGISAIITANLSIPSNWTTSVQVEYLQITSTPIVVDCATYVCNGNPRC
KNLLKQYTSACKTIEDALRLSAHLETNDVSSMLTFDSNAFSLANVTSFGDYNLSSVLPQRNIRSSRIAGRSALEDLLFSK
VVTSGLGTVDVDYKSCTKGLSIADLACAQYYNGIMVLPGVADAERMAMYTGSLIGGMVLGGLTSAAAIPFSLALQARLNY
VALQTDVLQENQKILAASFNKAINNIVASFSSVNDAITQTAEAIHTVTIALNKIQDVVNQQGSALNHLTSQLRHNFQAIS
NSIQAIYDRLDPPQADQQVDRLITGRLAALNAFVSQVLNKYTEVRGSRRLAQQKINECVKSQSNRYGFCGNGTHIFSIVN
SAPDGLLFLHTVLLPTDYKNVKAWSGICVDGIYGYVLRQPNLVLYSDNGVFRVTSRIMFQPRLPVLSDFVQIYNCNVTFV
NISRVELHTVIPDYVDVNKTLQEFAQNLPKYVKPNFDLTPFNLTYLNLSSELKQLEAKTASLFQTTVELQGLIDQINSTY
VDLKLLNRFENGGYIPEAPRDGQAYVRKDGEWVLLSTF

In some embodiments, the recombinant NL63-CoV S ectodomain trimer comprises protomers comprising the ectodomain sequence of SEQ ID NO: 19. In some embodiments, the recombinant NL63-CoV S ectodomain trimer comprises protomers comprising residues 16-1291 of SEQ ID NO: 19 or residues 16-1318 of SEQ ID NO: 36. In some embodiments, the recombinant NL63-CoV S ectodomain trimer comprises protomers comprising an ectodomain sequence at least 90% identical to the ectodomain sequence of SEQ ID NO: 19, wherein the NL63-CoV S ectodomain trimer is stabilized in the prefusion conformation and comprises the "2P" substitution and/or modifications to remove the S1/S2 cleavage site and the S2' cleavage site of the protomers. In some embodiments, the recombinant NL63-CoV S ectodomain trimer comprises protomers comprising an amino acid sequence at least 90% identical to residues 16-1291 of SEQ ID NO: 19 or residues 16-1318 of SEQ ID NO: 36, wherein the NL63-CoV S ectodomain trimer is stabilized in the prefusion conformation and comprises the "2P" substitution and/or modifications to remove the S1/S2 cleavage site and the S2' cleavage site of the protomers.

### I. 229E-CoV

In some embodiments, the immunogen comprises a recombinant 229E-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the 229E-CoV S ectodomain trimer in the prefusion conformation are located between residues 852 to 872 (such as between residues 862 to 872 or between residues 867 to 872) of the S ectodomain protomers in the trimer. In some embodiments, the 229E-CoV S ectodomain trimer is stabilized in the prefusion conformation by I869P and I870P substitutions ("2P") in the S ectodomain protomers in the trimer. The amino acid numbering for 229E-CoV S proteins is with reference to the 229E-CoV S sequence provided as SEQ ID NO: 20.

In some embodiments, the recombinant 229E-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant 229E-CoV S ectodomain trimer stabilized in the prefusion conformation by the one or more proline substitutions (such as I869P and I870P substitutions) comprises additional modifications for stabilization in the prefusion conformation.

With reference to the 229E-CoV S protein sequence provided as SEQ ID NO: 20, the ectodomain of the 229E-CoV S protein includes about residues 17-1108. Residues 1-16 are the signal peptide, which is removed during cellular processing. The S1/S2 cleavage site is located at about position 565/566. The S2' cleavage site is located at about position 687/688. The HR1 is located at about residues 784-866. The central helix is located at about residues 871-912. The HR2 is located at about 1050-1094. The C-terminal end of the S2 ectodomain is located at about residue 1108. In some embodiments, the protomers of the prefusion-stabilized 229E-CoV S ectodomain trimer can have a C-terminal residue (which can be linked to a trimerization domain, or a transmembrane domain, for example) of the C-terminal residue of the HR2 (e.g., position 1099), or the ectodomain (e.g., position 1108), or from one of positions 1099-1108. The position numbering of the S protein may vary between 229E-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary 229E-CoV S protein sequences are provided below. The prefusion stabilizing substitutions disclosed herein (and other modifications, such as substitutions to generate a single chain) can be incorporated into 229E-CoV S protein sequences.

An exemplary sequence of 229E-CoV S protein (including the ectodomain and TM and CT domains) is provided as SEQ ID NO: 20 (GenBank GI: 1060650120, incorporated by reference herein):

MFVLLVAYALLHIAGCQTTNGTNTSHSVCNGCVGHSENVFAVESGGYIPSNFAFNNWFLLTNTSSVVDGVVRSFQPLLLN
CLWSVSGSQFTTGFVYFNGTGRGACKGFYSNASSDVIRYNINFEENLRRGTILFKTSYGAVVFYCTNNTLVSGDAHIPSG
TVLGNFYCFVNTTIGNETTSAFVGALPKTVREFVISRTGHFYINGYRYFSLGDVEAVNFNVTNAATTVCTVALASYADVL
VNVSQTAIANIIYCNSVINRLRCDQLSFDVPDGFYSTSPIQPVELPVSIVSLPVYHKHTFIVLYVNFEHRRGPGKCYNCR
PAVINITLANFNETKGPLCVDTSHFTTQFVDNVKLARWSASINTGNCPFSFGKVNNFVKFGSVCFSLKDIPGGCAMPIMA
NLVNSKSHNIGSLYVSWSDGDVITGVPKPVEGVSSFMNVTLNKCTKYNIYDVSGVGVIRISNDTFLNGITYTSTSGNLLG
FKDVTNGTIYSITPCNPPDQLVVYQQAVVGAMLSENFTSYGFSNVVEMPKFFYASNGTYNCTDAVLTYSSFGVCADGSII
AVQPRNVSYDSVSAIVTANLSIPFNWTTSVQVEYLQITSTPIVVDCSTYVCNGNVRCVELLKQYTSACKTIEDALRNSAM
LESADVSEMLTFDKKAFTLANVSSFGDYNLSSVIPSLPRSGSRVAGRSAIEDILFSKLVTSGLGTVDADYKKCTKGLSIA
DLACAQYYNGIMVLPGVADAERMAMYTGSLIGGIALGGLTSAASIPFSLAIQSRLNYVALQTDVLQENQRILAASFNKAM
TNIVDAFTGVNDAITQTSQALQTVATALNKIQDVVNQQGNSLNHLTSQLRQNFQAISSSIQAIYDRLDIIQADQQVDRLI
TGRLAALNVFVSHTLTKYTEVRASRQLAQQKVNECVKSQSKRYGFCGNGTHIFSLVNAAPEGLVFLHTVLLPTQYKDVEA
WSGLCVDGINGYVLRQPNLALYKEGNYYRITSRIMFEPRIPTIADFVQIENCNVTFVNISRSELQTIVPEYIDVNKTLQE
LSYKLPNYTVPDLVVEQYNQTILNLTSEISTLENKSAELNYTVQKLQTLIDNINSTLVDLKWLNRVETYIKWPWWVWLCI
SVVLIFVVSMLLLCCCSTGCCGFFSCFASSIRGCCESTKLPYYDVEKIHIQ

An exemplary sequence of 229E-CoV S ectodomain including a double proline substitution for stabilization in the prefusion conformation is provided as SEQ ID NO: 21:

MFVLLVAYALLHIAGCQTTNGTNTSHSVCNGCVGHSENVFAVESGGYIPSNFAFNNWFLLTNTSSVVDGVVRSFQPLLLN
CLWSVSGSQFTTGFVYFNGTGRGACKGFYSNASSDVIRYNINFEENLRRGTILFKTSYGAVVFYCTNNTLVSGDAHIPSG
TVLGNFYCFVNTTIGNETTSAFVGALPKTVREFVISRTGHFYINGYRYFSLGDVEAVNFNVTNAATTVCTVALASYADVL
VNVSQTAIANIIYCNSVINRLRCDQLSFDVPDGFYSTSPIQPVELPVSIVSLPVYHKHTFIVLYVNFEHRRGPGKCYNCR
PAVINITLANFNETKGPLCVDTSHFTTQFVDNVKLARWSASINTGNCPFSFGKVNNFVKFGSVCFSLKDIPGGCAMPIMA
NLVNSKSHNIGSLYVSWSDGDVITGVPKPVEGVSSFMNVTLNKCTKYNIYDVSGVGVIRISNDTFLNGITYTSTSGNLLG
FKDVTNGTIYSITPCNPPDQLVVYQQAVVGAMLSENFTSYGFSNVVEMPKFFYASNGTYNCTDAVLTYSSFGVCADGSII
AVQPRNVSYDSVSAIVTANLSIPFNWTTSVQVEYLQITSTPIVVDCSTYVCNGNVRCVELLKQYTSACKTIEDALRNSAM
LESADVSEMLTFDKKAFTLANVSSFGDYNLSSVIPSLPRSGSRVAGRSAIEDILFSKLVTSGLGTVDADYKKCTKGLSIA
DLACAQYYNGIMVLPGVADAERMAMYTGSLIGGIALGGLTSAASIPFSLAIQSRLNYVALQTDVLQENQRILAASFNKAM
TNIVDAFTGVNDAITQTSQALQTVATALNKIQDVVNQQGNSLNHLTSQLRQNFQAISSSIQAIYDRLDPPQADQQVDRLI
TGRLAALNVFVSHTLTKYTEVRASRQLAQQKVNECVKSQSKRYGFCGNGTHIFSLVNAAPEGLVFLHTVLLPTQYKDVEA
WSGLCVDGINGYVLRQPNLALYKEGNYYRITSRIMFEPRIPTIADFVQIENCNVTFVNISRSELQTIVPEYIDVNKTLQE
LSYKLPNYTVPDLVVEQYNQTILNLTSEISTLENKSAELNYTVQKLQTLIDNINSTLVDLKWLNRVET

A C-terminal trimerization domain can be added to the protomers of the 229E-CoV S ectodomains trimer to promote trimerization of the ectodomain.

An exemplary sequence of 229E-CoV S ectodomain including a double proline substitution for stabilization in the prefusion conformation, and a T4 fibritin trimerization domain is provided as SEQ ID NO: 37:

MFVLLVAYALLHIAGCQTTNGTNTSHSVCNGCVGHSENVFAVESGGYIPSNFAFNNWFLLTNTSSVVDGVVRSFQPLLLN
CLWSVSGSQFTTGFVYFNGTGRGACKGFYSNASSDVIRYNINFEENLRRGTILFKTSYGAVVFYCTNNTLVSGDAHIPSG
TVLGNFYCFVNTTIGNETTSAFVGALPKTVREFVISRTGHFYINGYRYFSLGDVEAVNFNVTNAATTVCTVALASYADVL
VNVSQTAIANIIYCNSVINRLRCDQLSFDVPDGFYSTSPIQPVELPVSIVSLPVYHKHTFIVLYVNFEHRRGPGKCYNCR
PAVINITLANFNETKGPLCVDTSHFTTQFVDNVKLARWSASINTGNCPFSFGKVNNFVKFGSVCFSLKDIPGGCAMPIMA
NLVNSKSHNIGSLYVSWSDGDVITGVPKPVEGVSSFMNVTLNKCTKYNIYDVSGVGVIRISNDTFLNGITYTSTSGNLLG
FKDVTNGTIYSITPCNPPDQLVVYQQAVVGAMLSENFTSYGFSNVVEMPKFFYASNGTYNCTDAVLTYSSFGVCADGSII
AVQPRNVSYDSVSAIVTANLSIPFNWTTSVQVEYLQITSTPIVVDCSTYVCNGNVRCVELLKQYTSACKTIEDALRNSAM
LESADVSEMLTFDKKAFTLANVSSFGDYNLSSVIPSLPRSGSRVAGRSAIEDILFSKLVTSGLGTVDADYKKCTKGLSIA
DLACAQYYNGIMVLPGVADAERMAMYTGSLIGGIALGGLTSAASIPFSLAIQSRLNYVALQTDVLQENQRILAASFNKAM
TNIVDAFTGVNDAITQTSQALQTVATALNKIQDVVNQQGNSLNHLTSQLRQNFQAISSSIQAIYDRLDPPQADQQVDRLI
TGRLAALNVFVSHTLTKYTEVRASRQLAQQKVNECVKSQSKRYGFCGNGTHIFSLVNAAPEGLVFLHTVLLPTQYKDVEA
WSGLCVDGINGYVLRQPNLALYKEGNYYRITSRIMFEPRIPTIADFVQIENCNVTFVNISRSELQTIVPEYIDVNKTLQE
LSYKLPNYTVPDLVVEQYNQTILNLTSEISTLENKSAELNYTVQKLQTLIDNINSTLVDLKWLNRVETGGYIPEAPRD
GQAYVRKDGEWVLLSTF

In some embodiments, the recombinant 229E-CoV S ectodomain trimer comprises protomers comprising the ectodomain sequence of SEQ ID NO: 21. In some embodiments, the recombinant 229E-CoV S ectodomain trimer comprises protomers comprising residues 17-1108 of SEQ ID NO: 21 or residues 17-1135 of SEQ ID NO: 37. In some embodiments, the recombinant 229E-CoV S ectodomain trimer comprises protomers comprising an ectodomain sequence at least 90% identical to the ectodomain sequence of SEQ ID NO: 21, wherein the 229E-CoV S ectodomain trimer is stabilized in the prefusion conformation and comprises the "2P" substitution and/or modifications to remove the S1/S2 cleavage site and the S2' cleavage site of the protomers. In some embodiments, the recombinant 229E-CoV S ectodomain trimer comprises protomers comprising an amino acid sequence at least 90% identical to residues 17-1108 of SEQ ID NO: 21 or residues 17-1135 of SEQ ID NO: 37, wherein the 229E-CoV S ectodomain trimer is stabilized in the prefusion conformation and comprises the "2P" substitution and/or modifications to remove the S1/S2 cleavage site and the S2' cleavage site of the protomers.

### I. PEDV-CoV

In some embodiments, the immunogen comprises a recombinant PEDV-CoV S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the PEDV-CoV S ectodomain trimer in the prefusion conformation are located between residues 1059 to 1079 (such as between residues 1069 to 1079 or between residues 1073 to 1079) of the S ectodomain protomers in the trimer. In some embodiments, the PEDV-CoV S ectodomain trimer is stabilized in the prefusion conformation by I1076P and L1077P substitutions ("2P") in the S ectodomain protomers in the trimer. The amino acid numbering for PEDV-CoV S proteins is with reference to the PEDV-CoV S sequence provided as SEQ ID NO: 38.

In some embodiments, the recombinant PEDV-CoV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant PEDV-CoV S ectodomain trimer stabilized in the prefusion conformation by the one or more proline substitutions (such as I1076P and L1077P substitutions) comprises additional modifications for stabilization in the prefusion conformation.

With reference to the PEDV-CoV S protein sequence provided as SEQ ID NO: 38, the ectodomain of the PEDV-CoV S protein includes about residues 21-1322. Residues 1-20 are the signal peptide, which is removed during cellular processing. The S1/S2 cleavage site is located at about position 736/737. The S2' cleavage site is located at about position 743/744. The HR1 is located at about residues 991-1073. The central helix is located at about residues 1078-1119. The HR2 is located at about 1277-1308. The C-terminal end of the S2 ectodomain is located at about residue 1322. In some embodiments, the protomers of the prefusion-stabilized PEDV-CoV S ectodomain trimer can have a C-terminal residue (which can be linked to a trimerization domain, or a transmembrane domain, for example) of the C-terminal residue of the HR2 (e.g., position 1308), or the ectodomain (e.g., position 1322), or from one of positions 1308-1322. The position numbering of the S protein may vary between PEDV-CoV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary PEDV-CoV S protein sequences are provided below. The prefusion stabilizing substitutions disclosed herein (and other modifications, such as substitutions to generate a single chain) can be incorporated into PEDV-CoV S protein sequences.

An exemplary sequence of PEDV-CoV S protein (including the ectodomain and TM and CT domains) is provided as SEQ ID NO: 38 (GenBank GI: AHZ94887.1, incorporated by reference herein):

MKSLTYFWLFLPVLSTLSLPQDVTRCSANTNFRRFFSKFNVQAPAVVVLGGYLPIGENQGVNSTWYCAGQHPTASGVHGI
FVSHIRGGHGFEIGISQEPFDPSGYQLYLHKATNGNTNATARLRICQFPSIKTLGPTANNDVTTGRNCLFNKAIPAHMSE
HSVVGITWDNDRVTVFSDKIYYFYFKNDWSRVATKCYNSGGCAMQYVYEPTYYMLNVTSAGEDGISYQPCTANCIGYAAN
VFATEPNGHIPEGFSFNNWFLLSNDSTLVHGKVVSNQPLLVNCLLAIPKIYGLGQFFSFNQTIDGVCNGAAVQRAPEALR
FNINDISVILAEGSIVLHTALGTNFSFVCSNSSNPHLATFAIPLGATQVPYYCFFKVDTYNSTVYKFLAVLPPTVREIVI
TKYGDVYVNGFGYLHLGLLDAVTINFTGHGTDDDVSGFWTIASTNFVDALIEVQGTAIQRILYCDDPVSQLKCSQVAFDL
DDGFYTISSRNLLSHEQPISFVTLPSFNDHSFVNITVSASFGGHSGANLIASDTTINGFSSFCVDTRQFTISLFYNVTNS
YGYVSKSQDSNCPFTLQSVNDYLSFSKFCVSTSLLASACTIDLFGYPEFGSGVKFTSLYFQFTKGELITGTPKPLEGVTD
VSFMTLDVCTKYTIYGFKGEGIITLTNSSFLAGVYYTSDSGQLLAFKNVTSGAVYSVTPCSFSEQAAYVDDDIVGVISSL
SSSTFNSTRELPGFFYHSNDGSNCTEPVLVYSNIGVCKSGSIGYVPSQSGQVKIAPTVTGNISIPTNFSMSIRTEYLQLY
NTPVSVDCATYVCNGNSRCKQLLTQYTAACKTIESALQLSARLESVEVNSMLTISDEALQLATISSFNGDGYNFTNVLGV
SVYDPASRRVVQKRSFIEDLLFNKVVTNGLGTVDEDYKRCSNGRSVADLVCAQYYSGVMVLPGVVDAEKLHMYSASLIGG
MVLGGFTSAAALPFSYAVQARLNYLALQTDVLQRNQQLLAESFNSAIGNITSAFESVKEAISQTSKGLNTVAHALTKVQE
VVNSQGAALTQLTVQLQHNFQAISSSIDDIYSRLDILSADAQVDRLITGRLSALNAFVAQTLTKYTEVQASRKLAQQKVN
ECVKSQSQRYGFCGGDGEHIFSLVQAAPQGLLFLHTVLVPSDFVDVIAIAGLCVNDEIALTLREPGLVLFTHELQNHTAT
EYFVSSRRMFEPRKPTVSDFVQIESCVVTYVNLTRDQLPDVIPDYIDVNKTLYEILASLPNRTGPSLPLDVFNATYLNLT
GEIADLEQRSESLRNTTEELQSLIYNINNTLVDLEWLNRVETYIKWPWWVWLIIFIVLIFVVSLLVFCCISTGCCGCCGC
CCACFSGCCRGPRLQPYEVFEKVHVQ

An exemplary sequence of PEDV-CoV S ectodomain including a double proline substitution for stabilization in the prefusion conformation is provided as SEQ ID NO: 39:

MKSLTYFWLFLPVLSTLSLPQDVTRCSANTNFRRFFSKFNVQAPAVVVLGGYLPIGENQGVNSTWYCAGQHPTASGVHGI
FVSHIRGGHGFEIGISQEPFDPSGYQLYLHKATNGNTNATARLRICQFPSIKTLGPTANNDVTTGRNCLFNKAIPAHMSE
HSVVGITWDNDRVTVFSDKIYYFYFKNDWSRVATKCYNSGGCAMQYVYEPTYYMLNVTSAGEDGISYQPCTANCIGYAAN
VFATEPNGHIPEGFSFNNWFLLSNDSTLVHGKVVSNQPLLVNCLLAIPKIYGLGQFFSFNQTIDGVCNGAAVQRAPEALR
FNINDISVILAEGSIVLHTALGTNFSFVCSNSSNPHLATFAIPLGATQVPYYCFFKVDTYNSTVYKFLAVLPPTVREIVI
TKYGDVYVNGFGYLHLGLLDAVTINFTGHGTDDDVSGFWTIASTNFVDALIEVQGTAIQRILYCDDPVSQLKCSQVAFDL
DDGFYTISSRNLLSHEQPISFVTLPSFNDHSFVNITVSASFGGHSGANLIASDTTINGFSSFCVDTRQFTISLFYNVTNS
YGYVSKSQDSNCPFTLQSVNDYLSFSKFCVSTSLLASACTIDLFGYPEFGSGVKFTSLYFQFTKGELITGTPKPLEGVTD
VSFMTLDVCTKYTIYGFKGEGIITLTNSSFLAGVYYTSDSGQLLAFKNVTSGAVYSVTPCSFSEQAAYVDDDIVGVISSL
SSSTFNSTRELPGFFYHSNDGSNCTEPVLVYSNIGVCKSGSIGYVPSQSGQVKIAPTVTGNISIPTNFSMSIRTEYLQLY
NTPVSVDCATYVCNGNSRCKQLLTQYTAACKTIESALQLSARLESVEVNSMLTISDEALQLATISSFNGDGYNFTNVLGV
SVYDPASRRVVQKRSFIEDLLFNKVVTNGLGTVDEDYKRCSNGRSVADLVCAQYYSGVMVLPGVVDAEKLHMYSASLIGG
MVLGGFTSAAALPFSYAVQARLNYLALQTDVLQRNQQLLAESFNSAIGNITSAFESVKEAISQTSKGLNTVAHALTKVQE
VVNSQGAALTQLTVQLQHNFQAISSSIDDIYSRLDPPSADAQVDRLITGRLSALNAFVAQTLTKYTEVQASRKLAQQKVN
ECVKSQSQRYGFCGGDGEHIFSLVQAAPQGLLFLHTVLVPSDFVDVIAIAGLCVNDEIALTLREPGLVLFTHELQNHTAT
EYFVSSRRMFEPRKPTVSDFVQIESCVVTYVNLTRDQLPDVIPDYIDVNKTLYEILASLPNRTGPSLPLDVFNATYLNLT
GEIADLEQRSESLRNTTEELQSLIYNINNTLVDLEWLNRVET

A C-terminal trimerization domain can be added to the protomers of the PEDV-CoV S ectodomains trimer to promote trimerization of the ectodomain.

An exemplary sequence of PEDV-CoV S ectodomain including a double proline substitution for stabilization in the prefusion conformation, and a T4 fibritin trimerization domain is provided as SEQ ID NO: 40:

MKSLTYFWLFLPVLSTLSLPQDVTRCSANTNFRRFFSKFNVQAPAVVVLGGYLPIGENQGVNSTWYCAGQHPTASGVHGI
FVSHIRGGHGFEIGISQEPFDPSGYQLYLHKATNGNTNATARLRICQFPSIKTLGPTANNDVTTGRNCLFNKAIPAHMSE
HSVVGITWDNDRVTVFSDKIYYFYFKNDWSRVATKCYNSGGCAMQYVYEPTYYMLNVTSAGEDGISYQPCTANCIGYAAN
VFATEPNGHIPEGFSFNNWFLLSNDSTLVHGKVVSNQPLLVNCLLAIPKIYGLGQFFSFNQTIDGVCNGAAVQRAPEALR
FNINDISVILAEGSIVLHTALGTNFSFVCSNSSNPHLATFAIPLGATQVPYYCFFKVDTYNSTVYKFLAVLPPTVREIVI

TKYGDVYVNGFGYLHLGLLDAVTINFTGHGTDDDVSGFWTIASTNFVDALIEVQGTAIQRILYCDDPVSQLKCSQVAFDL
DDGFYTISSRNLLSHEQPISFVTLPSFNDHSFVNITVSASFGGHSGANLIASDTTINGFSSFCVDTRQFTISLFYNVTNS
YGYVSKSQDSNCPFTLQSVNDYLSFSKFCVSTSLLASACTIDLFGYPEFGSGVKFTSLYFQFTKGELITGTPKPLEGVTD
VSFMTLDVCTKYTIYGFKGEGIITLTNSSFLAGVYYTSDSGQLLAFKNVTSGAVYSVTPCSFSEQAAYVDDDIVGVISSL
SSSTFNSTRELPGFFYHSNDGSNCTEPVLVYSNIGVCKSGSIGYVPSQSGQVKIAPTVTGNISIPTNFSMSIRTEYLQLY
NTPVSVDCATYVCNGNSRCKQLLTQYTAACKTIESALQLSARLESVEVNSMLTISDEALQLATISSFNGDGYNFTNVLGV
SVYDPASRRVVQKRSFIEDLLFNKVVTNGLGTVDEDYKRCSNGRSVADLVCAQYYSGVMVLPGVVDAEKLHMYSASLIGG
MVLGGFTSAAALPFSYAVQARLNYLALQTDVLQRNQQLLAESFNSAIGNITSAFESVKEAISQTSKGLNTVAHALTKVQE
VVNSQGAALTQLTVQLQHNFQAISSSIDDIYSRLDPPSADAQVDRLITGRLSALNAFVAQTLTKYTEVQASRKLAQQKVN
ECVKSQSQRYGFCGGDGEHIFSLVQAAPQGLLFLHTVLVPSDFVDVIAIAGLCVNDEIALTLREPGLVLFTHELQNHTAT
EYFVSSRRMFEPRKPTVSDFVQIESCVVTYVNLTRDQLPDVIPDYIDVNKTLYEILASLPNRTGPSLPLDVFNATYLNLT
GEIADLEQRSESLRNTTEELQSLIYNINNTLVDLEWLNRVETGGYIPEAPRDGQAYVRKDGEWVLLSTF

In some embodiments, the recombinant PEDV-CoV S ectodomain trimer comprises protomers comprising the ectodomain sequence of SEQ ID NO: 39. In some embodiments, the recombinant PEDV-CoV S ectodomain trimer comprises protomers comprising residues 21-1322 of SEQ ID NO: 39 or residues 21-1349 of SEQ ID NO: 40. In some embodiments, the recombinant PEDV-CoV S ectodomain trimer comprises protomers comprising an ectodomain sequence at least 90% identical to the ectodomain sequence of SEQ ID NO: 39, wherein the PEDV-CoV S ectodomain trimer is stabilized in the prefusion conformation and comprises the "2P" substitution and/or modifications to remove the S1/S2 cleavage site and the S2' cleavage site of the protomers. In some embodiments, the recombinant PEDV-CoV S ectodomain trimer comprises protomers comprising an amino acid sequence at least 90% identical to residues 21-1322 of SEQ ID NO: 39 or residues 21-1349 of SEQ ID NO: 40, wherein the PEDV-CoV S ectodomain trimer is stabilized in the prefusion conformation and comprises the "2P" substitution and/or modifications to remove the S1/S2 cleavage site and the S2' cleavage site of the protomers.

### I. SDCV

In some embodiments, the immunogen comprises a recombinant swine delta coronavirus (SDCV) S ectodomain trimer comprising protomers comprising one or more (such as two, for example two consecutive) proline substitutions at or near the boundary between a HR1 domain and a central helix domain that stabilize the S ectodomain trimer in the prefusion conformation. In some such embodiments, the one or more (such as two, for example two consecutive) proline substitutions that stabilize the S ectodomain in the prefusion conformation are located between a position 15 amino acids N-terminal of a C-terminal residue of the HR1 and a position 5 amino acids C-terminal of a N-terminal residue of the central helix.

In some embodiments, the one or more (such as two, for example two consecutive) proline substitutions that stabilize the SDCV S ectodomain trimer in the prefusion conformation are located between residues 838 to 858 (such as between residues 848 to 858 or between residues 854 to 858) of the S ectodomain protomers in the trimer. In some embodiments, the SDCV S ectodomain trimer is stabilized in the prefusion conformation by E855P and V856P substitutions ("2P") in the S ectodomain protomers in the trimer. The amino acid numbering for SDCV S proteins is with reference to the SDCV S sequence provided as SEQ ID NO: 41.

In some embodiments, the recombinant SDCV S ectodomain trimer stabilized in the prefusion conformation comprises single-chain S ectodomain protomers comprising mutations to the S1/S2 and/or S2' protease cleavage sites to prevent protease cleavage at these sites.

In some embodiments, the protomers of the recombinant SDCV S ectodomain trimer stabilized in the prefusion conformation by the one or more proline substitutions (such as E855P and V856P substitutions) comprises additional modifications for stabilization in the prefusion conformation.

With reference to the SDCV S protein sequence provided as SEQ ID NO: 41, the ectodomain of the SDCV S protein includes about residues 20-1093. Residues 1-19 are the signal peptide, which is removed during cellular processing. The HR1 is located at about residues 770-854. The central helix is located at about residues 857-898. The HR2 is located at about 1034-1079. The C-terminal end of the S2 ectodomain is located at about residue 1093. In some embodiments, the protomers of the prefusion-stabilized SDCV S ectodomain trimer can have a C-terminal residue (which can be linked to a trimerization domain, or a transmembrane domain, for example) of the C-terminal residue of the HR2 (e.g., position 1079), or the ectodomain (e.g., position 1093), or from one of positions 1079-1093. The position numbering of the S protein may vary between SDCV stains, but the sequences can be aligned to determine relevant structural domains and cleavage sites. It will be appreciated that a few residues (such as up to 10) on the N and C-terminal ends of the ectodomain can be removed or modified in the disclosed immunogens without decreasing the utility of the S ectodomain trimer as an immunogen.

Exemplary SDCV S protein sequences are provided below. The prefusion stabilizing substitutions disclosed herein (and other modifications, such as substitutions to generate a single chain) can be incorporated into SDCV S protein sequences.

An exemplary sequence of SDCV S protein (including the ectodomain and TM and CT domains) is provided as SEQ ID NO: 41 (GenBank GI: AMN91621.1, incorporated by reference herein):

MQRALLIMTLLCLARAKFADDLLDLLTFPGAHRFLHKPTRNDSILYSRANNNFDVGVLPGYPTKNVNLFSPLTNSTLPIN
GLHRSYQPLMLNCLTKITNQTLSMYLQPSEIQTYSCGGAMVKYQTHDAVRIILDLIATDRISVEVVGQAGENYVFVCSDQ
FNYTTALHNSTFFSLNSQLYCFTNNTYLGILPPDLTDFTVYRTGQFYANGYLLGTLPITVNYVRLYRGQLSANSAHFALA
NLTDTLITLTNTTISQITYCDKSVVDSIACQRSSHQVEDGFYSDPKSAVRARQRTIVTLPKLPELEVVQLNISAHMDFGE
ARLDSVTINGNTSYCVTKPYFRLETNFLCRGCTMNLRTDTCSFDLSAVNNGMSFSQFCLSTESGACEMKIIVTYVWNYLL
RQRLYVTAVEGQTHTGTTSVHATDTSSVITDVCTDYTIYGVSGTGIIKPSDLLLHNGIAFTSPTGELYAFKNITTGKTLQ
VLPCETPSQLIVINNTVVGAITSSNSTENNRFTTTIVTPTFFYSTNATTLNCTKPVLSYGPISVCSDGAIAGTSTLQNTR
PSIVSLYDGEIEIPSAFSLSVQTEYLQVQAEQVIVDCPQYVCNGNSRCLQLLAQYTSACSNIEVALHSSAQLDSREIISM
FKTSTQSLQLANITNFKGDYNFSSILTSRVGGRSAIEDLLFNKVVTSGLGTVDQDYKSCSRNMAIADLVCSQYYNGIMVL
PGVVDAEKMAMYTGSLTGAMVFGGLTAAAAIPFATAVQARLNYVALQTNVLQENQKILAESFNQAVGNISLALSSVNDAI
QQTSEALNTVAIAIKKIQTVVNQQGEALSHLTAQLSNNFQAISTSIQDIYNRLEEVEANQQVDRLINGRLAALNAYVTQL
LNQMSQIRQSRLLAQQKINECVKSQSPRYGFCGNGTHIFSLTQTAPNGIFFMHAVLVPNKFTRVNASAGICVDNTRGYSL
QPQLILYQFNNSWRVTPRNMYEPRLPRQADFIQLTDCSVTFYNTTAANLPNIIPDVIDVNQTVSDIIDNLPTATPPQWDV
GIYNNTILNLTVEINDLQERSKNLSQIADRLQNYIDNLNNTLVDLEWLNRVETYLKWPWYIWLAIALALIAFVTILITIF
LCTGCCGGCFGCCGGCFGLFSKKKRYTDDQPTPSFKFKEW

An exemplary sequence of SDCV S ectodomain including a double proline substitution for stabilization in the prefusion conformation is provided as SEQ ID NO: 42:

MQRALLIMTLLCLARAKFADDLLDLLTFPGAHRFLHKPTRNDSILYSRANNNFDVGVLPGYPTKNVNLFSPLTNSTLPIN
GLHRSYQPLMLNCLTKITNQTLSMYLQPSEIQTYSCGGAMVKYQTHDAVRIILDLIATDRISVEVVGQAGENYVFVCSDQ
FNYTTALHNSTFFSLNSQLYCFTNNTYLGILPPDLTDFTVYRTGQFYANGYLLGTLPITVNYVRLYRGQLSANSAHFALA
NLTDTLITLTNTTISQITYCDKSVVDSIACQRSSHQVEDGFYSDPKSAVRARQRTIVTLPKLPELEVVQLNISAHMDFGE

ARLDSVTINGNTSYCVTKPYFRLETNFLCRGCTMNLRTDTCSFDLSAVNNGMSFSQFCLSTESGACEMKIIVTYVWNYLL
RQRLYVTAVEGQTHTGTTSVHATDTSSVITDVCTDYTIYGVSGTGIIKPSDLLLHNGIAFTSPTGELYAFKNITTGKTLQ
VLPCETPSQLIVINNTVVGAITSSNSTENNRFTTTIVTPTFFYSTNATTLNCTKPVLSYGPISVCSDGAIAGTSTLQNTR
PSIVSLYDGEIEIPSAFSLSVQTEYLQVQAEQVIVDCPQYVCNGNSRCLQLLAQYTSACSNIEVALHSSAQLDSREIISM
FKTSTQSLQLANITNFKGDYNFSSILTSRVGGRSAIEDLLFNKVVTSGLGTVDQDYKSCSRNMAIADLVCSQYYNGIMVL
PGVVDAEKMAMYTGSLTGAMVFGGLTAAAAIPFATAVQARLNYVALQTNVLQENQKILAESFNQAVGNISLALSSVNDAI
QQTSEALNTVAIAIKKIQTVVNQQGEALSHLTAQLSNNFQAISTSIQDIYNRLEPPEANQQVDRLINGRLAALNAYVTQL
LNQMSQIRQSRLLAQQKINECVKSQSPRYGFCGNGTHIFSLTQTAPNGIFFMHAVLVPNKFTRVNASAGICVDNTRGYSL
QPQLILYQFNNSWRVTPRNMYEPRLPRQADFIQLTDCSVTFYNTTAANLPNIIPDVIDVNQTVSDIIDNLPTATPPQWDV
GIYNNTILNLTVEINDLQERSKNLSQIADRLQNYIDNLNNTLVDLEWLNRVET

A C-terminal trimerization domain can be added to the protomers of the SDCV S ectodomains trimer to promote trimerization of the ectodomain.

An exemplary sequence of SDCV S ectodomain including a double proline substitution for stabilization in the prefusion conformation, and a T4 fibritin trimerization domain is provided as SEQ ID NO: 43:

MQRALLIMTLLCLARAKFADDLLDLLTFPGAHRFLHKPTRNDSILYSRANNNFDVGVLPGYPTKNVNLFSPLTNSTLPIN
GLHRSYQPLMLNCLTKITNQTLSMYLQPSEIQTYSCGGAMVKYQTHDAVRIILDLIATDRISVEVVGQAGENYVFVCSDQ
FNYTTALHNSTFFSLNSQLYCFTNNTYLGILPPDLTDFTVYRTGQFYANGYLLGTLPITVNYVRLYRGQLSANSAHFALA
NLTDTLITLTNTTISQITYCDKSVVDSIACQRSSHQVEDGFYSDPKSAVRARQRTIVTLPKLPELEVVQLNISAHMDFGE
ARLDSVTINGNTSYCVTKPYFRLETNFLCRGCTMNLRTDTCSFDLSAVNNGMSFSQFCLSTESGACEMKIIVTYVWNYLL
RQRLYVTAVEGQTHTGTTSVHATDTSSVITDVCTDYTIYGVSGTGIIKPSDLLLHNGIAFTSPTGELYAFKNITTGKTLQ
VLPCETPSQLIVINNTVVGAITSSNSTENNRFTTTIVTPTFFYSTNATTLNCTKPVLSYGPISVCSDGAIAGTSTLQNTR
PSIVSLYDGEIEIPSAFSLSVQTEYLQVQAEQVIVDCPQYVCNGNSRCLQLLAQYTSACSNIEVALHSSAQLDSREIISM
FKTSTQSLQLANITNFKGDYNFSSILTSRVGGRSAIEDLLFNKVVTSGLGTVDQDYKSCSRNMAIADLVCSQYYNGIMVL
PGVVDAEKMAMYTGSLTGAMVFGGLTAAAAIPFATAVQARLNYVALQTNVLQENQKILAESFNQAVGNISLALSSVNDAI
QQTSEALNTVAIAIKKIQTVVNQQGEALSHLTAQLSNNFQAISTSIQDIYNRLEPPEANQQVDRLINGRLAALNAYVTQL
LNQMSQIRQSRLLAQQKINECVKSQSPRYGFCGNGTHIFSLTQTAPNGIFFMHAVLVPNKFTRVNASAGICVDNTRGYSL
QPQLILYQFNNSWRVTPRNMYEPRLPRQADFIQLTDCSVTFYNTTAANLPNIIPDVIDVNQTVSDIIDNLPTATPPQWDV
GIYNNTILNLTVEINDLQERSKNLSQIADRLQNYIDNLNNTLVDLEWLNRVETGGYIPEAPRDGQAYVRKDGEWVLLSTF

In some embodiments, the recombinant SDCV S ectodomain trimer comprises protomers comprising the ectodomain sequence of SEQ ID NO: 42. In some embodiments, the recombinant SDCV S ectodomain trimer comprises protomers comprising residues 20-1093 of SEQ ID NO: 42 or residues 20-1120 of SEQ ID NO: 43. In some embodiments, the recombinant SDCV S ectodomain trimer comprises protomers comprising an ectodomain sequence at least 90% identical to the ectodomain sequence of SEQ ID NO: 39, wherein the SDCV S ectodomain trimer is stabilized in the prefusion conformation and comprises the "2P" substitution and/or modifications to remove the S1/S2 cleavage site and the S2' cleavage site of the protomers. In some embodiments, the recombinant SDCV S ectodomain trimer comprises protomers comprising an amino acid sequence at least 90% identical to residues 20-1093 of SEQ ID NO: 39 or residues 20-1120 of SEQ ID NO: 43, wherein the SDCV S ectodomain trimer is stabilized in the prefusion conformation and comprises the "2P" substitution and/or modifications to remove the S1/S2 cleavage site and the S2' cleavage site of the protomers.

### J. Single Chain S proteins

In some embodiments, the CoV S ectodomain trimer can be composed of three single-chain CoV S ectodomain protomers, each including a single polypeptide chain including the S1 protein and S2 ectodomain. Single chain CoV S ectodomain protomers can be generated by mutating the S1/S2 and S2' protease cleavage sites to prevent cleavage and formation of distinct S1 and S2 polypeptide chains. In some embodiments, the S1 and S2 polypeptides in the single chain CoV S ectodomain protomers are joined by a linker, such as a peptide linker. Examples of peptide linkers that can be used include glycine, serine, and glycine-serine linkers. Any of the stabilizing mutations (or combinations thereof) disclosed herein can be included in the single chain coronavirus S ectodomain protomers as long as the coronavirus S ectodomain trimer composed of such protomers retains the desired properties (e.g., the prefusion conformation).

### K. Linkage to a Trimerization Domain

In several embodiments, the S ectodomain protomers in the disclosed coronavirus S ectodomain trimers can be linked at their C-terminus (C-terminal linkage) to a trimerization domain to promote trimerization of the S ectodomain protomers, and to stabilize the membrane proximal aspect of the recombinant S ectodomains in a trimeric configuration.

Non-limiting examples of exogenous multimerization domains that promote stable trimers of soluble recombinant proteins include: the GCN4 leucine zipper (Harbury et al. 1993 Science 262:1401-1407), the trimerization motif from the lung surfactant protein (Hoppe et al. 1994 FEBS Lett 344:191-195), collagen (McAlinden et al. 2003 J Biol Chem 278:42200-42207), and the phage T4 fibritin Foldon (Miroshnikov et al. 1998 Protein Eng 11:329-414), any of which can be linked to a recombinant coronavirus S ectodomain described herein *(e.g.,* by linkage to the C-terminus of S2) to promote trimerization of the recombinant coronavirus S ectodomain.

In some examples, the C-terminus of the S2 subunit of the S ectodomain can be linked to a T4 fibritin Foldon domain. In specific examples, the T4 fibritin Foldon domain can include the amino acid sequence GYIPEAPRDGQAYVRKDGEWVLLSTF (SEQ ID NO: 27), which adopts a β-propeller conformation, and can fold and trimerize in an autonomous way (Tao et al. 1997 Structure 5:789-798).

Optionally, the heterologous trimerization is connected to the recombinant coronavirus S ectodomain via a peptide linker, such as an amino acid linker. Non-limiting examples of peptide linkers that can be used include glycine, serine, and glycine-serine linkers.

### L. Membrane anchored embodiments

In some embodiments, the coronavirus S ectodomain trimer can be membrane anchored, for example, for embodiments where the coronavirus S ectodomain trimer is expressed on an attenuated viral vaccine, or a virus like particle. In such embodiments, the protomers in the trimer typically each comprise a C-terminal linkage to a transmembrane domain, such as the transmembrane domain (and optionally the cytosolic tail) of corresponding coronavirus. For example, the protomers of a disclosed SARS-CoV S ectodomain trimer can be linked to a SARS-CoV S transmembrane and cytosolic tail. In some embodiments, one or more peptide linkers (such as a gly-ser linker, for example, a 10 amino acid glycine-serine peptide linker can be used to link the recombinant S ectodomain protomer to the transmembrane domain. The protomers linked to the transmembrane domain can include any of the stabilizing mutations provided herein (or combinations thereof) as long as the recombinant coronavirus S ectodomain trimer formed from the protomers linked to the transmembrane domain retains the desired properties (e.g., the coronavirus S prefusion conformation).

### M. Additional description

The coronavirus S protein or fragments thereof can be produced using recombinant techniques, or chemically or enzymatically synthesized.

Analogs and variants of the coronavirus S protein or fragments thereof may be used in the methods and systems of the present invention. Through the use of recombinant DNA technology, variants of the coronavirus S protein or fragments thereof may be prepared by altering the underlying DNA. All such variations or alterations in the structure of the coronavirus S ectodomain or fragments thereof resulting in variants are included within the scope of this invention. Such variants include insertions, substitutions, or deletions of one or more amino acid residues, glycosylation variants, unglycosylated coronavirus S ectodomain or fragments thereof, organic and inorganic salts, covalently modified derivatives of the coronavirus S protein or fragments thereof, or a precursor thereof. Such variants may maintain one or more of the functional, biological activities of the coronavirus S protein or fragment thereof, such as binding to cell surface receptor. The coronavirus S protein or a fragment thereof can be modified, for example, by PEGylation, to increase the half-life of the protein in the recipient, to retard clearance from the pericardial space, and/or to make the protein more stable for delivery to a subject.

In some embodiments, a coronavirus S protein or fragment thereof useful within the disclosure is modified to produce peptide mimetics by replacement of one or more naturally occurring side chains of the 20 genetically encoded amino acids (or D-amino acids) with other side chains, for example with groups such as alkyl, lower alkyl, cyclic 4-, 5-, 6-, to 7-membered alkyl, amide, amide lower alkyl, amide di(lower alkyl), lower alkoxy, hydroxy, carboxy and the lower ester derivatives thereof, and with 4-, 5-, 6-, to 7-membered heterocyclics. For example, proline analogs can be made in which the ring size of the proline residue is changed from a 5-membered ring to a 4-, 6-, or 7-membered ring. Cyclic groups can be saturated or unsaturated, and if unsaturated, can be aromatic or non-aromatic. Heterocyclic groups can contain one or more nitrogen, oxygen, and/or sulphur heteroatoms. Examples of such groups include furazanyl, furyl, imidazolidinyl, imidazolyl, imidazolinyl, isothiazolyl, isoxazolyl, morpholinyl (e.g., morpholino), oxazolyl, piperazinyl *(e.g.,* 1-piperazinyl), piperidyl *(e.g.,* 1-piperidyl, piperidino), pyranyl, pyrazinyl, pyrazolidinyl, pyrazolinyl, pyrazolyl, pyridazinyl, pyridyl, pyrimidinyl, pyrrolidinyl (e.g., 1-pyrrolidinyl), pyrrolinyl, pyrrolyl, thiadiazolyl, thiazolyl, thienyl, thiomorpholinyl (e.g., thiomorpholino), and triazolyl groups. These heterocyclic groups can be substituted or unsubstituted. Where a group is substituted, the substituent can be alkyl, alkoxy, halogen, oxygen, or substituted or unsubstituted phenyl. Peptides, as well as peptide analogs and mimetics, can also be covalently bound to one or more of a variety of nonproteinaceous polymers, for example, polyethylene glycol, polypropylene glycol, or polyoxyalkenes, as described in U.S. Patent Nos. 4,640,835; 4,496,668; 4,301,144; 4,668,417; 4,791,192; and 4,179,337.

### N. Protein Nanoparticles

In some embodiments a protein nanoparticle is provided that includes one or more of the disclosed recombinant coronavirus S ectodomain trimers (e.g., a MERS-CoV S ectodomain trimer or a SARS-CoV S ectodomain trimer). Non-limiting example of nanoparticles include ferritin nanoparticles, encapsulin nanoparticles, Sulfur Oxygenase Reductase (SOR) nanoparticles, and lumazine synthase nanoparticles, which are comprised of an assembly of monomeric subunits including ferritin proteins, encapsulin proteins, SOR proteins, and lumazine synthase, respectively. Additional protein nanoparticle structures are described by Heinze et al., J Phys Chem B., 120(26):5945-52, 2016; Hsia et al., Nature, 535(7610):136-9, 2016; and King et al., Nature, 510(7503):103-8, 2014; each of which is incorporated by reference herein. To construct such protein nanoparticles a protomer of the coronavirus S ectodomain trimer can be linked to a subunit of the protein nanoparticle (such as a ferritin protein, an encapsulin protein, a SOR protein, or a lumazine synthase protein) and expressed in cells under appropriate conditions. The fusion protein self-assembles into a nanoparticle any can be purified.

In some embodiments, a protomer of a disclosed recombinant coronavirus S ectodomain trimer (e.g., a MERS-CoV S ectodomain trimer or a SARS-CoV S ectodomain trimer) can be linked to a ferritin subunit to construct a ferritin nanoparticle. Ferritin nanoparticles and their use for immunization purposes (e.g., for immunization against influenza antigens) have been disclosed in the art (see, e.g., Kanekiyo et al., Nature, 499:102-106, 2013, incorporated by reference herein in its entirety). Ferritin is a globular protein that is found in all animals, bacteria, and plants, and which acts primarily to control the rate and location of polynuclear Fe(III)₂O₃ formation through the transportation of hydrated iron ions and protons to and from a mineralized core. The globular form of the ferritin nanoparticle is made up of monomeric subunits, which are polypeptides having a molecule weight of approximately 17-20 kDa. An example of the amino acid sequence of one such monomeric ferritin subunit is represented by:

ESQVRQQFSKDIEKLLNEQVNKEMQSSNLYMSMSSWCYTHSLDGAGLFLFDHAAEEYEHAKKLIIFLNENNVPVQLTSIS
APEHKFEGLTQIFQKAYEHEQHISESINNIVDHAIKSKDHATFNFLQWYVAEQHEEEVLFKDILDKIELIGNENHGLYLA
DQYVKGIAKSRKS (SEQ ID NO: 23)

Each monomeric subunit has the topology of a helix bundle which includes a four antiparallel helix motif, with a fifth shorter helix (the c-terminal helix) lying roughly perpendicular to the long axis of the 4 helix bundle. According to convention, the helices are labeled 'A, B, C, D & E' from the N-terminus respectively. The N-terminal sequence lies adjacent to the capsid three-fold axis and extends to the surface, while the E helices pack together at the four-fold axis with the C-terminus extending into the capsid core. The consequence of this packing creates two pores on the capsid surface. It is expected that one or both of these pores represent the point by which the hydrated iron diffuses into and out of the capsid. Following production, these monomeric subunit proteins self-assemble into the globular ferritin protein. Thus, the globular form of ferritin comprises 24 monomeric, subunit proteins, and has a capsid-like structure having 432 symmetry. Methods of constructing ferritin nanoparticles are known to the person of ordinary skill in the art and are further described herein (see, *e.g.,* Zhang, Int. J. Mol. Sci., 12:5406-5421, 2011, which is incorporated herein by reference in its entirety).

In specific examples, the ferritin polypeptide is E. *coli* ferritin, *Helicobacter pylori* ferritin, human light chain ferritin, bullfrog ferritin or a hybrid thereof, such as E. *coli-human* hybrid ferritin, E. coli-bullfrog hybrid ferritin, or human-bullfrog hybrid ferritin. Exemplary amino acid sequences of ferritin polypeptides and nucleic acid sequences encoding ferritin polypeptides for use to make a ferritin nanoparticle including a recombinant coronavirus S ectodomain can be found in GENBANK^{®}, for example at accession numbers ZP_03085328, ZP_06990637, EJB64322.1, AAA35832, NP_000137 AAA49532, AAA49525, AAA49524 and AAA49523, which are specifically incorporated by reference herein in their entirety as available April 10, 2015. In some embodiments, a recombinant coronavirus S ectodomain can be linked to a ferritin subunit including an amino acid sequence at least 80% (such as at least 85%, at least 90%, at least 95%, or at least 97%) identical to amino acid sequence set forth as SEQ ID NO: 122.

In some embodiments, a protomer of a disclosed recombinant coronavirus S ectodomain trimer (e.g., a MERS-CoV S ectodomain trimer or a SARS-CoV S ectodomain trimer) can be linked to a lumazine synthase subunit to construct a lumazine synthase nanoparticle. The globular form of lumazine synthase nanoparticle is made up of monomeric subunits; an example of the sequence of one such lumazine synthase subunit is provides as the amino acid sequence set forth as:

MQIYEGKLTAEGLRFGIVASRFNHALVDRLVEGAIDAIVRHGGREEDITLVRVPGSWEIPVAAGELARKEDIDAVIAIGV
LIRGATPHFDYIASEVSKGLADLSLELRKPITFGVITADTLEQAIERAGTKHGNKGWEAALSAIEMANLFKSLR (SEQ
ID NO: 24).

In some embodiments, a protomer of a disclosed recombinant coronavirus S ectodomain trimer can be linked to a lumazine synthase subunit including an amino acid sequence at least 80% (such as at least 85%, at least 90%, at least 95%, or at least 97%) identical to amino acid sequence set forth as SEQ ID NO: 24.

In some embodiments, a protomer of a disclosed recombinant coronavirus S ectodomain trimer (e.g., a MERS-CoV S ectodomain trimer or a SARS-CoV S ectodomain trimer) can be linked to an encapsulin nanoparticle subunit to construct an encapsulin nanoparticle. The globular form of the encapsulin nanoparticle is made up of monomeric subunits; an example of the sequence of one such encapsulin subunit is provides as the amino acid sequence set forth as




 (SEQ ID NO: 25).

In some embodiments, a protomer of a disclosed recombinant coronavirus S ectodomain trimer can be linked to an encapsulin subunit including an amino acid sequence at least 80% (such as at least 85%, at least 90%, at least 95%, or at least 97%) identical to amino acid sequence set forth as SEQ ID NO: 25.

Encapsulin proteins are a conserved family of bacterial proteins also known as linocin-like proteins that form large protein assemblies that function as a minimal compartment to package enzymes. The encapsulin assembly is made up of monomeric subunits, which are polypeptides having a molecule weight of approximately 30 kDa. Following production, the monomeric subunits self-assemble into the globular encapsulin assembly including 60, or in some cases, 180 monomeric subunits. Methods of constructing encapsulin nanoparticles are known to the person of ordinary skill in the art, and further described herein (see, for example, Sutter et al., Nature Struct. and Mol. Biol., 15:939-947, 2008, which is incorporated by reference herein in its entirety). In specific examples, the encapsulin polypeptide is bacterial encapsulin, such as *Thermotoga maritime* or *Pyrococcus furiosus* or *Rhodococcus erythropolis* or *Myxococcus xanthus* encapsulin.

In some embodiments, a protomer of a disclosed recombinant coronavirus S ectodomain trimer (e.g., a MERS-CoV S ectodomain trimer or a SARS-CoV S ectodomain trimer) can be linked to a Sulfur Oxygenase Reductase (SOR) subunit to construct a recombinant SOR nanoparticle. In some embodiments, the SOR subunit can include the amino acid sequence set forth as




 (SEQ ID NO: 26).

In some embodiments, a protomer of a disclosed recombinant coronavirus S ectodomain trimer can be linked to a SOR subunit including an amino acid sequence at least 80% (such as at least 85%, at least 90%, at least 95%, or at least 97%) identical to amino acid sequence set forth as SEQ ID NO: 26.

SOR proteins are microbial proteins (for example from the thermoacidophilic archaeon *Acidianus ambivalens* that form 24 subunit protein assemblies. Methods of constructing SOR nanoparticles are known to the person of ordinary skill in the art (see, *e.g.,* Urich et al., Science, 311:996-1000, 2006, which is incorporated by reference herein in its entirety). An example of an amino acid sequence of a SOR protein for use to make SOR nanoparticles is set forth in Urich et al., Science, 311:996-1000, 2006, which is incorporated by reference herein in its entirety.

For production purposes, the recombinant coronavirus S ectodomain linked to the nanoparticle subunit can include an N-terminal signal peptide that is cleaved during cellular processing. For example, the recombinant coronavirus S ectodomain protomer linked to the protein nanoparticle subunit can include a signal peptide at its N-terminus including, for example, a native coronavirus S signal peptide .

The protein nanoparticles can be expressed in appropriate cells (e.g., HEK 293 Freestyle cells) and fusion proteins are secreted from the cells self-assembled into nanoparticles. The nanoparticles can be purified using known techniques, for example by a few different chromatography procedures, *e.g.* Mono Q (anion exchange) followed by size exclusion (SUPEROSE^{®} 6) chromatography.

Several embodiments include a monomeric subunit of a ferritin, encapsulin, SOR, or lumazine synthase protein, or any portion thereof which is capable of directing self-assembly of monomeric subunits into the globular form of the protein. Amino acid sequences from monomeric subunits of any known ferritin, encapsulin, SOR, or lumazine synthase protein can be used to produce fusion proteins with the recombinant coronavirus S ectodomain or immunogenic fragment thereof, so long as the monomeric subunit is capable of self-assembling into a nanoparticle displaying the recombinant coronavirus S ectodomain or immunogenic fragment thereof on its surface.

The fusion proteins need not comprise the full-length sequence of a monomeric subunit polypeptide of a ferritin, encapsulin, SOR, or lumazine synthase protein. Portions, or regions, of the monomeric subunit polypeptide can be utilized so long as the portion comprises amino acid sequences that direct self-assembly of monomeric subunits into the globular form of the protein.

### III. Polynucleotides and Expression

Polynucleotides encoding a protomer of any of the disclosed recombinant S ectodomain trimers are also provided. These polynucleotides include DNA, cDNA and RNA sequences which encode the protomer, as well as vectors including the DNA, cDNA and RNA sequences, such as a DNA or RNA vector used for immunization. The genetic code to construct a variety of functionally equivalent nucleic acids, such as nucleic acids which differ in sequence but which encode the same protein sequence, or encode a conjugate or fusion protein including the nucleic acid sequence.

An exemplary nucleic acid sequence encoding MERS-CoV S protein is provided as SEQ ID NO: 5: 
atgattcactccgtgttcctgctgatgttcctgctgactcctacagagagctatgtggatgtgggacctgattccgtcaa
gagcgcctgcatcgaagtggacattcagcagaccttctttgataagacatggccaagacccatcgacgtgagcaaagccg
atggcatcatctaccctcagggaggacctattccaatatcacaattacttaccaggcctgttcccatatcagggagac
cacggcgatatgtacgtgtattctgctggccatgcaacagggaccacacctcagaagctgtttgtggctaactacagcca
ggacgtcaaacagttcgcaaatggatttgtggtccgcatcggcgccgctgcaaactctaccggcacagtgatcatttcac
ctagcacttccgcaaccatccgaaaaatctacccagccttcatgctgggaagctccgtgggcaatttagcgacgggaaa
atgggacggttctttaaccacaccctggtgctgctgcctgatggatgcggcacactgctgagggctttctactgtatcct
ggagccacgcagcggaaaccactgccccgcaggaaatagctacacctcctttgccacatatcatactccagctaccgact
gttccgatggcaactacaatcgaaacgcctctctgaatagtttcaaggaatacttcaacctgcggaattgcacattcatg
tacacttataacatcaccgaggacgaaattctggagtggttcggaatcactcagaccgcacagggcgtgcacctgttttc
tagtcgctacgtcgacctgtatggcgggaacatgttccagtttgccactctgcccgtgtacgataccatcaagtactatt
ccatcattcctcattcaatccgcagcattcagtccgatcgaaaggcttgggccgctttctacgtgtataaactgcagcca
ctgaccttcctgctggactttagcgtcgatggctacatccggagagccattgactgcgggtttaatgatctgtcccagct
gcactgttcttacgaaagtttcgacgtggagtccggcgtgtattctgtctcaagctttgaggccaagccctctgggagtg
tggtcgagcaggctgaaggagtggagtgcgatttcagtcctctgctgtcagggacccccccctcaggtgtacaacttcaag
cggctggtctttactaactgtaactacaatctgaccaagctgctgtcactgttcagcgtgaatgactttacatgctccca
gatcagccccgcagccattgctagtaactgttactcctctctgatcctggactacttctcatatccactgagtatgaaga
gcgacctgagcgtgagttcagccggccccatcagccagttcaactataaacagagcttcagcaatcctacatgcctgatt
ctggctactgtgccacataatctgactaccatcactaagcccctgaaatactcctatattaacaagtgcagccggttcct
gtccgacgatagaaccgaagtgccacagctggtcaacgccaatcagtactctccctgtgtgagtatcgtcccttcaaccg
tgtgggaagacggggattactatagaaaacagctgagcccccctggagggaggaggatggctggtggcatccggatctaca
gtcgccatgactgagcagctgcagatggggttcggaatcacagtgcagtacggcacagacactaactctgtctgtcccaa
gctggaattcgctaacgatactaagatcgcaagtcagctgggaaactgcgtggagtactctctgtatggcgtgagtggca
gaggggtcttccagaattgtaccgcagtgggcgtccgacagcagcggtttgtgtacgacgcctatcagaatctggtcggc
tactatagcgacgatgggaactactattgcctgagggcctgtgtgagcgtccctgtgtccgtcatctacgataaggaaac
caaaacacacgccacactgttcgggtccgtggcttgcgagcatattagctccacaatgtctcagtacagtagatcaacta
ggtcaatgctgaagaggcgcgatagcacctatggacctctgcagacaccagtggggtgtgtcctgggactggtgaactct
agtctgtttgtcgaggactgcaagctgcccctgggccagagcctgtgcgccctgcccgacacccccagcaccctgacccc
ccggagcgtgcggagcgtgcccggcgagatgcggctggccagcatcgccttcaaccaccccatccaggtggaccagctga
acagcagctacttcaagctgagcatccccaccaacttcagcttcggcgtgacccaggagtacatccagaccaccatccag
aaggtgaccgtggactgcaagcagtacgtgtgcaacggcttccagaagtgcgagcagctgctgcgggagtacggccagtt
ctgcagcaagatcaaccaggccctgcacggcgccaacctgcggcaggacgacagcgtgcggaacctgttcgccagcgtga
agagcagccagagcagccccatcatccccggcttcggcggcgacttcaacctgaccctgctggagcccgtgagcatcagc
accggcagccggagcgcccggagcgccatcgaggacctgctgttcgacaaggtgaccatcgccgaccccggctacatgca
gggctacgacgactgcatgcagcagggccccgccagcgcccgggacctgatctgcgcccagtacgtggccggctacaagg
tgctgccccccctgatggacgtgaacatggaggccgcctacaccagcagcctgctgggcagcatcgccggcgtgggctgg
accgccggcctgagcagcttcgccgccatccccttcgcccagagcatcttctaccggctgaacggcgtgggcatcaccca
 
gcaggtgctgagcgagaaccagaagctgatcgccaacaagttcaaccaggccctgggcgccatgcagaccggcttcacca
ccaccaacgaggccttccacaaggtgcaggacgccgtgaacaacaacgcccaggccctgagcaagctggccagcgagctg
agcaacaccttcggcgccatcagcgccagcatcggcgacatcatccagcggctggacgtgctggagcaggacgcccagat
cgaccggctgatcaacggccggctgaccaccctgaacgccttcgtggcccagcagctggtgcggagcgagagcgccgccc
tgagcgcccagctggccaaggacaaggtgaacgagtgcgtgaaggcccagagcaagcggagcggcttctgcggccagggc
acccacatcgtgagcttcgtggtgaacgcccccaacggcctgtacttcatgcacgtgggctactaccccagcaaccacat
cgaggtggtgagcgcctacggcctgtgcgacgccgccaaccccaccaactgcatcgcccccgtgaacggctacttcatca
agaccaacaacacccggatcgtggacgagtggagctacaccggcagcagcttctacgcccccgagcccatcaccagcctg
aacaccaagtacgtggcccccaggtgacctaccagaacatcagcaccaacctgccccccccctgctgggcaacagcac
cggcatcgacttccaggacgagctggacgagttcttcaagaacgtgagcaccagcatccccaacttcggcagcctgaccc
agatcaacaccaccctgctggacctgacctacgagatgctgagcctgcagcaggtggtgaaggccctgaacgagagctac
atcgacctgaaggagctgggcaactacacctactacaacaagtggccctggtacatctggctgggcttcatcgccggcct
ggtggccctggccctgtgcgtgttcttcatcctgtgctgcaccggctgcggcaccaactgcatgggcaagctgaagtgca
accggtgctgcgaccggtacgaggagtacgacctggagccccacaaggtgcacgtgcactga
 The DNA sequence of the MERS-CoV S protomer provided above can be modified to introduce the amino acid substitutions and deletions disclosed herein for prefusion stabilization, such as the "2P" substitutions.

In several embodiments, the nucleic acid molecule encodes a precursor of the protomer, that, when expressed in an appropriate cell, is processed into a disclosed coronavirus S ectodomain protomer that can self-assemble into the corresponding recombinant coronavirus S ectodomain trimer. For example, the nucleic acid molecule can encode a recombinant coronavirus S ectodomain including a N-terminal signal sequence for entry into the cellular secretory system that is proteolytically cleaved in the during processing of the recombinant coronavirus S ectodomain in the cell.

In several embodiments, the nucleic acid molecule encodes a precursor S polypeptide that, when expressed in an appropriate cell, is processed into a disclosed recombinant coronavirus S ectodomain protomer including S1 and S2 polypeptides, wherein the recombinant S ectodomain protomer includes any of the appropriate stabilizing modifications described herein, and optionally can be linked to a trimerization domain, such as a T4 Fibritin trimerization domain..

Exemplary nucleic acids can be prepared by cloning techniques. Examples of appropriate cloning and sequencing techniques, and instructions sufficient to direct persons of skill through many cloning exercises are known (see, *e.g.,* Sambrook et al. (Molecular Cloning: A Laboratory Manual, 4th ed, Cold Spring Harbor, New York, 2012) and Ausubel et al. (In Current Protocols in Molecular Biology, John Wiley & Sons, New York, through supplement 104, 2013).

Nucleic acids can also be prepared by amplification methods. Amplification methods include polymerase chain reaction (PCR), the ligase chain reaction (LCR), the transcription-based amplification system (TAS), the self-sustained sequence replication system (3SR). A wide variety of cloning methods, host cells, and *in vitro* amplification methodologies are well known to persons of skill.

The polynucleotides encoding a disclosed recombinant coronavirus S ectodomain protomer can include a recombinant DNA which is incorporated into a vector (such as an expression vector) into an autonomously replicating plasmid or virus or into the genomic DNA of a prokaryote or eukaryote, or which exists as a separate molecule (such as a cDNA) independent of other sequences. The nucleotides can be ribonucleotides, deoxyribonucleotides, or modified forms of either nucleotide. The term includes single and double forms of DNA.

Polynucleotide sequences encoding a disclosed recombinant coronavirus S ectodomain protomer can be operatively linked to expression control sequences. An expression control sequence operatively linked to a coding sequence is ligated such that expression of the coding sequence is achieved under conditions compatible with the expression control sequences. The expression control sequences include, but are not limited to, appropriate promoters, enhancers, transcription terminators, a start codon (*i.e.,* ATG) in front of a protein-encoding gene, splicing signal for introns, maintenance of the correct reading frame of that gene to permit proper translation of mRNA, and stop codons.

DNA sequences encoding the disclosed recombinant S ectodomain protomer can be expressed *in vitro* by DNA transfer into a suitable host cell. The cell may be prokaryotic or eukaryotic. The term also includes any progeny of the subject host cell. It is understood that all progeny may not be identical to the parental cell since there may be mutations that occur during replication. Methods of stable transfer, meaning that the foreign DNA is continuously maintained in the host, are known in the art.

Hosts can include microbial, yeast, insect and mammalian organisms. Methods of expressing DNA sequences having eukaryotic or viral sequences in prokaryotes are well known in the art. Non-limiting examples of suitable host cells include bacteria, archea, insect, fungi (for example, yeast), plant, and animal cells (for example, mammalian cells, such as human). Exemplary cells of use include *Escherichia coli, Bacillus subtilis, Saccharomyces cerevisiae, Salmonella typhimurium,* SF9 cells, C129 cells, 293 cells, *Neurospora,* and immortalized mammalian myeloid and lymphoid cell lines. Techniques for the propagation of mammalian cells in culture are well-known (see, *e.g.,* Helgason and Miller (Eds.), 2012, Basic Cell Culture Protocols (Methods in Molecular Biology), 4th Ed., Humana Press). Examples of commonly used mammalian host cell lines are VERO and HeLa cells, CHO cells, and WI38, BHK, and COS cell lines, although cell lines may be used, such as cells designed to provide higher expression, desirable glycosylation patterns, or other features. In some embodiments, the host cells include HEK293 cells or derivatives thereof, such as GnTI^{-/-} cells (ATCC^{®} No. CRL-3022), or HEK-293F cells.

Transformation of a host cell with recombinant DNA can be carried out by conventional techniques. Where the host is prokaryotic, such as, but not limited to, *E. coli,* competent cells which are capable of DNA uptake can be prepared from cells harvested after exponential growth phase and subsequently treated by the CaCl₂ method using standard procedures. Alternatively, MgCl₂ or RbCl can be used. Transformation can also be performed after forming a protoplast of the host cell if desired, or by electroporation.

When the host is a eukaryote, such methods of transfection of DNA as calcium phosphate coprecipitates, conventional mechanical procedures such as microinjection, electroporation, insertion of a plasmid encased in liposomes, or viral vectors can be used. Eukaryotic cells can also be co-transformed with polynucleotide sequences encoding a disclosed antigen, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein (see for example, Viral Expression Vectors, Springer press, Muzyczka ed., 2011). Appropriate expression systems such as plasmids and vectors of use in producing proteins in cells including higher eukaryotic cells such as the COS, CHO, HeLa and mycloma cell lines.

In one non-limiting example, a disclosed immunogen is expressed using the pVRC8400 vector (described in Barouch et al., J. Virol., 79 ,8828-8834, 2005, which is incorporated by reference herein).

Modifications can be made to a nucleic acid encoding a disclosed recombinant coronavirus S ectodomain protomer without diminishing its biological activity. Some modifications can be made to facilitate the cloning, expression, or incorporation of the targeting molecule into a fusion protein. Such modifications are well known to those of skill in the art and include, for example, termination codons, a methionine added at the amino terminus to provide an initiation, site, additional amino acids placed on either terminus to create conveniently located restriction sites, or additional amino acids (such as poly His) to aid in purification steps.

In some embodiments, the disclosed recombinant coronavirus S ectodomain protomer can be expressed in cells under conditions where the recombinant coronavirus S ectodomain protomer can self-assemble into trimers which are secreted from the cells into the cell media. In such embodiments, each recombinant coronavirus S ectodomain protomer contains a leader sequence (signal peptide) that causes the protein to enter the secretory system, where the signal peptide is cleaved and the protomers form a trimer, before being secreted in the cell media. The medium can be centrifuged and recombinant coronavirus S ectodomain trimer purified from the supernatant.

### IV. Viral Vectors

A nucleic acid molecule encoding a protomer of a disclosed recombinant coronavirus S ectodomain trimer can be included in a viral vector, for example, for expression of the immunogen in a host cell, or for immunization of a subject as disclosed herein. In some embodiments, the viral vectors are administered to a subject as part of a prime-boost vaccination. In several embodiments, the viral vectors are included in a vaccine, such as a primer vaccine or a booster vaccine for use in a prime-boost vaccination.

In several examples, the viral vector can be replication-competent. For example, the viral vector can have a mutation in the viral genome that does not inhibit viral replication in host cells. The viral vector also can be conditionally replication-competent. In other examples, the viral vector is replication-deficient in host cells.

A number of viral vectors have been constructed, that can be used to express the disclosed antigens, including polyoma, *i.e.,* SV40 (Madzak et al., 1992, J. Gen. Virol., 73:15331536), adenovirus (Berkner, 1992, Cur. Top. Microbiol. Immunol., 158:39-6; Berliner et al., 1988, Bio Techniques, 6:616-629; Gorziglia et al., 1992, J. Virol., 66:4407-4412; Quantin et al., 1992, Proc. Natl. Acad. Sci. USA, 89:2581-2584; Rosenfeld et al., 1992, Cell, 68:143-155; Wilkinson et al., 1992, Nucl. Acids Res., 20:2233-2239; Stratford-Perricaudet et al., 1990, Hum. Gene Ther., 1:241-256), vaccinia virus (Mackett et al., 1992, Biotechnology, 24:495-499), adeno-associated virus (Muzyczka, 1992, Curr. Top. Microbiol. Immunol., 158:91-123; On et al., 1990, Gene, 89:279-282), herpes viruses including HSV and EBV (Margolskee, 1992, Curr. Top. Microbiol. Immunol., 158:67-90; Johnson et al., 1992, J. Virol., 66:29522965; Fink et al., 1992, Hum. Gene Ther. 3:11-19; Breakfield et al., 1987, Mol. Neurobiol., 1:337-371; Fresse et al., 1990, Biochem. Pharmacol., 40:2189-2199), Sindbis viruses (H. Herweijer et al., 1995, Human Gene Therapy 6:1161-1167; U.S. Pat. Nos. 5,091,309 and 5,2217,879), alphaviruses (S. Schlesinger, 1993, Trends Biotechnol. 11: 18-22; I. Frolov et al., 1996, Proc. Natl. Acad. Sci. USA 93:11371-11377) and retroviruses of avian (Brandyopadhyay et al., 1984, Mol. Cell Biol., 4:749-754; Petropouplos et al., 1992, J. Virol., 66:3391-3397), murine (Miller, 1992, Curr. Top. Microbiol. Immunol., 158:1-24; Miller et al., 1985, Mol. Cell Biol., 5:431-437; Sorge et al., 1984, Mol. Cell Biol., 4:1730-1737; Mann et al., 1985, J. Virol., 54:401-407), and human origin (Page et al., 1990, J. Virol., 64:5370-5276; Buchschalcher et al., 1992, J. Virol., 66:2731-2739). Baculovirus (Autographa californica multinuclear polyhedrosis virus; AcMNPV) vectors are also known in the art, and may be obtained from commercial sources (such as PharMingen, San Diego, Calif.; Protein Sciences Corp., Meriden, Conn.; Stratagene, La Jolla, Calif.).

In several embodiments, the viral vector can include an adenoviral vector that expresses a protomer of a disclosed recombinant coronavirus S ectodomain trimer. Adenovirus from various origins, subtypes, or mixture of subtypes can be used as the source of the viral genome for the adenoviral vector. Non-human adenovirus (*e.g*., simian, chimpanzee, gorilla, avian, canine, ovine, or bovine adenoviruses) can be used to generate the adenoviral vector. For example, a simian adenovirus can be used as the source of the viral genome of the adenoviral vector. A simian adenovirus can be of serotype 1, 3, 7, 11, 16, 18, 19, 20, 27, 33, 38, 39, 48, 49, 50, or any other simian adenoviral serotype. A simian adenovirus can be referred to by using any suitable abbreviation known in the art, such as, for example, SV, SAdV, SAV or sAV. In some examples, a simian adenoviral vector is a simian adenoviral vector of serotype 3, 7, 11, 16, 18, 19, 20, 27, 33, 38, or 39. In one example, a chimpanzee serotype C Ad3 vector is used (see, *e.g.,* Peruzzi et al., Vaccine, 27:1293-1300, 2009). Human adenovirus can be used as the source of the viral genome for the adenoviral vector. Human adenovirus can be of various subgroups or serotypes. For instance, an adenovirus can be of subgroup A (*e.g*., serotypes 12, 18, and 31), subgroup B (*e.g*., serotypes 3, 7, 11, 14, 16, 21, 34, 35, and 50), subgroup C (*e.g.,* serotypes 1, 2, 5, and 6), subgroup D (*e.g.,* serotypes 8, 9, 10, 13, 15, 17, 19, 20, 22, 23, 24, 25, 26, 27, 28, 29, 30, 32, 33, 36-39, and 42-48), subgroup E (*e.g.,* serotype 4), subgroup F (*e.g.,* serotypes 40 and 41), an unclassified serogroup (*e.g*., serotypes 49 and 51), or any other adenoviral serotype. The person of ordinary skill in the art is familiar with replication competent and deficient adenoviral vectors (including singly and multiply replication deficient adenoviral vectors). Examples of replication-deficient adenoviral vectors, including multiply replication-deficient adenoviral vectors, are disclosed in U.S. Patent Nos. 5,837,51 1 ; 5,851 ,806; 5,994,106; 6,127,175; 6,482,616; and 7,195,896, and International Patent Application Nos. WO 94/28152, WO 95/02697, WO 95/16772, WO 95/34671, WO 96/22378, WO 97/12986, WO 97/21826, and WO 03/02231 1.

### V. Virus-Like Particles

In some embodiments, a virus-like particle (VLP) is provided that includes a disclosed recombinant coronavirus S ectodomain trimer. Typically such VLPs include a recombinant coronavirus S ectodomain trimer that is membrane anchored by a C-terminal transmembrane domain, for example the recombinant coronavirus S ectodomain protomers in the trimer each can be linked to a transmembrane domain and cytosolic tail from the corresponding coronavirus. VLPs lack the viral components that are required for virus replication and thus represent a highly attenuated, replication-incompetent form of a virus. However, the VLP can display a polypeptide (*e.g.,* a recombinant coronavirus S ectodomain trimer) that is analogous to that expressed on infectious virus particles and can eliciting an immune response to the corresponding coronavirus when administered to a subject. Virus like particles and methods of their production are known and familiar to the person of ordinary skill in the art, and viral proteins from several viruses are known to form VLPs, including human papillomavirus, HIV (Kang et al., Biol. Chem. 380: 353-64 (1999)), Semliki-Forest virus (Notka et al., Biol. Chem. 380: 341-52 (1999)), human polyomavirus (Goldmann et al., J. Virol. 73: 4465-9 (1999)), rotavirus (Jiang et al., Vaccine 17: 1005-13 (1999)), parvovirus (Casal, Biotechnology and Applied Biochemistry, Vol 29, Part 2, pp 141-150 (1999)), canine parvovirus (Hurtado et al., J. Virol. 70: 5422-9 (1996)), hepatitis E virus (Li et al., J. Virol. 71: 7207-13 (1997)), and Newcastle disease virus. The formation of such VLPs can be detected by any suitable technique. Examples of suitable techniques known in the art for detection of VLPs in a medium include, *e.g.,* electron microscopy techniques, dynamic light scattering (DLS), selective chromatographic separation (e.g., ion exchange, hydrophobic interaction, and/or size exclusion chromatographic separation of the VLPs) and density gradient centrifugation.

### VI. Immunogenic Compositions

Immunogenic compositions comprising a disclosed immunogen (*e.g*., a disclosed recombinant coronavirus S ectodomain trimer or nucleic acid molecule encoding a protomer of disclosed recombinant coronavirus S ectodomain trimer) and a pharmaceutically acceptable carrier are also provided. Such pharmaceutical compositions can be administered to subjects by a variety of administration modes known to the person of ordinary skill in the art, for example, intramuscular, intradermal, subcutaneous, intravenous, intra-arterial, intra-articular, intraperitoneal, intranasal, sublingual, tonsillar, oropharyngeal, or other parenteral and mucosal routes. In several embodiments, pharmaceutical compositions including one or more of the disclosed immunogens are immunogenic compositions. Actual methods for preparing administrable compositions will be known or apparent to those skilled in the art and are described in more detail in such publications as Remingtons Pharmaceutical Sciences, 19th Ed., Mack Publishing Company, Easton, Pennsylvania, 1995.

Thus, an immunogen described herein can be formulated with pharmaceutically acceptable carriers to help retain biological activity while also promoting increased stability during storage within an acceptable temperature range. Potential carriers include, but are not limited to, physiologically balanced culture medium, phosphate buffer saline solution, water, emulsions (*e.g*., oil/water or water/oil emulsions), various types of wetting agents, cryoprotective additives or stabilizers such as proteins, peptides or hydrolysates (*e.g.,* albumin, gelatin), sugars (*e.g.,* sucrose, lactose, sorbitol), amino acids (*e.g.,* sodium glutamate), or other protective agents. The resulting aqueous solutions may be packaged for use as is or lyophilized. Lyophilized preparations are combined with a sterile solution prior to administration for either single or multiple dosing.

Formulated compositions, especially liquid formulations, may contain a bacteriostat to prevent or minimize degradation during storage, including but not limited to effective concentrations (usually ≦ 1 % w/v) of benzyl alcohol, phenol, m-cresol, chlorobutanol, methylparaben, and/or propylparaben. A bacteriostat may be contraindicated for some patients; therefore, a lyophilized formulation may be reconstituted in a solution either containing or not containing such a component.

The immunogenic compositions of the disclosure can contain as pharmaceutically acceptable vehicles substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate.

The immunogenic composition may optionally include an adjuvant to enhance an immune response of the host. Suitable adjuvants are, for example, toll-like receptor agonists, alum, AlPO4, alhydrogel, Lipid-A and derivatives or variants thereof, oil-emulsions, saponins, neutral liposomes, liposomes containing the vaccine and cytokines, non-ionic block copolymers, and chemokines. Non-ionic block polymers containing polyoxyethylene (POE) and polyxylpropylene (POP), such as POE-POP-POE block copolymers, MPL^{™} (3-O-deacylated monophosphoryl lipid A; Corixa, Hamilton, IN) and IL-12 (Genetics Institute, Cambridge, MA), among many other suitable adjuvants well known in the art, may be used as an adjuvant (Newman et al., 1998, Critical Reviews in Therapeutic Drug Carrier Systems 15:89-142). These adjuvants have the advantage in that they help to stimulate the immune system in a non-specific way, thus enhancing the immune response to a pharmaceutical product.

In some instances it may be desirable to combine a disclosed immunogen, with other pharmaceutical products (*e.g*., vaccines) which induce protective responses to other agents. For example, a composition including a recombinant paramyxovirus as described herein can be can be administered simultaneously (typically separately) or sequentially with other vaccines recommended by the Advisory Committee on Immunization Practices (ACIP; cdc.gov/vaccines/acip/index.html) for the targeted age group (*e.g*., infants from approximately one to six months of age), such as an influenza vaccine or a varicella zoster vaccine. As such, a disclosed immunogen including a recombinant coronavirus S ectodomain trimer described herein may be administered simultaneously or sequentially with vaccines against, for example, hepatitis B (HepB), diphtheria, tetanus and pertussis (DTaP), pneumococcal bacteria (PCV), *Haemophilus influenzae* type b (Hib), polio, influenza and rotavirus.

In some embodiments, the composition can be provided as a sterile composition. The pharmaceutical composition typically contains an effective amount of a disclosed immunogen and can be prepared by conventional techniques. Typically, the amount of immunogen in each dose of the immunogenic composition is selected as an amount which induces an immune response without significant, adverse side effects. In some embodiments, the composition can be provided in unit dosage form for use to induce an immune response in a subject. A unit dosage form contains a suitable single preselected dosage for administration to a subject, or suitable marked or measured multiples of two or more preselected unit dosages, and/or a metering mechanism for administering the unit dose or multiples thereof. In other embodiments, the composition further includes an adjuvant.

### VII. Methods of Inducing an Immune Response

The disclosed immunogens (*e.g*., recombinant coronavirus S ectodomain trimer, a nucleic acid molecule (such as an RNA molecule) or vector encoding a protomer of a disclosed recombinant coronavirus S ectodomain trimer, or a protein nanoparticle or virus like particle comprising a disclosed recombinant coronavirus S ectodomain trimer) can be administered to a subject to induce an immune response to the corresponding coronavirus S ectodomain in the subject. In a particular example, the subject is a human. The immune response can be a protective immune response, for example a response that inhibits subsequent infection with the corresponding coronavirus. Elicitation of the immune response can also be used to treat or inhibit infection and illnesses associated with the corresponding coronavirus.

A subject can be selected for treatment that has, or is at risk for developing infection with the coronavirus corresponding to the S protein in the immunogen, for example because of exposure or the possibility of exposure to the coronavirus. Following administration of a disclosed immunogen, the subject can be monitored for infection or symptoms associated with the coronavirus, or both.

Typical subjects intended for treatment with the therapeutics and methods of the present disclosure include humans, as well as non-human primates and other animals. To identify subjects for prophylaxis or treatment according to the methods of the disclosure, accepted screening methods are employed to determine risk factors associated with a targeted or suspected disease or condition, or to determine the status of an existing disease or condition in a subject. These screening methods include, for example, conventional work-ups to determine environmental, familial, occupational, and other such risk factors that may be associated with the targeted or suspected disease or condition, as well as diagnostic methods, such as various ELISA and other immunoassay methods to detect and/or characterize coronavirus infection. These and other routine methods allow the clinician to select patients in need of therapy using the methods and pharmaceutical compositions of the disclosure. In accordance with these methods and principles, a composition can be administered according to the teachings herein, or other conventional methods, as an independent prophylaxis or treatment program, or as a follow-up, adjunct or coordinate treatment regimen to other treatments.

The administration of a disclosed immunogen can be for prophylactic or therapeutic purpose. When provided prophylactically, the disclosed therapeutic agents are provided in advance of any symptom, for example, in advance of infection. The prophylactic administration of the disclosed therapeutic agents serves to prevent or ameliorate any subsequent infection. When provided therapeutically, the disclosed therapeutic agents are provided at or after the onset of a symptom of disease or infection, for example, after development of a symptom of infection with the coronavirus corresponding to the S protein in the immunogen, or after diagnosis with the coronavirus infection. The therapeutic agents can thus be provided prior to the anticipated exposure to the coronavirus so as to attenuate the anticipated severity, duration or extent of an infection and/or associated disease symptoms, after exposure or suspected exposure to the virus, or after the actual initiation of an infection.

The immunogens described herein, and immunogenic compositions thereof, are provided to a subject in an amount effective to induce or enhance an immune response against the coronavirus S protein in the immunogen in the subject, preferably a human. The actual dosage of disclosed immunogen will vary according to factors such as the disease indication and particular status of the subject (for example, the subject's age, size, fitness, extent of symptoms, susceptibility factors, and the like), time and route of administration, other drugs or treatments being administered concurrently, as well as the specific pharmacology of the composition for eliciting the desired activity or biological response in the subject. Dosage regimens can be adjusted to provide an optimum prophylactic or therapeutic response.

An immunogenic composition including one or more of the disclosed immunogens can be used in coordinate (or prime-boost) vaccination protocols or combinatorial formulations. In certain embodiments, novel combinatorial immunogenic compositions and coordinate immunization protocols employ separate immunogens or formulations, each directed toward eliciting an anti-viral immune response, such as an immune response to coronavirus S proteins. Separate immunogenic compositions that elicit the anti-viral immune response can be combined in a polyvalent immunogenic composition administered to a subject in a single immunization step, or they can be administered separately (in monovalent immunogenic compositions) in a coordinate (or prime-boost) immunization protocol.

There can be several boosts, and each boost can be a different disclosed immunogen. In some examples that the boost may be the same immunogen as another boost, or the prime. The prime and boost can be administered as a single dose or multiple doses, for example two doses, three doses, four doses, five doses, six doses or more can be administered to a subject over days, weeks or months. Multiple boosts can also be given, such one to five (*e.g.,* 1, 2, 3, 4 or 5 boosts), or more. Different dosages can be used in a series of sequential immunizations. For example a relatively large dose in a primary immunization and then a boost with relatively smaller doses.

In some embodiments, the boost can be administered about two, about three to eight, or about four, weeks following the prime, or about several months after the prime. In some embodiments, the boost can be administered about 5, about 6, about 7, about 8, about 10, about 12, about 18, about 24, months after the prime, or more or less time after the prime. Periodic additional boosts can also be used at appropriate time points to enhance the subject's "immune memory." The adequacy of the vaccination parameters chosen, e.g., formulation, dose, regimen and the like, can be determined by taking aliquots of serum from the subject and assaying antibody titers during the course of the immunization program. In addition, the clinical condition of the subject can be monitored for the desired effect, *e.g.,* prevention of infection or improvement in disease state (*e.g.,* reduction in viral load). If such monitoring indicates that vaccination is sub-optimal, the subject can be boosted with an additional dose of immunogenic composition, and the vaccination parameters can be modified in a fashion expected to potentiate the immune response.

In some embodiments, the prime-boost method can include DNA-primer and protein-boost vaccination protocol to a subject. The method can include two or more administrations of the nucleic acid molecule or the protein.

For protein therapeutics, typically, each human dose will comprise 1-1000 µg of protein, such as from about 1 µg to about 100 µg, for example, from about 1 µg to about 50 µg, such as about 1 µg, about 2 µg, about 5 µg, about 10 µg, about 15 µg, about 20 µg, about 25 µg, about 30 µg, about 40 µg, or about 50 µg.

The amount utilized in an immunogenic composition is selected based on the subject population (*e.g.,* infant or elderly). An optimal amount for a particular composition can be ascertained by standard studies involving observation of antibody titers and other responses in subjects. It is understood that a therapeutically effective amount of a disclosed immunogen, such as a disclosed recombinant coronavirus S ectodomain trimer, viral vector, or nucleic acid molecule in a immunogenic composition, can include an amount that is ineffective at eliciting an immune response by administration of a single dose, but that is effective upon administration of multiple dosages, for example in a prime-boost administration protocol.

Upon administration of a disclosed immunogen of this disclosure, the immune system of the subject typically responds to the immunogenic composition by producing antibodies specific for the coronavirus S ectodomain trimer included in the immunogen. Such a response signifies that an immunologically effective dose was delivered to the subject.

In some embodiments, the antibody response of a subject will be determined in the context of evaluating effective dosages/immunization protocols. In most instances it will be sufficient to assess the antibody titer in serum or plasma obtained from the subject. Decisions as to whether to administer booster inoculations and/or to change the amount of the therapeutic agent administered to the individual can be at least partially based on the antibody titer level. The antibody titer level can be based on, for example, an immunobinding assay which measures the concentration of antibodies in the serum which bind to an antigen including, for example, the recombinant coronavirus S ectodomain trimer included in the immunogen.

Coronavirus infection does not need to be completely eliminated or reduced or prevented for the methods to be effective. For example, elicitation of an immune response to a coronavirus with one or more of the disclosed immunogens can reduce or inhibit infection with the coronavirus by a desired amount, for example, by at least 10%, at least 20%, at least 50%, at least 60%, at least 70%, at least 80%, at least 90%, at least 95%, at least 98%, or even at least 100% (elimination or prevention of detectable infected cells), as compared to infection with the coronavirus in the absence of the immunogen. In additional examples, coronavirus replication can be reduced or inhibited by the disclosed methods. Coronavirus replication does not need to be completely eliminated for the method to be effective. For example, the immune response elicited using one or more of the disclosed immunogens can reduce replication of the corresponding coronavirus by a desired amount, for example, by at least 10%, at least 20%, at least 50%, at least 60%, at least 70%, at least 80%, at least 90%, at least 95%, at least 98%, or even at least 100% (elimination or prevention of detectable replication of the coronavirus), as compared to replication of the coronavirus in the absence of the immune response.

In some embodiments, the disclosed immunogen is administered to the subject simultaneously with the administration of the adjuvant. In other embodiments, the disclosed immunogen is administered to the subject after the administration of the adjuvant and within a sufficient amount of time to induce the immune response.

One approach to administration of nucleic acids is direct immunization with plasmid DNA, such as with a mammalian expression plasmid. Immunization by nucleic acid constructs is well known in the art and taught, for example, in U.S. Patent No. 5,643,578 (which describes methods of immunizing vertebrates by introducing DNA encoding a desired antigen to elicit a cell-mediated or a humoral response), and U.S. Patent No. 5,593,972 and U.S. Patent No. 5,817,637 (which describe operably linking a nucleic acid sequence encoding an antigen to regulatory sequences enabling expression). U.S. Patent No. 5,880,103 describes several methods of delivery of nucleic acids encoding immunogenic peptides or other antigens to an organism. The methods include liposomal delivery of the nucleic acids (or of the synthetic peptides themselves), and immune-stimulating constructs, or ISCOMS^{™}, negatively charged cage-like structures of 30-40 nm in size formed spontaneously on mixing cholesterol and Quil A^{™} (saponin). Protective immunity has been generated in a variety of experimental models of infection, including toxoplasmosis and Epstein-Barr virus-induced tumors, using ISCOMS^{™} as the delivery vehicle for antigens (Mowat and Donachie, *Immunol. Today* 12:383, 1991). Doses of antigen as low as 1 µg encapsulated in ISCOMS^{™} have been found to produce Class I mediated CTL responses (Takahashi et al., Nature 344:873, 1990).

In some embodiments, a plasmid DNA vaccine is used to express a disclosed immunogen in a subject. For example, a nucleic acid molecule encoding a disclosed immunogen can be administered to a subject to induce an immune response to the coronavirus S protein included in the immunogen. In some embodiments, the nucleic acid molecule can be included on a plasmid vector for DNA immunization, such as the pVRC8400 vector (described in Barouch et al., J. Virol, 79, 8828-8834, 2005, which is incorporated by reference herein).

In another approach to using nucleic acids for immunization, a disclosed recombinant coronavirus S ectodomain or recombinant coronavirus S ectodomain trimer can be expressed by attenuated viral hosts or vectors or bacterial vectors. Recombinant vaccinia virus, adeno-associated virus (AAV), herpes virus, retrovirus, cytogmeglo virus or other viral vectors can be used to express the peptide or protein, thereby eliciting a CTL response. For example, vaccinia vectors and methods useful in immunization protocols are described in U.S. Patent No. 4,722,848. BCG (Bacillus Calmette Guerin) provides another vector for expression of the peptides (see Stover, Nature 351:456-460, 1991).

In one embodiment, a nucleic acid encoding a disclosed recombinant coronavirus S ectodomain or coronavirus S ectodomain trimer is introduced directly into cells. For example, the nucleic acid can be loaded onto gold microspheres by standard methods and introduced into the skin by a device such as Bio-Rad's HELIOS^{™} Gene Gun. The nucleic acids can be "naked," consisting of plasmids under control of a strong promoter. Typically, the DNA is injected into muscle, although it can also be injected directly into other sites. Dosages for injection are usually around 0.5 µg/kg to about 50 mg/kg, and typically are about 0.005 mg/kg to about 5 mg/kg (see, *e.g.,* U.S. Patent No. 5,589,466).

For example, the nucleic acid can be loaded onto gold microspheres by standard methods and introduced into the skin by a device such as Bio-Rad's HELTOS^{™} Gene Gun. The nucleic acids can be "naked," consisting of plasmids under control of a strong promoter. Typically, the DNA is injected into muscle, although it can also be injected directly into other sites. Dosages for injection are usually around 0.5 µg/kg to about 50 mg/kg, and typically are about 0.005 mg/kg to about 5 mg/kg (see, *e.g.,* U.S. Patent No. 5,589,466).

In another embodiment, an mRNA-based immunization protocol can be used to deliver a nucleic acid encoding a disclosed recombinant coronavirus S ectodomain or coronavirus S ectodomain trimer directly into cells. In some embodiments, nucleic acid-based vaccines based on mRNA may provide a potent alternative to the previously mentioned approaches. mRNA vaccines preclude safety concerns about DNA integration into the host genome and can be directly translated in the host cell cytoplasm. Moreover, the simple cell-free, *in vitro* synthesis of RNA avoids the manufacturing complications associated with viral vectors. Two exemplary forms of RNA-based vaccination that can be used to deliver a nucleic acid encoding a disclosed recombinant coronavirus S ectodomain or coronavirus S ectodomain trimer include conventional non-amplifying mRNA immunization (see, e.g., Petsch et al., "Protective efficacy of in vitro synthesized, specific mRNA vaccines against influenza A virus infection," Nature biotechnology, 30(12):1210-6, 2012) and self-amplifying mRNA immunization (see, e.g., Geall et al., "Nonviral delivery of self-amplifying RNA vaccines," PNAS, 109(36): 14604-14609, 2012; Magini et al., "Self-Amplifying mRNA Vaccines Expressing Multiple Conserved Influenza Antigens Confer Protection against Homologous and Heterosubtypic Viral Challenge," PLoS One, 11(8):e0161193, 2016; and Brito et al., "Self-amplifying mRNA vaccines," Adv Genet., 89:179-233, 2015).

In some embodiments, administration of a therapeutically effective amount of one or more of the disclosed immunogens to a subject induces a neutralizing immune response in the subject. To assess neutralization activity, following immunization of a subject, serum can be collected from the subject at appropriate time points, frozen, and stored for neutralization testing. Methods to assay for neutralization activity are known to the person of ordinary skill in the art and are further described herein, and include, but are not limited to, plaque reduction neutralization (PRNT) assays, microneutralization assays, flow cytometry based assays, single-cycle infection assays. In some embodiments, the serum neutralization activity can be assayed using a panel of coronavirus pseudoviruses. For example, to test the immunogenicity of the vaccine candidates against multiple MERS-CoV strains-without the requirement of a biosafety level 3 facility-a pseudotyped reporter virus neutralization assay was previously developed (Wand et al., Nat Commun, 6:7712, 2015), similar to that previously developed for SARS-CoV (Martin et al., Vaccine 26, 6338, 2008; Yang et al., Nature 428, 561, 2004; Naldini et al., PNAS 93, 11382, 1996; Yang et al., PNAS 102, 797, 2005).

In some embodiments, administration of a therapeutically effective amount of one or more of the disclosed immunogens to a subject induces a neutralizing immune response in the subject. To assess neutralization activity, following immunization of a subject, serum can be collected from the subject at appropriate time points, frozen, and stored for neutralization testing. Methods to assay for neutralization activity are known to the person of ordinary skill in the art and are further described herein, and include, but are not limited to, plaque reduction neutralization (PRNT) assays, microneutralization assays, flow cytometry based assays, single-cycle infection assays. In some embodiments, the serum neutralization activity can be assayed using a panel of coronavirus pseudoviruses.

### EXAMPLES

The following examples are provided to illustrate particular features of certain embodiments, but the scope of the claims should not be limited to those features exemplified.

### Example 1

### Prefusion stabilized MERS-CoV S protein

This example describes development of a recombinant MERS-CoV S ectodomain trimer that is stabilized in a prefusion conformation.

The structure of the prefusion S ectodomain trimer of a human betacoronavirus was recently elucidated (Kirchdoerfer et al., "Prefusion structure of a human coronavirus spike protein," Nature, 351:118-121, 2016). This structure was further investigated to reveal several key details about human coronavirus spike architecture. First, receptor-binding elements within S1 cap the fusion-mediating elements in S2, likely preventing their conformational rearrangement (FIG. 1) until triggering occurs.

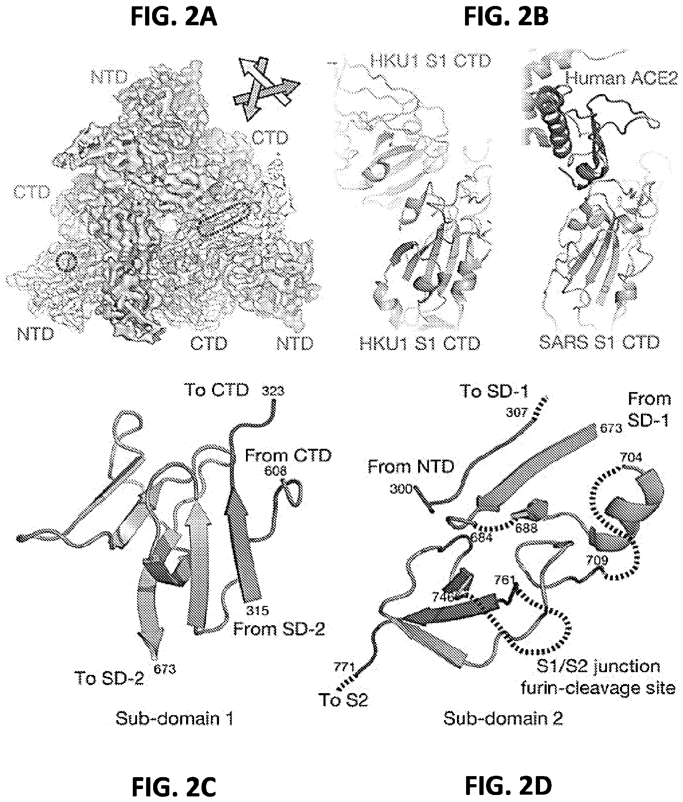

The S1 C-terminal domains appear interdigitated and form extensive quaternary interactions (FIG. 2A), suggesting conformational flexibility or "breathing" would be required for the HKU1-CoV Spike to make receptor interactions similar to those made between the SARS-CoV receptor binding domain (RBD) and ACE2 receptors (FIG. 2A). In addition, the structure revealed two sub-domains, SD-1 and SD-2, in HKU1-CoV S1 (FIGs. 2C, 2D). The SD-2 loop contains the site dedicated to HKU1-CoV S furin cleavage; and furin cleavage at the S1/S2 junction is a process necessary for infection (FIG. 2D). S2 contains four classical components of a Class 1 fusion machine: a fusion peptide (FIG. 3A), two heptad repeats, HR1 (FIG. 3B) and HR2, and a transmembrane domain.

*Structure-based stabilization of betacoronavirus prefusion trimers.* The HKU1-CoV prefusion S structure was used as a starting point to design mutations that would stabilize betacoronavirus S trimers in the prefusion conformation. Dozens of possible stabilizing mutations were designed and tested in the context of the MERS-CoV S protein. Two mutations were identified to be particularly effective for stabilizing the MERS-CoV S protein in its prefusion conformation: V1060P and L1061P (or their combination) (FIG. 4). MERS-CoV S proteins including these mutations also had >50 fold increased expression (FIG. 4). These two proline substitutions are located at the top portion (membrane distal) of the MERS-CoV S2 central helix and HR1 to prevent pre-to-postfusion conformational changes. Prefusion stabilization of the MERS-CoV S protein is preliminarily indicated by increased expression levels when these mutations are combined compared to an S2 truncated, but otherwise wild-type (WT) MERS-CoV S (C6) (FIGs. 4B,4C). WT MERS-CoV S likely spontaneously flips from pre-to-postfusion conformation. Corresponding double proline mutations in SARS-CoV and HKU1-CoV S also increased expression above WT S.

S protein immunogens were expressed from codon-optimized genes encoding the S ectodomain (without TM and CT) with a C-terminal T4 fibritin trimerization domain, an HRV3C cleavage site, a 6xHis-tag and a Twin-Strep-tag that were cloned into the eukaryotic-expression vector pαH. Following sequence verification, expression plasmids were transiently transfected into FreeStyle293 cells. Three hours after transfection, kifunensine was added to a final concentration of 5 µM. Cultures were harvested six days later, and secreted protein was purified from the supernatant and soluble protein w purified from the supernatant by passage over Ni²⁺-NTA and StrepTactin resin using affinity tags on the C-terminus of the proteins. The purified proteins were then be passed over a size-exclusion column to assess their oligomeric state and to isolate monodisperse fractions corresponding to trimeric ectodomains. Protein expression levels were then assessed by SDS-PAGE (10 µL of protein-bound resin was boiled and loaded per lane). This expression strategy was used to generated generate and test proline-substituted variants of MERS-CoV S (Eng1 strain, residues 1-1291), SARS-CoV S (Tor2 strain, residues 1-1190) and HCoV-HKU1 S (N5 strain, residues 1-1276). The MERS-CoV S ectodomain trimers included a 748-RSVR-751 (residues 748-751 of SEQ ID NO: 1) to 748-ASVG-751 (residues 748-751 of SEQ ID NO: 3) substitutions to remove the S1/S2 cleavage site.

Mice (N= 5/group) were vaccinated with 0.1 µg, 1 µg, or 10 µg of the MERS-CoV S trimer stabilized in the prefusion conformation by V1060P and L1061P substitutions to evaluate the effectiveness of the resulting immune response (FIG. 5). As a comparison, mice were also vaccinated with the MERS-CoV S1 protein, which was previously found to induce robust neutralizing antibody responses associated with protection, and MERS-CoV S ectodomain trimers with WT sequence. Control mice were given PBS. The immunogens were based on the England1 ("Eng") MERS-CoV strain.

Immunizations were performed as weeks 0 and 3. Two weeks following the last immunization, serum was collected and tested for neutralization against various MERS pseudovirus strains: England1, Florida USA2, Bisha1, Korea002, JordanN3, Buraidah1, and Indiana USA1. Serum was diluted, in triplicate, and incubated with MERS-CoV pseudovirus prior to inoculation of Huh7.5 cells. Dilution curves were fitted to mock cells and cells exposed to un-neutralized virus as 100% and 0% neutralization, respectively. IC90 titers were calculated as the dilution of serum needed to neutralize 90% of MERS-CoV pseudovirus.

Vaccination with the MERS-CoV S1, wild-type MERS-CoV S ectodomain, or the prefusion stabilized MERS-CoV S ectodomain induced similar robust levels of neutralizing antibodies against homologous MERS-CoV England1 reporter pseudovirus at dosages of 10 µg, but the prefusion-stabilized spike was superior at lower dosages (FIG. 5A). Further, when tested against homologous virus strains, the prefusion stabilized MERS-CoV ectodomain trimer produced a superior immune response (FIG. 5B).

Additional assays were performed to show that vaccination with MERS S-2P ectodomain trimer elicited more non-RBD binding antibodies than MERS S1 ectodomain trimer (FIG. 6A), and higher levels of neutralizing activity targeting a greater diversity of epitopes than antigens based on RBD or S1 monomer (FIG. 6B).

Additionally, challenge studies were performed to determine if the prefusion-stabilized MERS-CoV S ectodomain trimer could prevent MERS-CoV infection in an animal model (FIG. 7). The challenge studies were performed using C57BL/6J mice that were genetically engineered using CRISPR-Cas9 genomic editing to encode human DPP4 mutations (288L and 330R; "288/330^{+/+}") as previously described (see, Cockrell et al., "A mouse model for MERS coronavirus-induced acute respiratory distress syndrome." Nature Microbiology. 2:16226, 2016, which is incorporated by reference herein). These mice are known to be susceptible to invention with MERS-CoV. The 288/330^{+/+} mice were vaccinated with 0.1 µg MERS CoV-S ectodomain timer with the double proline mutation using the Sigma Adjuvant System at weeks 0 and 3. Four weeks following final vaccination, the mice were challenged with a lethal dose of mouse-adapted MERS virus and monitored for survival and weight loss. As shown in FIG. 7, prior immunization with the prefusion stabilized MERS-CoV S ectodomain trimer protected against lethal MERS challenge in mice.

### Example 2

### Prefusion stabilized Coronavirus Spike proteins

HKU1-CoV is closely related to other betacoronaviruses, such as the zoonotic viruses SARS-CoV and MERS-CoV, both of which are associated with high mortality. Accordingly, additional coronavirus S ectodomain trimers stabilized in the prefusion conformation by double proline mutations at the HR1/central helix junction were evaluated as vaccine candidates.

Due to the structural similarity of coronavirus S proteins, the sequences of these proteins can be readily aligned to identify structural domains, such as the HR1 and central helix. FIG. 8 illustrates the structural domains of the HKU1, SARS, and MERS-CoV S proteins, as well as positioning of double proline substitutions to stabilize these proteins in the prefusion conformation. FIG. 8 shows a sequence alignment of the S2 subunit of the HKU1-CoV, SARS-CoV, MERS-CoV, HKU9-CoV, NL63-CoV, and 229E-CoV S proteins, showing relevant sequence homology. The HR1 spans the α13, α14, α17, and α16 helixes, including approximately residues 996-1064 (relative to HKU1-CoV numbering shown in the figure). The central helix is the α17 helix, including approximately residues 1068-1110. The HR2 includes approximates residues 1245-1276 (relative to HKU1-CoV numbering shown in the figure). The transmembrane domain begins at approximately residue 1292 (relative to HKU1-CoV numbering shown in the figure).

Proline substitutions were introduced into the SARS-CoV, HKU1-CoV, OC43-CoV, HKU9-CoV, WIV1-CoV, MHV-CoV, NL63-CoV and 229E-CoV. The SARS-CoV substitutions were K968P, V969P, or K968P and V969P. The HKU1-CoV substitutions are N1067P, L1068P, or N1067P and L1068P. The OC43-CoV substitutions are A1079P, L1080P, or A1079P and L1080P. The HKU9-CoV substitutions are G983P, L984P, or G983P and L984P. The WIV1-CoV substitutions are K969P, V970P, or K969P and V970P. The MHV-CoV substitutions are A1073P, L1074P, or A1073P and L1074P. The NL63-CoV substitutions are S1052P, I1053P, or S1052P and I1053P. The 229E-CoV substitutions are I869P, I870P, or I869P and I870P. Soluble SARS-CoV, HKU1-CoV, OC43-CoV, HKU9-CoV, WIV1-CoV, MHV-CoV, NL63-CoV and 229E-CoV S ectodomain trimers containing the indicated mutations, a signal peptide, and a C-terminal linkage to a T4 Fibritin trimerization domain and streptavidin tag were expressed in cells and purified as described in Example 1. Including the signal peptide and T4 Fibritin trimerization domain, protomer sequences of the referenced ectodomain trimers including the double proline substitutions are as follows:
SARS-CoV S 2P (K968P and V969P, SEQ ID NO: 30)
HKU1-CoV S 2P (N1067P and L1068P, SEQ ID NO: 31)
HKU9-CoV S 2P (G983P and L984P, SEQ ID NO: 32)
OC43-CoV S 2P (A1079P and L1080P, SEQ ID NO: 33)
WIV1-CoV S 2P (K969P and V970P, SEQ ID NO: 34)
MHV-CoV S 2P (A1073P and L1074P, SEQ ID NO: 35)
NL63-CoV S 2P (S1052P and I1053P, SEQ ID NO: 36)
229E-CoV S 2P (I869P and I870P, SEQ ID NO: 37)
PEDV-CoV S 2P (I1076P and L1077P, SEQ ID NO: 40)
As shown in FIG. 10, the proline substitutions boosted the expression of the SARS-CoV and HKU1-CoV S ectodomains.

The thermal stability of the wild-type SARS-CoV S ectodomain (SARS-S-WT) and SARS-CoV S ectodomain with K968P and V969P (SARS-S-2P) was assessed (FIG. 11). About 3 µg SARS-S-WT or SARS-S-2P samples in TBS buffer (2mM Tris pH8.0, 200mM NaCl) were incubated at different temperature for 1 hour. The samples were then analyzed on the NativePAGE Novex Bis-Tris gels (Invitrogen) using procedures suggested by the manufacturer. As shown in FIG. 11, the SARS-S-2P has higher thermal stability than SARS-S-WT.

The expressed protein trimers were further analyzed by gel chromatography. FIG. 12 illustrates results from chromatography experiments concerning wild-type SARS-CoV S ectodomain (SARS-S-WT), SARS-CoV S ectodomain with K968P and V969P (SARS-S-2P), wild-type MERS-CoV S ectodomain (MERS-S-WT), MERS-CoV S ectodomain with V1060P and L1061P (MERS-S-2P), wild-type HKU1-CoV S ectodomain (HKU1-S-WT), HKU1-CoV S ectodomain with N1067P and L1068P (HKU1-S-2P). In all three cases, a larger peak was observed for the double proline mutant, show a many-fold increase in expression of the double proline mutant relative to the WT ectodomain trimer.

The conformation of the double proline mutant SARS-CoV, HKU1-CoV, and MERS-CoV S variants was assessed by negative stain electron microscopy (FIG. 13A). In each case the S variants with the double proline mutant were homogeneous and form trimers in the expected prefusion shape. Each of these ectodomain trimers was purified as a single peak and formed trimers in the typical prefusion conformation. In contrast, corresponding S proteins with native sequences formed trimers of mixed conformation, with some trimers in the typical prefusion conformation and others in the typical elongated post-fusion conformation.

Additionally, the conformation of the double proline mutant OC43-CoV, WIV1-CoV, and PEDV-CoV, and 229E-CoV S variants was also assessed by negative stain electron microscopy (FIGs. 13B-13C). In each case the S variants with the double proline mutant were homogeneous and form trimers in the expected prefusion shape. Each of these ectodomain trimers was purified as a single peak and formed trimers in the typical prefusion conformation.

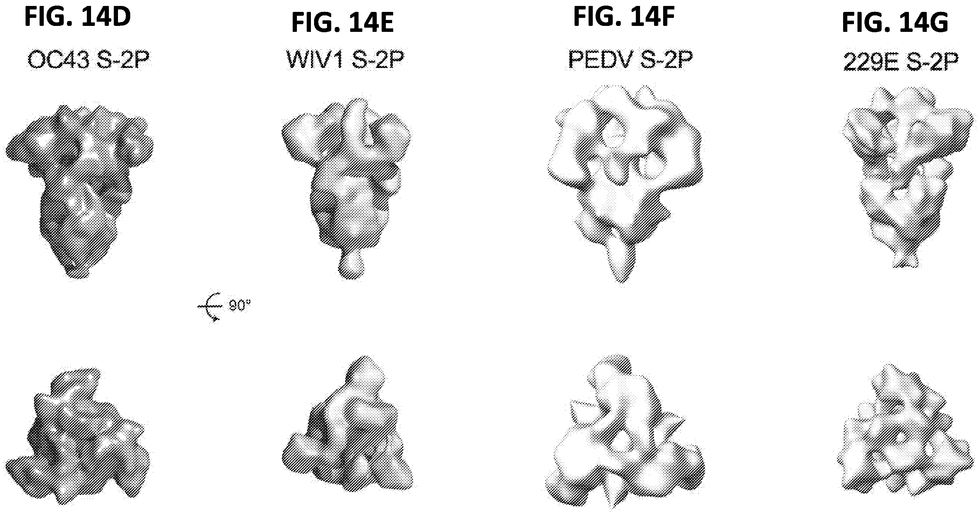

When low resolution negative stain reconstructions of S trimer constructs from HKU1-CoV (FIG. 14A), MERS-CoV (FIG. 14B), SARS-CoV (FIG. 14C), OC43-CoV S 2P (FIG. 14D), WIV1-CoV S 2P (FIG. 14E), PEDV-CoV S 2P (FIG. 14F), and 229E S-2P (FIG. 14G) were reconstructed from the EM data, the articles all formed homogeneous trimeric spike protein structures.

To assess the immunogenicity of the SARS-CoV S 2P ectodomain trimer, mice (N= 5/group) were vaccinated with 0.1 µg or 1 µg of the SARS-CoV S trimer stabilized in the prefusion conformation by K968P and V969P substitutions (SEQ ID NO: 30) to evaluate the effectiveness of the resulting immune response (FIG. 15). As a comparison, mice were also vaccinated with the SARS-CoV S ectodomain trimers with WT sequence. The immunogens were based on the TOR2 SARS-CoV strain. Immunizations were performed as weeks 0 and 3. Two weeks following the last immunization, serum was collected and tested for neutralization against autologous SARS pseudovirus. Serum was diluted, in triplicate, and incubated with SARS-CoV pseudovirus prior to inoculation of Huh7.5 cells. Dilution curves were fitted to mock cells and cells exposed to un-neutralized virus as 100% and 0% neutralization, respectively. IC90 titers were calculated as the dilution of serum needed to neutralize 90% of SARS-CoV pseudovirus. As shown in FIG. 15, vaccination with the prefusion stabilized SARS-CoV S ectodomain induced a superior immune response relative to the wild-type SARS-CoV S ectodomain, particularly at the 0.1 µg dose.

Additionally, mice (N= 5/group) were vaccinated with 0.1 µg, 1 µg, or 10 µg of the HKU1-CoV S trimer stabilized in the prefusion conformation by N1067P and L1068P substitutions (SEQ ID NO: 31) to evaluate the effectiveness of the resulting immune response (FIG. 15). As a comparison, mice were also vaccinated with the HKU1-CoV S ectodomain trimers with WT sequence. Immunizations were performed as weeks 0 and 3. Two weeks following the last immunization, serum was collected and tested for neutralization against autologous HKU1-CoV pseudovirus. Serum was diluted, in triplicate, and incubated with HKU1-CoV pseudovirus prior to inoculation of Huh7.5 cells. Dilution curves were fitted to mock cells and cells exposed to un-neutralized virus as 100% and 0% neutralization, respectively. IC90 titers were calculated as the dilution of serum needed to neutralize 90% of HKU1-CoV pseudovirus. As shown in FIG. 15, vaccination with the prefusion stabilized HKU1-CoV S ectodomain induced a superior immune response relative to the wild-type HKU1-CoV S ectodomain, particularly at the 0.1 µg dose.

In additional assays, mice (N= 5/group) were vaccinated with 1 µg of the OC43-CoV S ectodomain trimer stabilized in the prefusion conformation by A1079P and L1080P substitutions (SEQ ID NO: 33) or with 1 µg of the WIV1-CoV S ectodomain trimer stabilized in the prefusion conformation by K969P and V970P substitutions (SEQ ID NO: 34) to evaluate the effectiveness of the resulting immune response (FIG. 16). PBS was used as a control. Immunizations were performed as weeks 0 and 3. Two weeks following the last immunization, serum was collected and tested for binding to the corresponding immunogen by ELISA. As shown in FIG. 16, vaccination with the prefusion stabilized OC43-CoV S ectodomain trimer or the prefusion stabilized WIV1-CoV S ectodomain trimer elicited antibodies that target the corresponding ectodomain trimers.

It will be apparent that the precise details of the methods or compositions described may be varied or modified without departing from the spirit of the described embodiments. We claim all such modifications and variations that fall within the scope and spirit of the claims below.
Furthermore, the present invention relates to the following items:
1. An immunogen, comprising:
   a recombinant coronavirus S ectodomain trimer comprising protomers comprising one or two proline substitutions at or near a junction between a heptad repeat 1 (HR1) and a central helix that stabilize the S ectodomain trimer in a prefusion conformation.
2. The immunogen of item 1, wherein the one or two proline substitutions comprise two consecutive proline substitutions.
3. The immunogen of item 1 or item 2, wherein the one or two proline substitutions are located between a position 15 amino acids N-terminal of a C-terminal residue of the HR1 and a position 5 amino acids C-terminal of an N-terminal residue of the central helix.
4. The immunogen of any one of the prior items, wherein the coronavirus is one of MERS-CoV, SARS-CoV, NL63-CoV, 229E-CoV, OC43-CoV, HKU1-CoV, WIV1-CoV, MHV, HKU9-CoV, PEDV-CoV, or SDCV.
5. The immunogen of any one of the prior items, wherein the coronavirus is a betacoronavirus.
6. The immunogen of any one of items 1-5, wherein the recombinant coronavirus S ectodomain trimer is a recombinant MERS-CoV S ectodomain trimer, and the one or two proline substitutions are between residues 1050 to 1069 of the protomers in the trimer.
7. The immunogen of item 6, wherein the one or two proline substitutions are between residues 1053 to 1063 of the protomers in the trimer.
8. The immunogen of item 6, wherein the one or two proline substitutions are between residues 1058 to 1063 of the protomers in the trimer.
9. The immunogen of item 6, wherein the one or two proline substitutions are V1060P and/or L1061P substitutions.
10. The immunogen of any one of items 6-9, wherein the protomers in the recombinant MERS-CoV S ectodomain trimer comprise the amino acid sequence set forth as the ectodomain of any one of SEQ ID NOs: 2-4, or an amino acid sequence at least 90% identical thereto wherein the protomers comprise the one or two proline substitutions for stabilization in the ectodomain trimer in the prefusion conformation.
11. The immunogen of item 10, wherein the protomers in the recombinant MERS-CoV S ectodomain trimer comprise residues 18-1291 of any one of SEQ ID NOs: 2-4, or an amino acid sequence at least 90% identical thereto wherein the protomers comprise the one or two proline substitutions for stabilization in the ectodomain trimer in the prefusion conformation.
12. The immunogen of any one of items 6-11, wherein a C-terminal residue of the protomers in the ectodomain is linked to a trimerization domain by a peptide linker, or is directly linked to the trimerization domain.
13. The immunogen of item 12, wherein the trimerization domain is a T4 fibritin trimerization domain.
14. The immunogen of item 13, wherein the protomers linked to the T4 fibritin trimerization domain in the recombinant coronavirus S ectodomain trimer comprise residues 18-1318 of SEQ ID NO: 29 (MERS-2P), or an amino acid sequence at least 90% identical to any one of residues 18-1318 of SEQ ID NO: 29 (MERS-2P), wherein the protomers comprise the one or two proline substitutions for stabilization in the ectodomain trimer in the prefusion conformation.
15. The immunogen of item any one of items 1-5, wherein the recombinant coronavirus S ectodomain trimer is a recombinant SARS-CoV S ectodomain trimer, and the one or two proline substitutions are between residues 951 to 971 of the protomers in the trimer.
16. The immunogen of item 15, wherein the one or two proline substitutions are between residues 961 to 971 of the protomers in the trimer.
17. The immunogen of item 15, wherein the one or two proline substitutions are between residues 966 to 971 of the protomers in the trimer.
18. The immunogen of item 15, wherein the one or two proline substitutions are K968P and/or V969P substitutions.
19. The immunogen of any one of items 15-18, wherein the protomers in the recombinant SARS-CoV S ectodomain trimer comprise the amino acid sequence set forth as SEQ ID NO: 7, or an amino acid sequence at least 90% identical thereto.
20. The immunogen of item 19, wherein the protomers in the recombinant SARS-CoV S ectodomain trimer comprise residues 14-1190 of SEQ ID NO: 7, or an amino acid sequence at least 90% identical thereto wherein the protomers comprise the one or two proline substitutions for stabilization in the ectodomain trimer in the prefusion conformation.
21. The immunogen of any one of items 15-20, wherein a C-terminal residue of the protomers in the ectodomain is linked to a trimerization domain by a peptide linker, or is directly linked to the trimerization domain.
22. The immunogen of item 21, wherein the trimerization domain is a T4 fibritin trimerization domain.
23. The immunogen of item 22, wherein the protomers linked to the T4 fibritin trimerization domain in the recombinant coronavirus S ectodomain trimer comprise residues 14-1217 of SEQ ID NO: 30 (SARS-2P), or an amino acid sequence at least 90% identical to any one of residues 14-1217 of SEQ ID NO: 30 (SARS-2P), wherein the protomers comprise the one or two proline substitutions for stabilization in the ectodomain trimer in the prefusion conformation.
24. The immunogen of item any one item 1-5, wherein the recombinant coronavirus S ectodomain trimer is:
   a recombinant HKU1-CoV S ectodomain trimer, and the one or two proline substitutions are between residues 1050 to 1070, residues 1060 to 1070, and/or residues 1065 to 1070 of the protomers in the trimer;
   a recombinant OC43-CoV S ectodomain trimer, and the one or two proline substitutions are between residues 1062 to 1082, residues 1072 to 1082, and/or residues 1077 to 1082 of the protomers in the trimer;
   a recombinant HKU9-CoV S ectodomain trimer, and the one or two proline substitutions are between residues 966 to 986, residues 976 to 986, and/or residues 981 to 986 of the protomers in the trimer;
   a recombinant NL63-CoV S ectodomain trimer, and the one or two proline substitutions are between residues 1035 to 1055, residues 1045 to 1055, and/or residues 1050 to 1055 of the protomers in the trimer;
   a recombinant 229E-CoV S ectodomain trimer, and the one or two proline substitutions are between residues 852 to 872, residues 862 to 872, and/or residues 867 to 872 of the protomers in the trimer;
   a recombinant WIV1-CoV S ectodomain trimer, and the one or two proline substitutions are between residues 952 to 972, residues 962 to 972, and/or residues 967 to 972 of the protomers in the trimer;
   a recombinant MHV S ectodomain trimer, and the one or two proline substitutions are between residues 1056 to 1076, residues 1066 to 1076, and/or residues 1071 to 1076 of the protomers in the trimer;
   a recombinant PEDV S ectodomain trimer, and the one or two proline substitutions are between residues 1059 to 1079, residues 1069 to 1079, and/or residues 1073 to 1079 of the protomers in the trimer; or
   a recombinant SDCV S ectodomain trimer, and the one or two proline substitutions are between residues 838 to 858, residues 848 to 858, and/or residues 854 to 858 of the protomers in the trimer.
25. The immunogen of item 24, wherein the recombinant coronavirus S ectodomain trimer is:
   a recombinant HKU1-CoV S ectodomain trimer, and the one or two proline substitutions comprise N1067P and/or L1068P substitutions;
   a recombinant OC43-CoV S ectodomain trimer, and the one or two proline substitutions comprise A1079P and/or L1080P substitutions;
   a recombinant HKU9-CoV S ectodomain trimer, and the one or two proline substitutions comprise G1018P and/or L1019P substitutions;
   a recombinant NL63-CoV S ectodomain trimer, and the one or two proline substitutions comprise S1052P and/or I1053P substitutions;
   a recombinant 229E-CoV S ectodomain trimer, and the one or two proline substitutions comprise I869P and/or I870P substitutions;
   a recombinant WIV1-CoV S ectodomain trimer, and the one or two proline substitutions comprise K969P and/or V970P substitutions;
   a recombinant MHV S ectodomain trimer, and the one or two proline substitutions comprise A1073P and/or L1074P substitutions;
   a recombinant PEDV S ectodomain trimer, and the one or two proline substitutions comprise I1076P and L1077P substitutions; or
   a recombinant SDCV S ectodomain trimer, and the one or two proline substitutions comprise E855P and V856P substitutions.
26. The immunogen of item 24 or item 25, wherein the protomers in the recombinant coronavirus S ectodomain trimer comprise the amino acid sequence set forth as the ectodomain of any one of: SEQ ID NO: 9 (HKU1-2P), SEQ ID NO: 11 (OC43-2P), SEQ ID NO: 13 (HKU9-2P), SEQ ID NO: 15 (WIV1-2P), SEQ ID NO: 17 (MHV-2P), SEQ ID NO: 19 (NL63-2P), SEQ ID NO: 21 (229E-2P), SEQ ID NO: 39 (PEDV-2P), or SEQ ID NO: 42 (SDCV-2P), or an amino acid sequence at least 90% identical thereto wherein the protomers comprise the one or two proline substitutions for stabilization in the ectodomain trimer in the prefusion conformation.
27. The immunogen of item 26, wherein the protomers in the recombinant coronavirus S ectodomain trimer comprise any one of:
   residues 14-1276 of SEQ ID NO: 9 (HKU1-2P), residues 15-1287 of SEQ ID NO: 11 (OC43-2P), residues 15-1207 of SEQ ID NO: 13 (HKU9-2P), residues 16-1191 of SEQ ID NO: 15 (WIV1-2P), residues 15-1297 of SEQ ID NO: 17 (MHV-2P), residues 16-1291 of SEQ ID NO: 19 (NL63-2P), residues 17-1108 of SEQ ID NO: 21 (229E-2P), residues 21-1322 of SEQ ID NO: 39 (PEDV-2P), or residues 20-1093 of SEQ ID NO: 42 (SDCV-2P); or
   an amino acid sequence at least 90% identical to any one of residues 14-1276 of SEQ ID NO: 9 (HKU1-2P), residues 15-1287 of SEQ ID NO: 11 (OC43-2P), residues 15-1207 of SEQ ID NO: 13 (HKU9-2P), residues 16-1191 of SEQ ID NO: 15 (WIV1-2P), residues 15-1297 of SEQ ID NO: 17 (MHV-2P), residues 16-1291 of SEQ ID NO: 19 (NL63-2P), residues 17-1108 of SEQ ID NO: 21 (229E-2P), residues 21-1322 of SEQ ID NO: 39 (PEDV-2P), or residues 20-1093 of SEQ ID NO: 42 (SDCV-2P), wherein the protomers comprise the one or two proline substitutions for stabilization in the ectodomain trimer in the prefusion conformation.
28. The immunogen of any one of item 24-25, wherein a C-terminal residue of the protomers in the ectodomain is linked to a trimerization domain by a peptide linker, or is directly linked to the trimerization domain.
29. The immunogen of item 28, wherein the trimerization domain is a T4 fibritin trimerization domain.
30. The immunogen of item 29, wherein the protomers linked to the T4 fibritin trimerization domain in the recombinant coronavirus S ectodomain trimer comprise any one of
   residues 14-1303 of SEQ ID NO: 31 (HKU1-2P), residues 15-1234 of SEQ ID NO: 32 (OC43-2P), residues 15-1314 of SEQ ID NO: 33 (HKU9-2P), residues 16-1218 of SEQ ID NO: 34 (WIV1-2P), residues 15-1324 of SEQ ID NO: 35 (MHV-2P), residues 16-1318 of SEQ ID NO: 36 (NL63-2P), residues 17-1135 of SEQ ID NO: 37 (229E-2P), residues 21-1349 of SEQ ID NO: 40, or residues 20-1120 of SEQ ID NO: 43 (SDCV 2P); or
   an amino acid sequence at least 90% identical to any one of residues 14-1303 of SEQ ID NO: 31 (HKU1-2P), residues 15-1234 of SEQ ID NO: 32 (OC43-2P), residues 15-1314 of SEQ ID NO: 33 (HKU9-2P), residues 16-1218 of SEQ ID NO: 34 (WIV1-2P), residues 15-1324 of SEQ ID NO: 35 (MHV-2P), residues 16-1318 of SEQ ID NO: 36 (NL63-2P), residues 17-1135 of SEQ ID NO: 37 (229E-2P), residues 21-1349 of SEQ ID NO: 40, or residues 20-1120 of SEQ ID NO: 43 (SDCV 2P), wherein the protomers comprise the one or two proline substitutions for stabilization in the ectodomain trimer in the prefusion conformation.
31. The immunogen of any one of the prior items, wherein the protomers of the recombinant coronavirus S ectodomain trimer further comprise one or more additional amino acid substitutions that stabilize the recombinant coronavirus S ectodomain trimer in the prefusion conformation.
32. The immunogen of any one of the prior items, wherein a S1/S2 protease cleavage site and/or a S2' protease cleavage site of the S ectodomain are mutated to inhibit protease cleavage.
33. The immunogen of any one of the prior items, wherein the recombinant coronavirus S ectodomain trimer is soluble.
34. The immunogen of any one of items 1-33, wherein a C-terminal residue of the protomers in the ectodomain is linked to a transmembrane domain by a peptide linker, or is directly linked to the transmembrane domain.
35. The immunogen of any one of items 1-33, wherein a C-terminal residue of the S2 ectodomain is linked to a protein nanoparticle subunit by a peptide linker, or is directly linked to the protein nanoparticle subunit.
36. The immunogen of item 35, wherein the protein nanoparticle subunit is a ferritin nanoparticle subunit.
37. A protein nanoparticle, comprising the immunogen of item 35 or item 36.
38. A virus-like particle comprising the immunogen of any one of items 1-34.
39. An isolated nucleic acid molecule encoding a protomer of the recombinant coronavirus S ectodomain trimer of any one of the prior items.
40. The nucleic acid molecule of item 39, operably linked to a promoter.
41. The nucleic acid molecule of item 39 or item 40, wherein the nucleic acid molecule is an RNA molecule.
42. A vector comprising the nucleic acid molecule of any one of items 39-41.
43. The vector of item 42, wherein the vector is a viral vector.
43. An immunogenic composition comprising the immunogen, protein nanoparticle, virus-like particle, nucleic acid molecule, or vector of any one of items 1-43, and a pharmaceutically acceptable carrier.
45. A method of producing a recombinant coronavirus S ectodomain trimer stabilized in a prefusion conformation, comprising:
   expressing the nucleic acid molecule or vector of any one of items 39-43 in a host cell to produce the recombinant coronavirus S ectodomain trimer; and
   purifying the recombinant coronavirus S ectodomain trimer.
46. The recombinant coronavirus S ectodomain trimer produced by the method of item 45.
47. A method for generating an immune response to an coronavirus S ectodomain in a subject, comprising administering to the subject an effective amount of the immunogen, protein nanoparticle, virus-like particle, nucleic acid molecule, vector, or immunogenic composition of any one of items 1-44 to generate the immune response.
48. The method of item 47, wherein the immune response treats or inhibits infection with the coronavirus.
49. The method of item 47 or item 48, wherein generating the immune response inhibits replication of the coronavirus in the subject.
50. Use of the immunogen, protein nanoparticle, virus-like particle, nucleic acid molecule, vector, or immunogenic composition of any one of items 1-44, to induce an immune response to a coronavirus S ectodomain in a subject.

## Claims

1. An immunogen, comprising:
a recombinant betacoronavirus S ectodomain trimer comprising protomers comprising two consecutive proline substitutions at the junction between the heptad repeat 1 (HR1) and the central helix that stabilize the S ectodomain trimer in a prefusion conformation.

2. The immunogen of claim 1, wherein the betacoronavirus is one of MERS-CoV, SARS-CoV, OC43-CoV, HKU1-CoV, WIV1-CoV, or HKU9-CoV.

3. The immunogen of claim 1 or 2, wherein the recombinant betacoronavirus S ectodomain trimer is:
a recombinant MERS-CoV S ectodomain trimer, and the two proline substitutions are between residues 1058 to 1063 of the protomers in the trimer, preferably, wherein the two proline substitutions are V1060P and L1061P substitutions;
a recombinant SARS-CoV S ectodomain trimer, and the two proline substitutions are between residues 966 to 971 of the protomers in the trimer, preferably, wherein the two proline substitutions are K968P and V969P substitutions;
a recombinant HKU1-CoV S ectodomain trimer, and the two proline substitutions are between residues 1065 to 1070 of the protomers in the trimer, preferably, wherein the two proline substitutions are N1067P and L1068P substitutions;
a recombinant OC43-CoV S ectodomain trimer, and the two proline substitutions are between residues 1077 to 1082 of the protomers in the trimer, preferably, wherein the two proline substitutions comprise A1079P and L1080P substitutions;
a recombinant HKU9-CoV S ectodomain trimer, and the two proline substitutions are between residues 981 to 986 of the protomers in the trimer, preferably, wherein the two proline substitutions comprise G983P and L984P substitutions; or
a recombinant WIV1-CoV S ectodomain trimer, and the two proline substitutions are between residues 967 to 972 of the protomers in the trimer, preferably, wherein the two proline substitutions comprise K969P and V970P substitutions.

4. The immunogen of claim 3, wherein a C-terminal residue of the protomers in the ectodomain is linked to a trimerization domain by a peptide linker, or is directly linked to the trimerization domain, preferably, wherein the trimerization domain is a T4 fibritin trimerization domain.

5. The immunogen of any one of claims 1 to 4, wherein a S1/S2 protease cleavage site and/or a S2' protease cleavage site of the S ectodomain are mutated to inhibit protease cleavage and/or
wherein the recombinant betacoronavirus S ectodomain trimer is soluble and/or
wherein a C-terminal residue of the protomers in the ectodomain is linked to a transmembrane domain by a peptide linker, or is directly linked to the transmembrane domain.

6. The immunogen of any one of claims 1-5, wherein a C-terminal residue of the S2 ectodomain is linked to a protein nanoparticle subunit by a peptide linker, or is directly linked to the protein nanoparticle subunit, preferably, wherein the protein nanoparticle subunit is a ferritin nanoparticle subunit.

7. A protein nanoparticle, comprising the immunogen of claim 6.

8. A virus-like particle comprising the immunogen of any one of claims 1-5.

9. An isolated nucleic acid molecule encoding a protomer of the recombinant betacoronavirus S ectodomain trimer of any one of claims 1-5.

10. A nucleic acid molecule encoding a recombinant betacoronavirus S protein comprising two consecutive proline substitutions at the junction between the heptad repeat 1 (HR1) and the central helix of the S protein according to claim 1.

11. The nucleic acid molecule of claim 9 or 10, operably linked to a promoter.

12. The nucleic acid molecule of claim 9 or 10, wherein the nucleic acid molecule is an RNA molecule.

13. A vector comprising the nucleic acid molecule of any one of claims 9-12.

14. The vector of claim 13, wherein the vector is a viral vector.

15. An immunogenic composition comprising the immunogen, protein nanoparticle, virus-like particle, nucleic acid molecule, or vector of any one of claims 1-14, and a pharmaceutically acceptable carrier.

16. A method of producing a recombinant betacoronavirus S ectodomain trimer stabilized in a prefusion conformation, comprising:
expressing the nucleic acid molecule or vector of any one of claims 9-14 in a host cell to produce the recombinant betacoronavirus S ectodomain trimer; and
purifying the recombinant betacoronavirus S ectodomain trimer.

17. An immunogen, protein nanoparticle, virus-like particle, nucleic acid molecule, vector, or immunogenic composition of any one of claims 1 to 15 for use in generating an immune response to a betacoronavirus S ectodomain in a subject.

18. An immunogen, protein nanoparticle, virus-like particle, nucleic acid molecule, vector, or immunogenic composition of any one of claims 1 to 15 for use as a vaccine.
